# EUROPEAN PATENT APPLICATION

(11) **EP 4 843 989 A1**
(43) Date of publication of application: **25.11.2026**
(21) Application number: 25741651.1
(22) Date of filing: 20.01.2025
(51) Int. Cl.: C07D 498/00, C07D 498/02, C07D 471/04, A61K 31/553, A61P 29/00, A61P 37/00, A61P 25/16

(54) **POLYCYCLIC COMPOUND, PHARMACEUTICAL COMPOSITION THEREOF, AND USE THEREOF**

(30) Priority: 19.01.2024 CN 202410083608
(71) Applicant: Etern Therapeutics (Wuxi) Co., Ltd., Wuxi, Jiangsu 214131 (CN)
(72) Inventor: LU, Jiting, Shanghai 201203 (CN); ZHENG, Qiangang, Shanghai 201203 (CN)
(74) Representative: Keltie LLP
(86) International application number: PCT/CN2025/073253
(87) International publication number: WO 2025/153090

(57) **Abstract**

The present invention provides a polycyclic compound, a pharmaceutical composition thereof, and a use thereof. The polycyclic compound of the present invention has a structure as shown in formula A, wherein the definitions of R₁-R₆, ring A-ring E, L, n, m, q, and t are as described herein. The compound of the present application is an RIPK1 inhibitor, which can be used for treating an RIPK1-related disease.

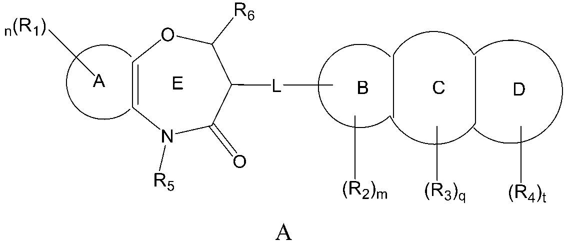

## Description

### Technical field

The present disclosure belongs to the field of biomedicine, and specifically relates to a Ripk1 inhibitor, a pharmaceutical composition thereof and use thereof.

### Background

Receptor-interacting protein kinase 1 (Ripk1) is a key mediator in multiple signaling pathways activated by the death receptor family and pattern recognition receptors. Ripk1 comprises an N-terminal kinase domain, a C-terminal death domain (DD), and an intermediate domain comprising a RIP homotypic interaction motif (RHIM). In TNFα-stimulated cells, Ripk1 kinase exerts pro-cell death and pro-inflammatory activities by activating Ripk1-dependent apoptosis and pyroptosis. The resulting complexes trigger various cellular responses, comprising cytokine release, microglial activation, and necroptosis (a regulated form of cell death). The early role of Ripk1 in this signaling cascade has led to the hypothesis that inhibiting Ripk1 signaling may be beneficial for diseases characterized by excessive cell death and inflammation. In fact, inhibition of Ripk1 activity has been shown to exert protective effects. An increasing number of studies indicate that the development of highly active and highly selective inhibitors targeting Ripk1 can provide new approaches for the treatment of inflammatory diseases, neurological disorders, tumors, sepsis, etc.

Necroptosis can be triggered by numerous mechanisms, comprising TNF receptor activation, Toll-like receptor engagement, genotoxic stress, and viral infection. Downstream of various stimuli, the signaling pathways leading to necroptosis depend on the kinase activities of Ripk1 and Ripk3. (He et al., (2009) Cell 137:1100-1111; Cho et al., (2009) Cell 137:1112-1123; and Zhang et al., (2009) Science 325:332-332). Dysregulation of the necroptosis signaling pathway is associated with inflammatory diseases such as macrophage necrosis in the development of atherosclerosis, virus-induced inflammation, systemic inflammatory response syndrome, and ethanol-induced liver injury; and neurodegeneration such as retinal detachment, ischemia, amyotrophic lateral sclerosis (ALS), and Gaucher's disease (Trichonas et al., (2010) Proc. Natl. Acad. Sci. 107, 21695-21700; Lin et al., (2013) Cell Rep. 3, 200-210; and Cho et al., (2009) Cell, 137, 1112-1123).

Nec-1s is the first Ripkl-specific inhibitor discovered through phenotypic screening. GlaxoSmithKline plc has reported a series of benzoxazepinone compounds, comprising GSK'481, and GSK'772 in clinical trials, as well as dihydropyrazole compounds GSK'547 and GSK'963. R552 is a RIPK1 inhibitor for once-a-day oral dosing, which has previously completed Phase I safety trials in healthy human subjects. In preclinical studies, R552 was demonstrated to prevent joint and skin inflammation in mice. Known indications for the trial to date comprise autoimmune diseases and inflammatory diseases.

### Summary

The purpose of the present disclosure is to provide a Ripk1 inhibitor. The present disclosure also provides a method of preparing the inhibitor, a pharmaceutical composition comprising the inhibitor, and use thereof for treating or preventing Ripk1-mediated diseases.

A first aspect of the present disclosure provides a compound of Formula A, having the structure shown below, or a pharmaceutically acceptable salt thereof, or an enantiomer, diastereomer, tautomer, solvate, polymorph, prodrug or metabolite thereof: 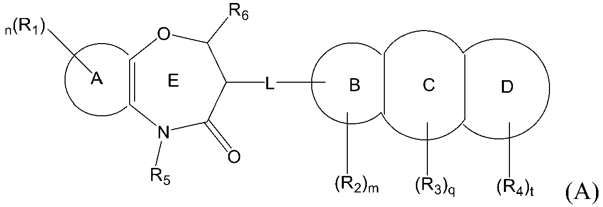 wherein:
ring A is selected from: substituted or unsubstituted C₃₋₈ cycloalkyl, substituted or unsubstituted 3- to 8- membered heterocyclyl, substituted or unsubstituted aryl, substituted or unsubstituted heteroaryl, wherein the heterocyclyl or the heteroaryl comprises 1, 2, or 3 heteroatoms selected from the group consisting of N, O, S, and P;
L is selected from: 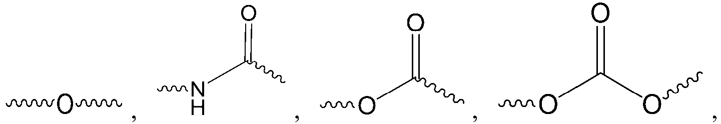 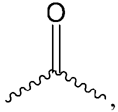 C₁₋₁₀ alkylene, or a chemical bond;
when L is 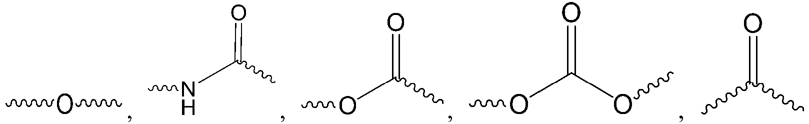 or C₁₋₁₀ alkylene, ring B is selected from: substituted or unsubstituted C₃₋₈ cycloalkyl, substituted or unsubstituted 3- to 8- membered heterocyclyl, substituted or unsubstituted aryl, and substituted or unsubstituted heteroaryl, wherein the heterocyclyl or the heteroaryl comprises 1, 2 or 3 heteroatoms selected from the group consisting of N, O, S and P; ring C and ring D are each independently selected from: substituted or unsubstituted C₃₋₈ cycloalkyl, substituted or unsubstituted 3- to 8- membered heterocyclyl, substituted or unsubstituted C₆₋₁₀ aryl, substituted or unsubstituted 5- to 10- membered heteroaryl, or absent, wherein the heterocyclyl or the heteroaryl comprises 1, 2 or 3 heteroatoms selected from the group consisting of N, O, S and P;
when L is the chemical bond, ring E is directly connected to ring B via the chemical bond, and ring B, ring C and ring D are each independently selected from: substituted or unsubstituted C₃₋₈ cycloalkyl, substituted or unsubstituted heterocyclyl, substituted or unsubstituted aryl, substituted or unsubstituted heteroaryl, wherein the heterocyclyl or the heteroaryl comprises 1, 2 or 3 heteroatoms selected from the group consisting of N, O, S and P;
R₁ is selected from H, halogen, substituted or unsubstituted C₂₋₆ alkynyl, substituted or unsubstituted C₂₋₆ alkenyl, substituted or unsubstituted C₁₋₆ alkyl, substituted or unsubstituted C₁₋₆ alkoxy, cyano, -NO₂, -OH, substituted or unsubstituted C₃₋₈ cycloalkyl, substituted or unsubstituted 3- to 8- membered heterocyclyl, substituted or unsubstituted C₆₋₁₀ aryl, substituted or unsubstituted 5- to 10- membered heteroaryl, substituted or unsubstituted 3- to 8- membered heterocyclyl spiro 3- to 8- membered heterocyclyl, substituted or unsubstituted 3- to 8- membered heterocyclyl fused 3- to 8- membered heterocyclyl, substituted or unsubstituted C₃₋₈ cycloalkyl spiro C₃₋₈ cycloalkyl, substituted or unsubstituted C₃₋₈ cycloalkyl fused C₃₋₈ cycloalkyl, substituted or unsubstituted 3- to 8- membered heterocyclyl fused C₆₋₁₀ aryl, substituted or unsubstituted 3- to 8- membered heterocyclyl fused 5- to 10- membered heteroaryl, substituted or unsubstituted C₃₋₈ cycloalkyl fused C₆₋₁₀ aryl, substituted or unsubstituted C₃₋₈ cycloalkyl fused 5- to 10- membered heteroaryl, wherein the heterocyclyl or the heteroaryl comprises 1, 2 or 3 heteroatoms selected from the group consisting of N, O, S and P;
R₂ is selected from H, oxo (=O), substituted or unsubstituted C₁₋₆ alkoxy, halogen, substituted or unsubstituted C₂₋₆ alkynyl, substituted or unsubstituted C₂₋₆ alkenyl, substituted or unsubstituted C₁₋₆ alkyl, cyano, -NO₂, -OH, substituted or unsubstituted C₃₋₈ cycloalkyl, substituted or unsubstituted 3- to 8- membered heterocyclyl, substituted or unsubstituted C₆₋₁₀ aryl, substituted or unsubstituted 5- to 10- membered heteroaryl, wherein the heterocyclyl or the heteroaryl comprises 1, 2 or 3 heteroatoms selected from the group consisting of N, O, S and P;
R₃ is selected from H, oxo, substituted or unsubstituted C₁₋₆ alkoxy, halogen, substituted or unsubstituted C₂₋₆ alkynyl, substituted or unsubstituted C₂₋₆ alkenyl, substituted or unsubstituted C₁₋₆ alkyl, cyano, -NO₂, -OH, substituted or unsubstituted C₃₋₈ cycloalkyl, substituted or unsubstituted 3- to 8- membered heterocyclyl, substituted or unsubstituted C₆₋₁₀ aryl, substituted or unsubstituted 5- to 10- membered heteroaryl, wherein the heterocyclyl or the heteroaryl comprises 1, 2 or 3 heteroatoms selected from the group consisting of N, O, S and P;
R₄ is selected from H, oxo, substituted or unsubstituted C₁₋₆ alkoxy, halogen, substituted or unsubstituted C₂₋₆ alkynyl, substituted or unsubstituted C₂₋₆ alkenyl, substituted or unsubstituted C₁₋₆ alkyl, cyano, -NO₂, -OH, substituted or unsubstituted C₃₋₈ cycloalkyl, substituted or unsubstituted 3- to 8- membered heterocyclyl, substituted or unsubstituted C₆₋₁₀ aryl, substituted or unsubstituted 5- to 10- membered heteroaryl, wherein the heterocyclyl or the heteroaryl comprises 1, 2 or 3 heteroatoms selected from the group consisting of N, O, S and P;
R₅ is selected from H, halogen, substituted or unsubstituted C₂₋₆ alkynyl, substituted or unsubstituted C₂₋₆ alkenyl, substituted or unsubstituted C₁₋₆ alkyl, substituted or unsubstituted C₁₋₆ alkoxy, cyano, -NO₂, -OH, substituted or unsubstituted C₃₋₈ cycloalkyl, substituted or unsubstituted 3- to 8- membered heterocyclyl, substituted or unsubstituted C₆₋₁₀ aryl, substituted or unsubstituted 5- to 10- membered heteroaryl, wherein the heterocyclyl or the heteroaryl comprises 1, 2 or 3 heteroatoms selected from the group consisting of N, O, S and P;
R₆ is selected from H, halogen, substituted or unsubstituted C₂₋₆ alkynyl, substituted or unsubstituted C₂₋₆ alkenyl, substituted or unsubstituted C₁₋₆ alkyl, substituted or unsubstituted C₁₋₆ alkoxy, cyano, -NO₂, -OH, substituted or unsubstituted C₃₋₈ cycloalkyl, substituted or unsubstituted 3- to 8- membered heterocyclyl, substituted or unsubstituted C₆₋₁₀ aryl, substituted or unsubstituted 5- to 10- membered heteroaryl, wherein the heterocyclyl or the heteroaryl comprises 1, 2 or 3 heteroatoms selected from the group consisting of N, O, S and P;
n, m, q and t are each independently any integer selected from 0, 1, 2 and 3;
the "substituted" means that one or more hydrogen atoms on a group are substituted with a substituent selected from the group consisting of: halogen, -OH, -NO₂, -NH₂, -NH(unsubstituted or halogenated C₁₋₆ alkyl), -N(unsubstituted or halogenated C₁₋₆ alkyl)₂, -CN, unsubstituted C₁₋₈ alkyl or C₁₋₈ alkyl substituted with halogen or hydroxy, unsubstituted or halogenated C₁₋₈ alkoxy, unsubstituted or halogenated C₁₋₈ alkoxy-C₁₋₈ alkyl, unsubstituted or halogenated C₃₋₈ cycloalkyl-C₁₋₈ alkyl, unsubstituted or halogenated C₁₋₆ alkylcarbonyl, unsubstituted or halogenated C₁₋₆ alkoxycarbonyl, hydroxamic acid group, unsubstituted or halogenated C₁₋₆ alkylthio, - S(O)₂N(unsubstituted or halogenated C₁₋₆ alkyl)₂, -S(O)₂ unsubstituted or halogenated C₁₋₆ alkyl, - N(unsubstituted or halogenated C₁₋₆ alkyl)S(O)₂N(unsubstituted or halogenated C₁₋₆ alkyl)₂, - S(O)N(unsubstituted or halogenated C₁₋₆ alkyl)₂, -S(O)(unsubstituted or halogenated C₁₋₆ alkyl), - N(unsubstituted or halogenated C₁₋₆ alkyl)S(O)N(unsubstituted or halogenated C₁₋₆ alkyl)₂, - N(unsubstituted or halogenated C₁₋₆ alkyl)S(O)(unsubstituted or halogenated C₁₋₆ alkyl), unsubstituted or halogenated C₅₋₁₀ aryl, unsubstituted or halogenated 5- to 10- membered heteroaryl, unsubstituted or halogenated C₃₋₈ cycloalkyl and unsubstituted or halogenated 3- to 8- membered heterocyclyl, wherein the heterocyclyl and the heteroaryl comprise 1, 2, 3 or 4 heteroatoms selected from the group consisting of N, O and S;
" 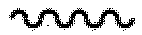 " represents the position connected to other parts of the molecule via a chemical bond.

A second aspect of the present disclosure provides a pharmaceutical composition comprising: (a) the compound of Formula A according to the first aspect of the present disclosure, or a pharmaceutically acceptable salt thereof, or a solvate, isotopically substituted derivative, polymorph, prodrug or metabolite thereof, as an active ingredient, and (b) a pharmaceutically acceptable carrier or excipient.

A third aspect of the present disclosure provides use of the compound of Formula A according to the first aspect of the present disclosure, or a pharmaceutically acceptable salt thereof, or a solvate, isotopically substituted derivative, polymorph, prodrug or metabolite thereof in the manufacture of a medicament for treating or preventing Ripk1-related diseases.

### Detailed Description

The following detailed embodiments are provided to enable those skilled in the art to more clearly understand the content of the present disclosure. It is noted that these embodiments are described for illustrative purposes only and are not intended to limit the scope of protection of the present disclosure.

In order to enable those skilled in the art to understand the features and effects of the present disclosure, a general description and definition of terms and words mentioned in the specification and claims are provided below. Unless otherwise specified, all technical and scientific terms used herein are intended to be the ordinary meaning as understood by those skilled in the art for the present disclosure, and in case of a conflict, the definition of this specification shall prevail.

The theories or mechanisms described and disclosed herein, whether right or wrong, should not limit the scope of the present disclosure in any way. That is to say the technical solutions of present disclosure can be practiced without limited by any particular theory or mechanism.

As used herein, the terms "comprising", "including", "containing" and the like encompass meanings of "consisting essentially of" and "consisting of". For example, where it is disclosed herein that "A comprises B and C", it is considered that "A consists essentially of B and C" and "A consists of B and C" are disclosed herein.

In the present disclosure, all features, such as numerical values, quantities, amounts and concentrations, which are defined by numerical ranges or percentage ranges, are only for the sake of simplicity and convenience. Accordingly, the recitation of a numerical range or a percentage range shall be construed as covering and specifically disclosing all possible sub-ranges and individual values (including integers and fractions) in the range.

In the present disclosure, unless otherwise specified, a percentage refers to a mass percentage, and a proportion refers to a mass ratio.

In the present disclosure, when embodiments or Examples are described, it is understood that they are not intended to limit the disclosure to these embodiments or Examples. Rather, all alternatives, modifications and equivalents of the methods and materials described in the technical solutions of the present disclosure can be comprised in the scope defined by the claims.

Herein, for the sake of brevity of description, all possible combinations of various technical features in the various embodiments or Examples are not described. Therefore, as long as there is no contradiction in the combination of these technical features, the various technical features in the various embodiments or Examples can be combined in any combination, and all possible combinations are considered to be within the scope of this specification.

### Terms

Unless otherwise defined, all scientific and technical terms used herein have the same meanings as commonly understood by those skilled in the art to which the claimed subject matter pertains. Unless otherwise stated, all patents, patent applications and publications cited herein are incorporated herein by reference in their entirety.

It is understood that the foregoing brief description and the following detailed description are exemplary and explanatory only and do not limit the subject matter of the present disclosure in any way. In this disclosure, unless otherwise specifically stated, the use of the singular also comprises the plural. It is noted that, unless otherwise clearly stated, the singular forms used in the specification and the claims comprise the plural referents. It is also noted that, unless otherwise stated, "or" as used mean "and/or". In addition, the term "comprise" and other forms such as "include", "contain" and "containing" are not restrictive and may be open-ended, semi-closed-ended or closed-ended. In other words, the terms also comprise the meanings of "consist essentially of" or "consist of".

Definitions of standard chemical terms can be found in references (including Carey and Sundberg "ADVANCED ORGANIC CHEMISTRY 4TH ED." Vols. A (2000) and B (2001), Plenum Press, New York). Unless otherwise specified, conventional methods within the scope of the art are employed, such as mass spectrometry, NMR, IR and UV/VIS spectroscopy, and pharmacological methods. Unless specific definitions are provided, the terms used in the relevant descriptions of analytical chemistry, organic synthetic chemistry, and drugs and medicinal chemistry herein are those known in the art. Standard techniques can be used in chemical synthesis, chemical analysis, drug preparation, formulation and delivery, and treatment of patients. For example, reactions can be performed and purification can be carried out using the manufacturer's instructions for the kit, or according to methods known in the art or the descriptions of the present disclosure. The above techniques and methods can generally be carried out based on conventional methods well known in the art, according to the descriptions in the various general and more specific references cited and discussed in this specification. In this specification, those skilled in the art can select groups and their substituents to provide stable structural moieties and compounds.

When a substituent is described by a conventional chemical formula written from left to right, the substituent also comprises chemically equivalent substituents obtained when the structural formula is written from right to left. For example, -CH₂O- is equivalent to -OCH₂-.

The section headings used herein are for organizational purposes only and are not construed as limiting the subject matter described. All or some references cited in this disclosure, including but not limited to patents, patent applications, articles, books, operation manuals and papers, are incorporated herein by reference in their entirety.

Certain chemical groups defined in the present disclosure are preceded by a simplified symbol indicating the total number of carbon atoms present in the group. For example, C₁₋₆ alkyl refers to an alkyl group as defined below having a total of 1 to 6 carbon atoms. The total number of carbon atoms in the simplified symbol does not comprise carbons that may be present in substituents of the group.

When a variable is recited as "selected from: ......" in the present disclosure, it means that the variable is selected from any one of the options listed after the colon, or where possible, the variable is selected from one or more of the options listed after the colon.

All numerical ranges mentioned in the present disclosure are intended to comprise both endpoints of the range, all integers within the range, and subranges formed by these integers.

In addition to the foregoing, when used in the specification and claims of the present disclosure, the following terms have the meanings indicated below, unless otherwise specifically indicated.

The term "halogen" refers to fluorine, chlorine, bromine or iodine.

"Hydroxyl" refers to a -OH group.

"Hydroxyalkyl" refers to an alkyl group as defined below substituted with hydroxyl (-OH). "Carbonyl" refers to a -C(=O)- group.

"Cyano" refers to -CN.

"Oxo" refers to =O.

"Thioxo" refers to =S.

"Nitro" refers to -NO₂.

"Amino" refers to -NH₂.

"Substituted amino" refers to an amino group substituted with one or two alkyl, alkylcarbonyl, arylalkyl, aryl, heteroaryl, heterocyclyl, heteroarylalkyl as defined below, such as monoalkylamino, dialkylamino, alkylcarbonylamino, arylalkylamino, heteroarylalkylamino, heterocyclylamino, heteroarylamino and arylamino. In some embodiments herein, "substituted amino" is represented as -NR'R", wherein R' and R" are each independently selected from H, C1-C4 alkyl, C1-C4 alkylS(O)₂- and halogenated C1-C4 alkyl (provided that at least one of R' and R" is not H).

"Carboxyl" refers to -COOH.

"Amino" refers to -NH₂.

In the present disclosure, the term "Ci-Cj" indicates the range of the number of carbon atoms, where i and j are integers, and the range of the number of carbon atoms comprises the endpoints (i.e., i and j) and every integer point between the endpoints, where j is greater than i. For example, C1-C6 indicates a range of from 1 to 6 carbon atoms, comprising 1 carbon atom, 2 carbon atoms, 3 carbon atoms, 4 carbon atoms, 5 carbon atoms and 6 carbon atoms. Further examples comprise the term "C1-C12" indicating 1 to 12, particularly 1 to 10, particularly 1 to 8, particularly 1 to 6, particularly 1 to 5, particularly 1 to 4, particularly 1 to 3 or particularly 1 to 2 carbon atoms.

In the present disclosure, as a group or part of another group (e.g., in groups such as alkyl substituted with halogen (e.g., fluorine, chlorine, bromine or iodine)), the term "alkyl" refers to a fully saturated straight or branched hydrocarbon chain group consisting only of carbon and hydrogen atoms, and attached to the rest of the molecule via a single bond. In some embodiments, alkyl has 1 to 12 carbon atoms. In some embodiments, alkyl comprises 1 to 11 carbon atoms. In some embodiments, alkyl comprises 1 to 10, 1 to 9, 1 to 8, 1 to 7, 1 to 6, 1 to 5, 1 to 4, 1 to 3 or 1 to 2 carbon atoms. Non-limiting examples of alkyl include, but are not limited to, methyl, ethyl, n-propyl, isopropyl, n-butyl, isobutyl, sec-butyl, tert-butyl, n-pentyl, tert-pentyl, 2-methylbutyl, 2,2-dimethylpropyl, n-hexyl, n-heptyl, 2-methylhexyl, 3-methylhexyl, n-octyl, n-nonyl, n-decyl, n-undecyl and n-dodecyl. For the purposes of the present disclosure, the term "alkyl" refers to alkyl comprising 1 to 6 carbon atoms. Unless otherwise specifically specified in this specification, alkyl may be optionally substituted.

In the present disclosure, as a group or part of another group, the term "alkenyl" means a straight or branched hydrocarbon chain group consisting only of carbon and hydrogen atoms and comprising at least one double bond, which is attached to the rest of the molecule via a single bond. In some embodiments, alkenyl comprises 2 to 20 atoms. In some embodiments, alkenyl comprises 2 to 19 carbon atoms. In some embodiments, alkenyl comprises 2 to 18 carbon atoms. In some embodiments, alkenyl comprises 2 to 17 carbon atoms. In some embodiments, alkenyl comprises 2 to 16 carbon atoms. In some embodiments, alkenyl comprises 2 to 17 carbon atoms. In some embodiments, alkenyl comprises 2 to 16 carbon atoms. In some embodiments, alkenyl comprises 2 to 15 carbon atoms. In some embodiments, alkenyl comprises 2 to 14 carbon atoms. In some embodiments, alkenyl comprises 2 to 13 carbon atoms. In some embodiments, alkenyl comprises 2 to 12 carbon atoms. In some embodiments, alkenyl comprises 2 to 11 carbon atoms. In some embodiments, alkenyl comprises 2 to 10 carbon atoms. In some embodiments, alkenyl comprises 2 to 9 carbon atoms. In some embodiments, alkenyl comprises 2 to 8 carbon atoms. In some embodiments, alkenyl comprises 2 to 7 carbon atoms. In some embodiments, alkenyl comprises 2 to 6 carbon atoms. In some embodiments, alkenyl comprises 2 to 5 carbon atoms. In some embodiments, alkenyl comprises 2 to 4 carbon atoms. In some embodiments, alkenyl comprises 2 to 3 carbon atoms. In some embodiments, alkenyl comprises 2 carbon atoms. Non-limiting examples of alkenyl include but are not limited to vinyl, propenyl, allyl, butenyl, but-1-enyl, but-2-enyl, pentenyl, pent-1-enyl, pentadienyl, pent-1,4-dienyl. Unless otherwise specified in this specification, the alkenyl may be optionally substituted.

In the present disclosure, as a group or part of another group, the term "alkynyl" refers to a straight or branched hydrocarbon chain group consisting only of carbon and hydrogen atoms andhaving one or more carbon-carbon triple bonds (-C=C-), which is attached to the rest of the molecule via a single bond. In some embodiments, alkynyl comprises 2 to 20 carbon atoms. In some embodiments, alkynyl comprises 2 to 19 carbon atoms. In some embodiments, alkynyl comprises 2 to 18 carbon atoms. In some embodiments, alkynyl comprises 2 to 17 carbon atoms. In some embodiments, alkynyl comprises 2 to 16 carbon atoms. In some embodiments, alkynyl comprises 2 to 17 carbon atoms. In some embodiments, alkynyl comprises 2 to 16 carbon atoms. In some embodiments, alkynyl comprises 2 to 15 carbon atoms. In some embodiments, alkynyl comprises 2 to 14 carbon atoms. In some embodiments, alkynyl comprises 2 to 13 carbon atoms. In some embodiments, alkynyl comprises 2 to 12 carbon atoms. In some embodiments, alkynyl comprises 2 to 11 carbon atoms. In some embodiments, alkynyl comprises 2 to 10 carbon atoms. In some embodiments, alkynyl comprises 2 to 9 carbon atoms. In some embodiments, alkynyl comprises 2 to 8 carbon atoms. In some embodiments, alkynyl comprises 2 to 7 carbon atoms. In some embodiments, alkynyl comprises 2 to 6 carbon atoms. In some embodiments, alkynyl comprises 2 to 5 carbon atoms. In some embodiments, alkynyl comprises 2 to 4 carbon atoms. In some embodiments, alkynyl comprises 2 to 3 carbon atoms. In some embodiments, alkynyl comprises 2 carbon atoms. Non-limiting examples of alkynyl comprise ethynyl, 1-propynyl, 1-methyl-2-propynyl, 2-propynyl, 1-butynyl and 2-butynyl. Unless otherwise specifically specified in this specification, the alkynyl may be optionally substituted.

In the present disclosure, as a group or part of another group, the term "alkylene" refers to a group that is the same as alkyl but is divalent. In some embodiments, alkylene comprises 1-20 carbon atoms, such as C₁₋₁₀ alkylene, C₁₋₆ alkylene, C₁₋₄ alkylene. Examples of alkylene comprise methylene (-CH₂-), ethylene (-CH₂CH₂-), propylene (-CH₂CH₂CH₂-), butylene (-CH₂CH₂CH₂CH₂-), etc.

In the present disclosure, as a group or part of another group, the term "alkoxy" refers to an "-O-alkyl" group, wherein the alkyl has the meaning described above. In some embodiments, alkoxy is C₁₋₂₀ alkoxy, C₁₋₁₉ alkoxy, C₁₋₁₈ alkoxy, C₁₋₁₇ alkoxy, C₁₋₁₆ alkoxy, C₁₋₁₅ alkoxy, C₁₋₁₄ alkoxy, C₁₋₁₃ alkoxy, C₁₋₁₂ alkoxy, C₁₋₁₁ alkoxy. In some embodiments, alkoxy is C₁₋₁₀ alkoxy. In some embodiments, alkoxy is C₁₋₈ alkoxy. In some embodiments, alkoxy is C₁₋₆ alkoxy. In some embodiments, alkoxy is C₁₋₄ alkoxy, C₁₋₃ alkoxy or C₁₋₂ alkoxy. Non-limiting examples of alkoxy include, but are not limited to, methoxy, ethoxy, n-propoxy, isopropoxy, n-butoxy, tert-butoxy, sec-butoxy, n-pentoxy, n-hexoxy, 1,2-dimethylbutoxy. Alkenyloxy refers to "alkenyl-O-", wherein the alkenyl has the meaning described above. Alkynyloxy refers to "alkynyl-O-", wherein the alkynyl has the meaning described above. "Substituted alkoxy" refers to substituted alkyl-O-.

In the present disclosure, as a group or part of another group, the term "haloalkyl" refers to an alkyl group substituted with one or more halogens, wherein the alkyl has the meaning described above and the number of halogens can be up to the number of hydrogens that can be substituted on alkyl.

In the present disclosure, as a group or part of another group, the term "acyl" refers to a monovalent group remaining after the hydroxyl group is removed from an aliphatic carboxylic acid, which can be represented by the general formula "G-C(=O)-", wherein G represents H or alkyl, alkenyl or alkynyl as described above. For example, specific examples of C1-C4 acyl include, but are not limited to, formyl, acetyl, propionyl, butyryl, or the like.

In the present disclosure, as a group or part of another group, the term "cyclohydrocarbyl" refers to a saturated or partially unsaturated non-aromatic monocyclic or polycyclic hydrocarbon group or moiety (referring to a structural fragment of an organic molecule) consisting only of carbon and hydrogen atoms, optionally having one or more carbon-carbon double bonds (-C=C-) or carbon-carbon triple bonds (-C≡C-), which may be attached to the rest of the molecule via any suitable carbon atom. It comprises "cycloalkyl", "cycloalkenyl" and "cycloalkynyl". In some embodiments, cyclohydrocarbyl comprises 3, 4, 5, 6, 7, 8, 9, 10, 11 or 12 ring carbon atoms. Non-limiting examples of cyclohydrocarbyl include, but are not limited to, cyclopropyl, cyclobutyl, cyclopentyl, 1-cyclopent-1-enyl, 1-cyclopent-2-enyl, 1-cyclopent-3-enyl, cyclohexyl, 1-cyclohex-1-enyl, 1-cyclohex-2-enyl, 1-cyclohex-3-enyl, cyclohexadienyl, cycloheptyl, cyclooctyl, cyclononyl, cyclodecyl, cycloundecyl, cyclododecyl and cyclooctynyl. Unless otherwise specifically specified in this specification, carbon atoms in cyclohydrocarbyl may be optionally oxidized (thus forming an oxo (=O) group), and the cyclohydrocarbyl may be optionally substituted.

In the present disclosure, as a group or part of another group, the term "cycloalkyl" refers to a saturated non-aromatic monocyclic or polycyclic alkyl or moiety consisting only of carbon and hydrogen atoms, which may be attached to the rest of the molecule via any suitable carbon atom. In some embodiments, cycloalkyl may comprise fused ring systems, bridged ring systems or spiro ring systems. In some embodiments, cycloalkyl comprises 3 to 15, 3 to 14, 3 to 13, 3 to 12, 3 to 11, 3 to 10, 3 to 9, 3 to 8 carbon atoms. In some embodiments, cycloalkyl has 3, 4, 5, 6, 7 or 8 carbon atoms. Unless otherwise specified in this specification, the carbon atoms in cycloalkyl may be optionally oxidized. Non-limiting examples of cycloalkyl include, but are not limited to, cyclopropyl, cyclobutyl, cyclopentyl, cyclopentenyl, cyclohexyl, cyclohexenyl, cyclohexadienyl, cycloheptyl, cyclooctyl, 1H-indenyl, 2,3-dihydroindenyl, 1,2,3,4-tetrahydro-naphthyl, 5,6,7,8-tetrahydro-naphthyl, 8,9-dihydro-7H-benzocyclohepten-6-yl, 6,7,8,9-tetrahydro-5H-benzocycloheptenyl, 5,6,7,8,9,10-hexahydro-benzocyclooctenyl, fluorenyl, bicyclo[2.2.1]heptyl, 7,7-dimethyl-bicyclo[2.2.1]heptyl, bicyclo[2.2.1]heptenyl, bicyclo[2.2.2]octyl, bicyclo[3.1.1]heptyl, bicyclo[3.2.1]octyl, bicyclo[2.2.2]octenyl, bicyclo[3.2.1]octenyl, adamantyl, octahydro-4,7-methano-1H-indenyl and octahydro-2,5-methanopentalenyl.

In the present disclosure, as a group or part of another group, the term "cycloalkenyl" refers to a partially unsaturated non-aromatic monocyclic or polycyclic hydrocarbon group or moiety consisting only of carbon and hydrogen atoms and having one or more carbon-carbon double bonds (-C=C-), which may be attached to the rest of the molecule via any suitable carbon atom. In some embodiments, cycloalkenyl comprises 5 to 12, 5 to 10, 5 to 9, 5 to 8, 5 to 7, or 5 to 6 ring carbon atoms. Non-limiting examples of cycloalkenyl comprise cyclopentenyl, cyclohexenyl, cycloheptenyl and cyclooctenyl, etc. Unless otherwise specifically specified in this specification, the carbon atoms in cycloalkenyl may be optionally oxidized (thus forming an oxo (=O) group), and the cycloalkenyl may be optionally substituted.

In the present disclosure, as a group or part of another group, the term "cycloalkynyl" refers to a partially unsaturated non-aromatic monocyclic or polycyclic hydrocarbon group or moiety consisting only of carbon and hydrogen atoms and having one or more carbon-carbon triple bonds (-C=C-), which may be attached to the rest of the molecule via any suitable carbon atom. In some embodiments, cycloalkynyl comprises 8 to 12, 8 to 11, 8 to 10, or 8 to 9 ring carbon atoms. Non-limiting examples of cycloalkynyl comprise cyclooctynyl, etc. Unless otherwise specifically specified in this specification, the carbon atoms in cycloalkynyl may be optionally oxidized (thus forming an oxo (=O) group), and cycloalkynyl may be optionally substituted.

In the present disclosure, as a group or part of another group, the term "spirocyclyl" or "spiro ring" refers to a ring system of cyclohydrocarbyl consisting of multiple (e.g., two, three or more) rings, wherein at least two rings are bonded to each other sharing one carbon atom. In some embodiments, spirocyclyl comprises 6 to 12, 6 to 11, 6 to 10, 6 to 9, 6 to 8, 6 to 7 ring carbon atoms. Non-limiting examples of spirocyclyl include, but are not limited to, spiro[5.5]undecyl, spiro-pentadienyl, spiro[3.6]decyl. Unless otherwise specifically specified in this specification, spirocyclyl may be optionally substituted.

In the present disclosure, as a group or part of another group, the term "fused cyclyl" or "fused ring (also known as 'condensed ring')" refers to a ring system of cyclohydrocarbyl consisting of multiple (e.g., two, three or more) rings, wherein at least two rings are bonded to each other sharing two adjacent carbon atoms (i.e., the at least two rings share one covalent bond, such that bridgehead atoms are directly connected). In some embodiments, fused cyclyl comprises 5 to 12, 5 to 11, 5 to 10, 5 to 9, 5 to 8, 5 to 7, 5 to 6 ring carbon atoms. Non-limiting examples of fused cyclyl include, but are not limited to, 2,3-dihydroindenyl, 1,2,3,4-tetrahydro-naphthyl, 5,6,7,8-tetrahydro-naphthyl, 8,9-dihydro-7H-benzocyclohepten-6-yl, 6,7,8,9-tetrahydro-5H-benzocycloheptenyl, 5,6,7,8,9,10-hexahydro-benzocyclooctenyl. Unless otherwise specifically specified in this specification, fused cyclyl may be optionally substituted.

In the present disclosure, as a group or part of another group, the term "bridged cyclyl" or "bridged ring" refers to a ring system of cyclohydrocarbyl consisting of multiple (e.g., two, three or more) rings, wherein at least two rings are bonded to each other sharing three or more carbon atoms (wherein the at least two rings separate two bridgehead atoms by a bridge containing at least one atom). In some embodiments, bridged cyclyl comprises 8 to 12, 8 to 11, 8 to 10, 8 to 9 ring carbon atoms. Non-limiting examples of bridged cyclyl include, but are not limited to, bicyclo[1.1.1]pentenyl, bicyclo[2.2.1]heptenyl, bicyclo[2.2.1]heptyl, bicyclo[3.1.1]heptyl, bicyclo[2.2.2]octyl, bicyclo[3.2.1]octyl, bicyclo[3.2.1]octenyl, bicyclo[3.3.1]nonyl, bicyclo[3.3.3]undecyl, adamantyl, or the like. Unless otherwise specifically specified in this specification, bridged cyclyl may be optionally substituted.

In the present disclosure, as a group or part of another group, the term "heterocyclyl" or "heterocycle" refers to a stable saturated or partially unsaturated 3- to 20-membered non-aromatic cyclic group or moiety consisting of 2 to 14 carbon atoms (e.g., 2, 3, 4, 5, 6, 7, 8, 9, 10, 11, 12, 13 or 14 carbon atoms) and 1 to 6 heteroatoms selected from nitrogen, phosphorus, oxygen and sulfur (e.g., 1, 2, 3, 4, 5 or 6 heteroatoms). In some embodiments, heterocyclyl may be 3-, 4-, 5-, 6-, 7-, 8-, 9-, 10-, 11-, 12-, 13-, 14-, 15-, 16-, 17-, 18-, 19- or 20-membered heterocyclyl, such as 3- to 20-membered heterocyclyl, 3- to 19- membered heterocyclyl, 3- to 18- membered heterocyclyl, 3- to 17- membered heterocyclyl, 3- to 16- membered heterocyclyl, 3- to 15- membered heterocyclyl, 4-to 12- membered heterocyclyl, 4- to 10- membered heterocyclyl, 4- to 9- membered heterocyclyl, 4- to 8- membered heterocyclyl, 4- to 7- membered heterocyclyl, 4- to 6- membered heterocyclyl, 4- to 5- membered heterocyclyl. Unless otherwise specifically specified in this specification, the heterocyclyl may be a monocyclic, bicyclic, tricyclic or polycyclic ring system, which may comprise condensed/fused ring systems (i.e., fused heterocyclyl, e.g., 4- to 9- membered fused heterocyclyl), bridged ring systems (i.e., bridged heterocyclyl, e.g., 6- to 12- membered bridged heterocyclyl) or spiro ring systems (i.e., spiro heterocyclyl, e.g., 6- to 12- membered spiro heterocyclyl). Fused heterocyclyl refers to a ring system of heterocyclyl consisting of multiple (e.g., two, three or more) rings, wherein at least two rings are bonded to each other sharing two adjacent atoms (i.e., the at least two rings share one covalent bond, such that bridgehead atoms are directly connected). In some embodiments, the heterocyclyl is bicyclic fused heterocyclyl. Bridged heterocyclyl refers to a ring system of heterocyclyl consisting of multiple (e.g., two, three or more) rings, wherein at least two rings are bonded to each other sharing three or more atoms (wherein the at least two rings separate two bridgehead atoms by a bridge containing at least one atom). Spiro heterocyclyl refers to a ring system of heterocyclyl consisting of multiple (e.g., two, three or more) rings, wherein at least two rings are bonded to each other sharing one carbon atom. The nitrogen, carbon or sulfur atoms in heterocyclyl may be optionally oxidized; the nitrogen atom may be optionally quaternized; and the heterocyclyl may be partially or fully saturated. The heterocyclyl may be attached to the rest of the molecule via a carbon atom or a heteroatom and through a single bond. In some cases, the heterocyclyl may be carbon-linked, nitrogen-linked or sulfur-linked. In some embodiments, the heterocyclyl is carbon-linked. In some embodiments, heterocyclyl is nitrogen-linked. In some embodiments, heterocyclyl is sulfur-linked. Unless otherwise specifically specified in this specification, the heterocyclyl may be optionally substituted. Unless otherwise specifically specified in this specification, the heterocyclyl may be optionally substituted.

The heterocyclyl also comprises groups where said heterocyclyl moiety is fused to a saturated, partially unsaturated or fully unsaturated (i.e., aromatic) cyclohydrocarbyl, aryl, heterocyclyl or heteroaryl. In heterocyclyl comprising fused rings, one or more rings may be aryl or heteroaryl as defined below. Examples of fused heterocyclyl include, but are not limited to, phenyl-fused heterocyclyl, pyridyl-fused heterocyclyl, as well as quinolinyl, isoquinolinyl, quinoxalinyl, quinolizinyl, quinazolinyl, azaindolizinyl, pteridinyl, chromenyl, isochromenyl, indolyl, isoindolyl, indolizinyl, indazolyl, purinyl, benzofuranyl, isobenzofuranyl, benzimidazolyl, benzothienyl, benzothiazolyl, carbazolyl, phenazinyl, phenothiazinyl, phenanthridinyl, imidazo[1,2-a]pyridyl, [1,2,4]triazolo[4,3-a]pyridyl and [1,2,3]triazolo[4,3-a]pyridyl-fused heterocyclyl.

In some embodiments, the heterocyclyl is a stable 3- to 12-membered, 4- to 11-membered, 5-to 12-membered, 6- to 10-membered, 4- to 10-membered, 4- to 9-membered or 3- to 8-membered non-aromatic monocyclic, bicyclic, bridged or spiro ring group comprising 1 to 3 heteroatoms selected from nitrogen, oxygen and sulfur. In some embodiments, the heterocyclyl is a stable 3- to 10- membered (e.g., 3- to 8- membered, 5- to 10- membered or 4- to 9- membered) non-aromatic monocyclic, bicyclic, tricyclic or polycyclic group (including fused, bridged or spiro ring groups) comprising 1 to 3 heteroatoms selected from nitrogen, oxygen and sulfur. Non-limiting examples of the heterocyclyl include, but are not limited to: pyrrolidinyl, morpholinyl, piperazinyl, homopiperazinyl, piperidinyl, thiomorpholinyl, 2,7-diaza-spiro[3.5]nonan-7-yl, 2-oxa-6-aza-spiro[3.3]heptan-6-yl, 2-oxa-6-aza-spiro[3.4]octan-7-yl, 8-oxa-2-aza-spiro[4.5]decan-6-yl, 2,5-diaza-bicyclo[2.2.1]heptan-2-yl, azetidinyl, oxetanyl, thietanyl, thiolanyl, pyranyl, tetrahydropyranyl, thiopyranyl, tetrahydrofuranyl, oxazinyl, dioxolanyl, tetrahydroisoquinolinyl, decahydroisoquinolinyl, imidazolinyl, imidazolidinyl, quinolizinyl, thiazolidinyl, isothiazolidinyl, isoxazolidinyl, indolinyl, octahydroindolyl, octahydroisoindolyl, pyrrolidinyl, pyrazolidinyl, phthalimido, dioxothiomorpholinyl, dioxothiolanyl, dioxothietanyl, thianyl, dioxothianyl, thiomorpholinyl, 1,4-oxathianyl.

In the present disclosure, as a group or part of another group, the term "aryl" or "aromatic ring" refers to a conjugated hydrocarbon ring system group or moiety having 6 to 18 carbon atoms (e.g., 6 to 14 carbon atoms or 6 to 10 carbon atoms, such as 6, 7, 8, 9, 10, 11, 12, 13 or 14 carbon atoms). For the purposes of the present disclosure, aryl may be a monocyclic, bicyclic, tricyclic or polycyclic ring system, and may also be fused to cyclohydrocarbyl or heterocyclyl as defined above. Examples of aryl include, but are not limited to the following groups: phenyl, naphthyl, anthracenyl, phenanthryl, fluorenyl, 2,3-dihydro-1H-isoindolyl, 2-benzoxazolinone, 2H-1,4-benzoxazin-3(4H)-one-7-yl. Unless otherwise specifically specified in this specification, aryl may be optionally substituted.

In the present disclosure, as a group or part of another group, the term "heteroaryl" or "heteroaromatic ring" refers to a conjugated ring system group or moiety having carbon atoms (e.g., 1 to 15, 1 to 14, 1 to 13, 1 to 12, 1 to 11, 1 to 10 carbon atoms, such as 1, 2, 3, 4, 5, 6, 7, 8, 9 or 10 carbon atoms) and heteroatoms (e.g., 1 to 6 heteroatoms, such as 1, 2, 3, 4, 5 or 6 heteroatoms) selected from nitrogen, oxygen and sulfur in the ring. In some embodiments, the heteroaryl may comprise 5, 6, 7, 8, 9, 10, 11, 12, 13, 14, 15, 16, 17, 18, 19 or 20 ring-forming atoms, such as 5-20 ring-forming atoms, 5-19 ring-forming atoms, 5-18 ring-forming atoms, 5-17 ring-forming atoms, 5-16 ring-forming atoms, 5-15 ring-forming atoms, 5-14 ring-forming atoms, 5-13 ring-forming atoms, 5-12 ring-forming atoms, 5-10 ring-forming carbon atoms, 5-9 ring-forming atoms, 5-8 ring-forming atoms, 5-7 ring-forming atoms, or 5-6 ring-forming atoms. Unless otherwise specifically specified in this specification, the heteroaryl may be a monocyclic, bicyclic, tricyclic or polycyclic ring system, and may also be fused to cyclohydrocarbyl, aryl or heterocyclyl as defined above. Examples of heteroaryl fused to aryl include, but are not limited to, benzopyridyl, benzopyrazolyl, benzimidazolyl, benzopyrrolyl, or the like. The nitrogen, carbon or sulfur atoms in heteroaryl may be optionally oxidized; and the nitrogen atom may be optionally quaternized. For the purposes of the present disclosure, in some embodiments, heteroaryl is a stable 5- to 12- membered aromatic group comprising 1 to 5 (e.g., 1, 2, 3, 4 or 5) heteroatoms selected from nitrogen, oxygen and sulfur. In some embodiments, heteroaryl is a stable 5- to 10- membered aromatic group comprising 1 to 4 (e.g., 1, 2, 3 or 4) heteroatoms selected from nitrogen, oxygen and sulfur or a 5- to 6- membered aromatic group comprising 1, 2 or 3 heteroatoms selected from nitrogen, oxygen and sulfur. Unless otherwise specifically specified in this specification, heteroaryl may be optionally substituted.

Non-limiting examples of heteroaryl include, but are not limited to, thienyl, imidazolyl, pyrazolyl, thiazolyl, oxazolyl, oxadiazolyl, isoxazolyl, pyridyl, pyrimidinyl, pyrazinyl, pyridazinyl, benzimidazolyl, benzopyrazolyl, indolyl, furanyl, pyrrolyl, triazolyl, tetrazolyl, triazinyl, indolizinyl, isoindolyl, indazolyl, isoindazolyl, purinyl, quinolinyl, isoquinolinyl, diazanaphthalenyl, naphthyridinyl, quinoxalinyl, pteridinyl, carbazolyl, carbolinyl, phenanthridinyl, phenanthrolinyl, acridinyl, phenazinyl, isothiazolyl, benzothiazolyl, benzothienyl, oxatriazolyl, cinnolinyl, quinazolinyl, indolizinyl, phenanthrolinyl, isoxazolyl, phenoxazinyl, phenothiazinyl, 4,5,6,7-tetrahydrobenzo[b]thienyl, naphthopyridinyl, [1,2,4]triazolo[4,3-b]pyridazinyl, [1,2,4]triazolo[4,3-a]pyrazinyl, [1,2,4]triazolo[4,3-c]pyrimidinyl, [1,2,4]triazolo[4,3-a]pyridinyl, imidazo[1,2-a]pyridinyl, imidazo[1,2-b]pyridazinyl, imidazo[1,2-a]pyrazinyl.

In the present disclosure, as a group or part of another group, the term "heterocyclyl spiro heterocyclyl" or "heterocyclyl spiro heterocycle" refers to a fully saturated 5- to 20- membered (e.g., 5-, 6-, 7-, 8-, 9-, 10-, 11-, 12-, 13-, 14-, 15-, 16-, 17-, 18-, 19- or 20- membered) polycyclic ring where one or more ring atoms in the polycyclic ring are heteroatoms selected from sulfur, silicon, phosphorus, oxygen and/or nitrogen (e.g., 1 or 2 heteroatoms), and the remaining ring atoms are carbon atoms, wherein heterocyclyl groups share one carbon atom (called a spiro atom). Spiro heterocycles are classified into mono-spiro heterocycles, di-spiro heterocycles or poly-spiro heterocycles, according to the number of spiro atoms shared between rings. In some embodiments, heterocyclyl spiro heterocyclyl is mono-spiro heterocycle or di-spiro heterocycle. In some embodiments, heterocyclyl spiro heterocyclyl is 4-membered/4-membered, 4-membered/5-membered, 4-membered/6-membered, 5-membered/5-membered or 5-membered/6-membered mono-spiro heterocycle. Non-limiting examples of heterocycles include

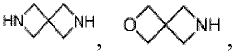

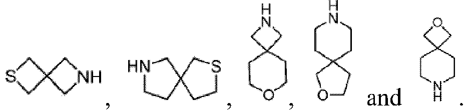

In the present disclosure, all cyclic groups may be present as substituents on the parent core, or themselves be part of a chain (including straight and branched chain) structure, the parent core or part of the parent core.

In the present disclosure, the term "heteroarylalkyl" or "heteroaralkyl" refers to an alkyl group as defined above substituted with a heteroaryl group as defined above.

In the present disclosure, "optionally" or "optional" means that the subsequently described event or condition may or may not occur, and the description comprises both instances where the event or condition occurs and where it does not occur. For example, "optionally substituted aryl" means that the aryl group is substituted or unsubstituted, and the description comprises both substituted and unsubstituted aryl groups. The "optional" substituents described in the claims and specification of the present disclosure are selected from alkyl, alkenyl, alkynyl, alkoxy, halogen, haloalkyl, haloalkenyl, haloalkynyl, cyano, hydroxyl, nitro, optionally substituted aryl, optionally substituted heteroaryl, optionally substituted cycloalkyl, and optionally substituted heterocyclyl.

In the present disclosure, the term "substituted", whether preceded by the term "optionally" (i.e., equivalent to substituted or unsubstituted) or not, means that one or more hydrogens on the specified group or moiety are replaced with "suitable substituents". Herein, the number of substituents may be one or more, i.e., 1, 2, 3, 4, 5 or 6 or more, depending on the nature of the group being substituted and the substituents. For example, according to the structure of the group being substituted, when the substituent of ethyl is halogen, the group may be substituted with 1, 2, 3, 4 or 5 substituents, such as trifluoroethyl, or pentafluoroethyl. In some embodiments, the number of substituents is 1, 2 or 3. In some embodiments, the number of substituents is 1 or 2. In some embodiments, the number of substituents is 1. It is understood that "substituted" or "substituted with" comprises the implicit condition that such substitution is in accordance with the allowed valency of the substituted atom, and that the substitution results in a stable or chemically feasible compound, e.g., a compound that does not undergo spontaneous conversion such as rearrangement, cyclization, elimination. Unless otherwise specified, an "optionally substituted" group may have suitable substituents at any substitutable position of the group, and when more than one position in any given structure may be substituted with more than one substituents selected from a specified group, the substituents may be the same or different at each position. Those skilled in the art will understand that substituents themselves may be substituted if appropriate. Unless specifically indicated as "unsubstituted", a chemical moiety mentioned herein shall be understood to comprise substituted variants. For example, reference to an "aryl" group or moiety implicitly comprises both unsubstituted aryl group and substituted variants.

Throughout this application, the above "suitable substituents" should be understood to include, but are not limited to, alkyl, alkenyl, alkynyl, halogen, haloalkyl, haloalkenyl, haloalkynyl, alkoxy, cyano, hydroxyl, amino, monoalkylamino, dialkylamino, nitro, aryl, heteroaryl, cyclohydrocarbyl (e.g., cycloalkyl, cycloalkenyl, or the like), or heterocyclyl, as described herein. These groups as substituents, including alkyl, alkenyl, alkynyl, alkyl in haloalkyl, alkenyl in haloalkenyl, alkynyl in haloalkynyl, alkoxy, alkyl in monoalkylamino, alkyl in dialkylamino, aryl, heteroaryl, cyclohydrocarbyl and heterocyclyl themselves are optionally substituted. For example, they may also be optionally substituted with one or more groups selected from alkyl, halogen, haloalkyl, alkoxy, hydroxyl, amino, monoalkylamino, dialkylamino, nitro, aryl, heteroaryl, cyclohydrocarbyl and heterocyclyl.

In some embodiments, the "suitable substituents" are selected from hydroxyl, halogen, C1-C4 alkoxy optionally substituted with halogen, C1-C4 alkoxycarbonyl, cyano, -S(O)₂-C1-C4 alkyl optionally substituted with halogen, C1-C4 alkyl optionally substituted with halogen, -NR'R", - S(O)₂-NR'R", -S(O)₂-NH₂, -C(O)-C1-C4 alkyl, -NR'-C(O)-C1-C4 alkyl, C3-C8 cyclohydrocarbyloxy, and 6- to 14- membered aryl, 5- to 10- membered heteroaryl, C3-C8 cyclohydrocarbyl and 4- to 9- membered heterocyclyl optionally substituted with 1, 2 or 3 substituents selected from C1-C4 alkyl, halogenated C1-C4 alkyl, C1-C4 alkoxy, halogenated C1-C4 alkoxy, halogen, cyano, C1-C4 alkyl-S(O)₂-, hydroxyl, carboxyl, -NR'R", 6- to 14- membered aryl, 5- to 10- membered heteroaryl, C3-C8 cyclohydrocarbyl and 4- to 9- membered heterocyclyl, wherein R' and R" are each independently selected from H and C1-C4 alkyl. In some embodiments, the "suitable substituents" are hydroxyl, amino, halogen, substituted or unsubstituted alkyl, -NR'R" and substituted or unsubstituted alkoxy, wherein R' and R" are each independently selected from H, C1-C4 alkyl, C1-C4 alkyl-S(O)₂- and halogenated C1-C4 alkyl. In some embodiments, the "suitable substituents" are selected from deuterium, hydroxyl, cyano, C1-C4 alkyl, halogen and C1-C4 alkoxy. In some embodiments, the "suitable substituents" are deuterium, hydroxyl, methyl, F and methoxy. In some embodiments, the "suitable substituents" are selected from hydroxyl, halogen, C1-C4 alkoxy, cyano and -S(O)₂-C1-C4 alkyl. In some embodiments, the "suitable substituents" are selected from halogen, hydroxyl, cyano, -S(O)₂-C1-C4 alkyl, C1-C4 alkyl and C1-C4 alkoxy. In some embodiments, the "suitable substituents" are selected from hydroxyl, C1-C4 alkyl, C1-C4 alkoxy and cyano. In some embodiments, the "suitable substituents" are F, hydroxyl and cyano.

The terms "moiety", "structural moiety", "chemical moiety", "radical", "chemical group" as used herein refer to a specific segment or functional group in a molecule. Chemical moieties are often recognized chemical entities embedded in or appended to a molecule.

Those skilled in the art should also understand that in the methods described below, functional groups of intermediate compounds may need to be protected with appropriate protecting groups. Such functional groups comprise hydroxyl, amino, amidino, guanidino, mercapto and carboxyl. Suitable protecting groups for hydroxyl comprise trialkylsilyl or diarylalkylsilyl (e.g., tert-butyldimethylsilyl, tert-butyldiphenylsilyl or trimethylsilyl), tetrahydropyranyl, benzyl, etc. Suitable protecting groups for amino, amidino and guanidino comprise tert-butoxycarbonyl, benzyloxycarbonyl, etc. Suitable protecting groups for mercapto comprise -C(O)-R^{X} (where R^{X} is alkyl, aryl or aralkyl), p-methoxybenzyl, triphenylmethyl, etc. Suitable protecting groups for carboxyl comprise alkyl esters, aryl esters or aralkyl esters.

"Stereoisomers" refer to compounds composed of the same atoms, bonded by the same bonds, but having different three-dimensional structures. The present disclosure covers all stereoisomers and mixtures thereof.

When the compounds of the present disclosure comprise an olefinic double bond, unless otherwise specified, the compounds of the present disclosure are intended to comprise E- and Z-geometric isomers.

"Tautomers" refer to isomers formed by transfer of a proton from one atom of a molecule to another atom of the same molecule. All tautomeric forms of the compounds of the present disclosure are also included within the scope of the present disclosure.

The compounds of the present disclosure or pharmaceutically acceptable salts thereof may comprise one or more chiral carbon atoms, and thus may give rise to enantiomers, diastereomers and other stereoisomeric forms. Each chiral carbon atom may be defined as (R)- or (S)- based on stereochemistry. The present disclosure is intended to comprise all possible isomers, as well as racemates and optically pure forms thereof. The compounds of the present disclosure may be prepared using racemates, diastereomers or enantiomers as starting materials or intermediates. Optically active isomers may be prepared using chiral synthons or chiral reagents, or resolved using conventional techniques such as crystallization and chiral chromatography.

Unless otherwise specified, "(D)" or "(+)" indicates dextrorotatory, "(L)" or "(-)" indicates levorotatory, and "(DL)" or "(±)" indicates racemic. Unless otherwise specified, wedge-shaped solid bonds ( 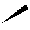 ) and wedge-shaped dashed bonds ( 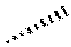 ) indicate the absolute configuration of a stereocenter.

Conventional techniques for preparation/isolation of individual isomers comprise chiral synthesis from a suitable optically pure precursor, or resolution of the racemate (or racemate of salts or derivatives) using, for example, chiral high-performance liquid chromatography. See e.g., Gerald Gübitz and Martin G. Schmid (Eds.), Chiral Separations, Methods and Protocols, Methods in Molecular Biology, Vol. 243, 2004; A.M. Stalcup, Chiral Separations, Annu. Rev. Anal. Chem. 3:341-63, 2010; Fumiss et al. (eds.), VOGEL'S ENCYCLOPEDIA OF PRACTICAL ORGANIC CHEMISTRY 5.sup.TH ED., Longman Scientific and Technical Ltd., Essex, 1991, 809-816; Heller, Acc. Chem. Res. 1990, 23, 128, the disclosures of which are incorporated herein by reference in their entirety.

The compounds of the present disclosure also comprise all suitable isotopically substituted derivatives (or isotopic variants) of pharmaceutically acceptable salts thereof. Isotopic variants of the compounds of the present disclosure or pharmaceutically acceptable salts thereof are defined as those in which at least one atom is replaced by an atom having the same atomic number but an atomic mass different from that normally found in nature. Isotopes that can be incorporated into the compounds of the present disclosure and pharmaceutically acceptable salts thereof include, but are not limited to, isotopes of H, C, N and O, such as ²H, ³H, ¹¹C, ¹³C, ¹⁴C, ¹⁵N, ¹⁷O, ¹⁸O, ³⁵S, ¹⁸F, ³⁶Cl and ¹²⁵I. Isotopic variants of the compounds described herein or pharmaceutically acceptable salts thereof can be prepared by conventional techniques using appropriate isotopic variants of suitable reagents.

Herein, unless otherwise specified, a wavy line on each structural formula or group generally indicates the position where the structural formula or group is connected to other parts of the compound.

As used herein, "solvate" refers to a compound that comprises a stoichiometric or non-stoichiometric amount of solvent, often formed during crystallization. Solvates comprise aggregates of one or more molecules of the compounds of the present disclosure with one or more solvent molecules. Hydrates are formed when the solvent is water. Alternatively, the solvent may be an organic solvent (e.g., alcoholates are formed when the solvent is alcohol). Thus, the compounds of the present disclosure may exist as hydrates, comprising monohydrates, dihydrates, hemihydrates, sesquihydrates, trihydrates, tetrahydrates, etc., as well as corresponding solvated forms. The compounds of the present disclosure may be true solvates, while in other cases, the compounds of the present disclosure may merely retain non-stoichiometric water or a mixture of water plus some non-stoichiometric solvate.

As used herein, the term "polymorph" refers to different crystal packing arrangements (all having the same elemental composition) consisting of the same elemental of a compound (or a salt, solvate or other derivatives thereof such as a prodrug or metabolite). Polymorphs typically have different X-ray diffraction patterns, infrared spectra, melting points, density, hardness, crystal forms, optical or electrical properties, stability and solubility. Various factors such as recrystallization solvent, crystallization rate and storage temperature can cause a single crystal form to predominate.

In the present disclosure, the term "pharmaceutically acceptable salt" comprises pharmaceutically acceptable acid addition salts and pharmaceutically acceptable base addition salts.

"Pharmaceutically acceptable acid addition salt" refers to a salt of a free base formed with an inorganic acid or an organic acid, which can retain the biological effectiveness of the free base without other side effects. Inorganic acid salts include, but are not limited to, hydrochlorides, hydrobromides, sulfates, nitrates, phosphates or the like; organic acid salts include, but are not limited to, formates, acetates, 2,2-dichloroacetates, trifluoroacetates, propionates, hexanoates, octanoates, decanoates, undecylenates, glycolates, gluconates, lactates, sebacates, adipates, glutarates, malonates, oxalates, maleates, succinates, fumarates, tartrates, citrates, palmitates, stearates, oleates, cinnamates, laurates, malates, glutamates, pyroglutamates, aspartates, benzoates, methanesulfonates, benzenesulfonates, p-toluenesulfonates, alginates, ascorbates, salicylates, 4-aminosalicylates, naphthalene disulfonates or the like. These salts can be prepared by methods known in the art.

"Pharmaceutically acceptable base addition salt" refers to a salt of a free acid formed with an inorganic base or an organic base that can retain the biological effectiveness of the free acid without other side effects. Salts derived from inorganic bases include, but are not limited to, sodium salts, potassium salts, lithium salts, ammonium salts, calcium salts, magnesium salts, iron salts, zinc salts, copper salts, manganese salts, aluminum salts, and the like. Preferred inorganic salts are slats of ammonium, sodium, potassium, calcium and magnesium. Salts derived from organic bases include, but are not limited to, those of primary amines, secondary amines and tertiary amines, substituted amines comprising naturally occurring substituted amines, cyclic amines and basic ion exchange resins, such as ammonia, isopropylamine, trimethylamine, diethylamine, triethylamine, tripropylamine, ethanolamine, diethanolamine, triethanolamine, dimethylethanolamine, 2-dimethylaminoethanol, 2-diethylaminoethanol, dicyclohexylamine, lysine, arginine, histidine, caffeine, procaine, choline, betaine, ethylenediamine, glucosamine, methylglucamine, theobromine, purine, piperazine, piperidine, N-ethylpiperidine, polyamine resins, or the like. Preferred organic bases include isopropylamine, diethylamine, ethanolamine, trimethylamine, dicyclohexylamine, choline and caffeine. These salts can be prepared by methods known in the art.

As used herein, the term "prodrug" refers to those compounds that are metabolized (i.e., transformed in vivo) into the compounds of the present disclosure having pharmacological activity after administration. When the compounds of the present disclosure themselves are difficult to absorb through the gastrointestinal tract, their bioavailability can be improved by making them into prodrugs. Examples of prodrugs of the compounds of the present disclosure may comprise simple esters of compounds comprising carboxyl groups (e.g., esters obtained by condensation with C1-C4 alcohols according to methods known in the art); esters of compounds comprising hydroxyl groups (e.g., esters obtained by condensation with C1-C4 monocarboxylic acids, C3-C6 dicarboxylic acids or anhydrides thereof such as succinic anhydride or fumaric anhydride according to methods known in the art); imines of compounds comprising amino groups (e.g., imines obtained by condensation with C1-C4 aldehydes or ketones according to methods known in the art); carbamates of compounds comprising amino groups, such as those described by Leu et al. (J. Med. Chem., 42:3623-3628 (1999)) and Greenwald et al. (J. Med. Chem., 42:3657-3667 (1999)); acetals or ketals of compounds comprising hydroxyl groups (e.g., those obtained by condensation with chloromethyl methyl ether or chloromethyl ethyl ether according to methods known in the art).

In the present disclosure, "pharmaceutical composition" refers to a formulation of a compound of the present disclosure or a pharmaceutically acceptable salt, enantiomer, diastereomer, tautomer, solvate, isotopically substituted derivative, polymorph, prodrug or metabolite thereof with a medium generally accepted in the art for delivering biologically (pharmacologically) active compounds to mammals (e.g., humans). The medium comprises pharmaceutically acceptable carriers or excipients. The purpose of a pharmaceutical composition is to facilitate administration to an organism, facilitate absorption of the active ingredient and thereby exert biological (pharmacological) activity. Generally, the pharmaceutical compositions of the present disclosure comprise 0.1% by weight to 99.5% by weight of the pharmaceutically active ingredient (i.e., the compound of the present disclosure or a pharmaceutically acceptable salt, enantiomer, diastereomer, tautomer, solvate, isotopically substituted derivative, polymorph, prodrug or metabolite thereof). In some embodiments, the pharmaceutical compositions of the present disclosure comprise 0.5% by weight to 90% by weight, such as 1% by weight, 1.5% by weight, 2% by weight, 5% by weight, 10% by weight, 15% by weight, 20% by weight, 25% by weight, 30% by weight or 50% by weight of the pharmaceutically active ingredient.

The term "pharmaceutically acceptable" as used herein refers to substances (such as carriers or diluents) that do not affect the biological activity or properties of the compounds of the present disclosure and are relatively non-toxic, i.e., the substances can be administered to an individual without causing adverse biological reactions or interacting in an adverse manner with any components contained in the composition.

In the present disclosure, "pharmaceutically acceptable excipient" includes, but is not limited to, any adjuvants, carriers, excipients, glidants, sweeteners, diluents, preservatives, dyes/colorants, flavoring agents, surfactants, wetting agents, dispersants, suspending agents, stabilizers, isotonic agents, solvents or emulsifiers approved by relevant government regulatory agencies as acceptable for human or domestic animal use.

The terms "prophylactic", "prevention" and "preventing" comprise reducing the likelihood of the occurrence or worsening of a disease or condition in a patient. "Prophylactic", "prevention" and "preventing" refer to preventing the disease or condition from occurring in a mammal (preferably a human), especially when the mammal is susceptible to the condition but has not yet been diagnosed with the condition, or preventing the recurrence of a cured disease or condition in a mammal (preferably a human).

As used herein, the term "treatment" encompasses the treatment of a disease or condition of interest in a mammal (preferably a human) having the disease or condition of interest, comprising one or more of the following:
(i) inhibiting the disease or condition, i.e., arresting its development;
(ii) alleviating the disease or condition, i.e., causing regression of the state of the disease or condition; or
(iii) ameliorating the symptoms caused by the disease or condition, i.e., relieving pain without addressing the underlying disease or condition.

As used herein, the terms "effective amount", "therapeutically effective amount" or "pharmaceutically effective amount" refer to an amount of at least one agent or compound that, upon administration, is sufficient to alleviate, to some extent, one or more symptoms of the disease or condition being treated. The result may be reduction and/or alleviation of signs, symptoms, or causes, or any other desired change in a biological system. For example, an "effective amount" for therapeutic use is the amount of a composition comprising a compound disclosed herein that is required to achieve clinically significant symptom relief. An effective amount for any individual case can be determined using techniques such as dose escalation trials.

As used herein, the terms "administering", "administration" and "administrate" refer to methods that enable delivery of a compound or pharmaceutical composition to the desired site for biological action. Any administration method well known in the art can be used for the present disclosure. These methods include, but are not limited to, oral administration, intraduodenal administration, parenteral injection (comprising intrapulmonary, intranasal administration; intrathecal, intravenous, subcutaneous, intraperitoneal, intramuscular, intraarterial injection or infusion), topical administration, and rectal administration. Those skilled in the art are familiar with administration techniques applicable to the compounds and methods described herein, such as those discussed in Goodman and Gilman, The Pharmacological Basis of Therapeutics, current ed.; Pergamon; and Remington's, Pharmaceutical Sciences (current edition), Mack Publishing Co., Easton, Pa. In preferred embodiments, the compounds discussed herein, pharmaceutically acceptable salts, enantiomers, diastereomers, tautomers, solvates, isotopically substituted derivatives, polymorphs, prodrugs or metabolites thererof, or pharmaceutical compositions thereof, are administered orally.

As used herein, the terms "pharmaceutical combination", "pharmaceutical composition", "combination", "drug combination", "combination therapy", "administration of other therapy", "administration of other therapeutic agent" and the like refer to a pharmaceutical treatment obtained by mixing or combining more than one active ingredient, comprising fixed and non-fixed combinations of active ingredients. The term "fixed combination" means that at least one compound described herein and at least one other active ingredient are administered simultaneously to a patient in a form of a single entity or a single dosage. The term "non-fixed combination" means that at least one compound described herein and at least one other active ingredient are administered as separate entities concurrently, concomitantly, or sequentially with variable intervening time periods to a patient. These also apply to cocktail therapy, e.g., administration of three or more active ingredients.

Those skilled in the art also understand that in the methods described below, functional groups of intermediate compounds may need to be protected with appropriate protecting groups. Such functional groups comprise hydroxyl, amino, mercapto and carboxyl. Suitable protecting groups for hydroxyl comprise trialkylsilyl or diarylalkylsilyl (e.g., tert-butyldimethylsilyl, tert-butyldiphenylsilyl or trimethylsilyl), tetrahydropyranyl, benzyl, etc. Suitable protecting groups for amino, amidino and guanidino comprise tert-butoxycarbonyl, benzyloxycarbonyl, etc. Suitable protecting groups for mercapto comprise -C(O)-R" (where R" is alkyl, aryl or aralkyl), p-methoxybenzyl, triphenylmethyl, etc. Suitable protecting groups for carboxyl comprise alkyl, aryl or aralkyl esters.

Protecting groups can be introduced and removed according to standard techniques known to those skilled in the art and as described herein. The use of protecting groups is detailed in Greene, T. W. and P. G. M. Wuts, Protective Groups in Organic Synthesis, (1999), 4th Ed., Wiley. Protecting groups can also be polymeric resins.

### Compound of formula A

The present disclosure provides a compound of Formula A having the structure shown below, or a pharmaceutically acceptable salt thereof, or an enantiomer, diastereomer, tautomer, solvate, polymorph, prodrug or metabolite thereof, 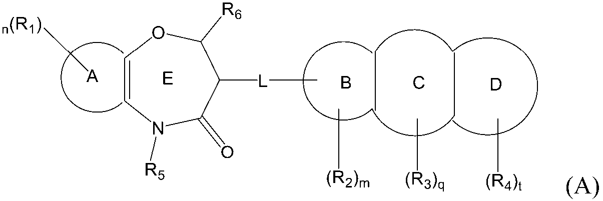 wherein,
ring A is selected from: substituted or unsubstituted C₃₋₈ cycloalkyl, substituted or unsubstituted 3- to 8- membered heterocyclyl, substituted or unsubstituted aryl, substituted or unsubstituted heteroaryl, wherein the heterocyclyl or the heteroaryl comprises 1, 2, or 3 heteroatoms selected from the group consisting of N, O, S, and P;
L is selected from : 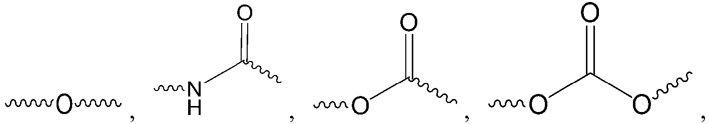 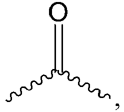 C₁₋₁₀ alkylene, or a chemical bond;
when L is 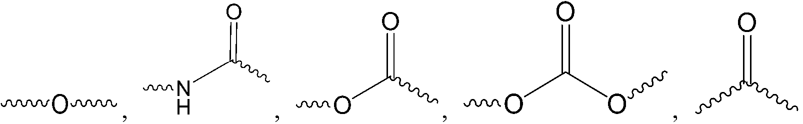 or C₁₋₁₀ alkylene, ring B is selected from: substituted or unsubstituted C₃₋₈ cycloalkyl, substituted or unsubstituted 3- to 8- membered heterocyclyl, substituted or unsubstituted aryl, substituted or unsubstituted heteroaryl, wherein the heterocyclyl or the heteroaryl comprises 1, 2, or 3 heteroatoms selected from the group consisting of N, O, S, and P; ring C and ring D are each independently selected from: substituted or unsubstituted C₃₋₈ cycloalkyl, substituted or unsubstituted 3- to 8-membered heterocyclyl, substituted or unsubstituted C₆₋₁₀ aryl, substituted or unsubstituted 5- to 10- membered heteroaryl, or absent, wherein the heterocyclyl or the heteroaryl comprises 1, 2, or 3 heteroatoms selected from the group consisting of N, O, S, and P;
when L is a chemical bond, ring E is directly connected to ring B via the chemical bond, and ring B, ring C and ring D are each independently selected from: substituted or unsubstituted C₃₋₈ cycloalkyl, substituted or unsubstituted heterocyclyl, substituted or unsubstituted aryl, substituted or unsubstituted heteroaryl, wherein the heterocyclyl or the heteroaryl comprises 1, 2 or 3 heteroatoms selected from the group consisting of N, O, S and P;
R₁ is selected from H, halogen, substituted or unsubstituted C₂₋₆ alkynyl, substituted or unsubstituted C₂₋₆ alkenyl, substituted or unsubstituted C₁₋₆ alkyl, substituted or unsubstituted C₁₋₆ alkoxy, cyano, -NO₂, -OH, substituted or unsubstituted C₃₋₈ cycloalkyl, substituted or unsubstituted 3- to 8- membered heterocyclyl, substituted or unsubstituted C₆₋₁₀ aryl, substituted or unsubstituted 5- to 10- membered heteroaryl, substituted or unsubstituted 3- to 8- membered heterocyclyl spiro 3- to 8- membered heterocyclyl, substituted or unsubstituted 3- to 8- membered heterocyclyl fused 3- to 8- membered heterocyclyl, substituted or unsubstituted C₃₋₈ cycloalkyl spiro C₃₋₈ cycloalkyl, substituted or unsubstituted C₃₋₈ cycloalkyl fused C₃₋₈ cycloalkyl, substituted or unsubstituted 3- to 8- membered heterocyclyl fused C₆₋₁₀ aryl, substituted or unsubstituted 3- to 8- membered heterocyclyl fused 5- to 10- membered heteroaryl, substituted or unsubstituted C₃₋₈ cycloalkyl fused C₆₋₁₀ aryl, substituted or unsubstituted C₃₋₈ cycloalkyl fused 5- to 10- membered heteroaryl, wherein the heterocyclyl or heteroaryl comprises 1, 2 or 3 heteroatoms selected from the group consisting of N, O, S and P;
R₂ is selected from H, oxo (=O), substituted or unsubstituted C₁₋₆ alkoxy, halogen, substituted or unsubstituted C₂₋₆ alkynyl, substituted or unsubstituted C₂₋₆ alkenyl, substituted or unsubstituted C₁₋₆ alkyl, cyano, -NO₂, -OH, substituted or unsubstituted C₃₋₈ cycloalkyl, substituted or unsubstituted 3- to 8- membered heterocyclyl, substituted or unsubstituted C₆₋₁₀ aryl, substituted or unsubstituted 5- to 10- membered heteroaryl, wherein the heterocyclyl or the heteroaryl comprises 1, 2 or 3 heteroatoms selected from the group consisting of N, O, S and P;
R₃ is selected from H, oxo, substituted or unsubstituted C₁₋₆ alkoxy, halogen, substituted or unsubstituted C₂₋₆ alkynyl, substituted or unsubstituted C₂₋₆ alkenyl, substituted or unsubstituted C₁₋₆ alkyl, cyano, -NO₂, -OH, substituted or unsubstituted C₃₋₈ cycloalkyl, substituted or unsubstituted 3- to 8- membered heterocyclyl, substituted or unsubstituted C₆₋₁₀ aryl, substituted or unsubstituted 5- to 10- membered heteroaryl, wherein the heterocyclyl or the heteroaryl comprises 1, 2 or 3 heteroatoms selected from the group consisting of N, O, S and P;
R₄ is selected from H, oxo, substituted or unsubstituted C₁₋₆ alkoxy, halogen, substituted or unsubstituted C₂₋₆ alkynyl, substituted or unsubstituted C₂₋₆ alkenyl, substituted or unsubstituted C₁₋₆ alkyl, cyano, -NO₂, -OH, substituted or unsubstituted C₃₋₈ cycloalkyl, substituted or unsubstituted 3- to 8- membered heterocyclyl, substituted or unsubstituted C₆₋₁₀ aryl, substituted or unsubstituted 5- to 10- membered heteroaryl, wherein the heterocyclyl or the heteroaryl comprises 1, 2 or 3 heteroatoms selected from the group consisting of N, O, S and P;
R₅ is selected from H, halogen, substituted or unsubstituted C₂₋₆ alkynyl, substituted or unsubstituted C₂₋₆ alkenyl, substituted or unsubstituted C₁₋₆ alkyl, substituted or unsubstituted C₁₋₆ alkoxy, cyano, -NO₂, -OH, substituted or unsubstituted C₃₋₈ cycloalkyl, substituted or unsubstituted 3- to 8- membered heterocyclyl, substituted or unsubstituted C₆₋₁₀ aryl, substituted or unsubstituted 5- to 10- membered heteroaryl, wherein the heterocyclyl or the heteroaryl comprises 1, 2 or 3 heteroatoms selected from the group consisting of N, O, S and P;
R₆ is selected from H, halogen, substituted or unsubstituted C₂₋₆ alkynyl, substituted or unsubstituted C₂₋₆ alkenyl, substituted or unsubstituted C₁₋₆ alkyl, substituted or unsubstituted C₁₋₆ alkoxy, cyano, -NO₂, -OH, substituted or unsubstituted C₃₋₈ cycloalkyl, substituted or unsubstituted 3- to 8- membered heterocyclyl, substituted or unsubstituted C₆₋₁₀ aryl, substituted or unsubstituted 5- to 10- membered heteroaryl, wherein the heterocyclyl or the heteroaryl comprises 1, 2 or 3 heteroatoms selected from the group consisting of N, O, S and P;
n, m, q and t are each independently any integer selected from 0, 1, 2 and 3;
the "substituted" means that one or more hydrogen atoms on the group are substituted with substituents selected from the group consisting of: halogen, -OH, -NO₂, -NH₂, -NH(unsubstituted or halogenated C₁₋₆ alkyl), -N(unsubstituted or halogenated C₁₋₆ alkyl)₂, -CN, unsubstituted C₁₋₈ alkyl or C₁₋₈ alkyl substituted with halogen or hydroxy, unsubstituted or halogenated C₁₋₈ alkoxy, unsubstituted or halogenated C₁₋₈ alkoxy-C₁₋₈ alkyl, unsubstituted or halogenated C₃₋₈ cycloalkyl-C₁₋₈ alkyl, unsubstituted or halogenated C₁₋₆ alkylcarbonyl, unsubstituted or halogenated C₁₋₆ alkoxycarbonyl, hydroxamic acid group, unsubstituted or halogenated C₁₋₆ alkylthio, - S(O)₂N(unsubstituted or halogenated C₁₋₆ alkyl)₂, -S(O)₂ unsubstituted or halogenated C₁₋₆ alkyl, - N(unsubstituted or halogenated C₁₋₆ alkyl)S(O)₂N(unsubstituted or halogenated C₁₋₆ alkyl)₂, - S(O)N(unsubstituted or halogenated C₁₋₆ alkyl)₂, -S(O)(unsubstituted or halogenated C₁₋₆ alkyl), - N(unsubstituted or halogenated C₁₋₆ alkyl)S(O)N(unsubstituted or halogenated C₁₋₆ alkyl)₂, - N(unsubstituted or halogenated C₁₋₆ alkyl)S(O)(unsubstituted or halogenated C₁₋₆ alkyl), unsubstituted or halogenated C₅₋₁₀ aryl, unsubstituted or halogenated 5- to 10- membered heteroaryl, unsubstituted or halogenated C₃₋₈ cycloalkyl and unsubstituted or halogenated 3- to 8- membered heterocyclyl, wherein the heterocyclyl and the heteroaryl comprise 1, 2, 3 or 4 heteroatoms selected from the group consisting of N, O and S;
" 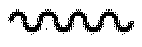 " represents the position connected to other parts of the molecule via a chemical bond.

In some embodiments, ring A is substituted or unsubstituted aryl. Ring A is 6- to 14-membered aryl, 6- to 12-membered aryl, 6- to 10-membered aryl, 6- to 8-membered aryl. In certain embodiments, ring A is phenyl or naphthyl. In certain embodiments, ring A is phenyl.

In some embodiments, ring A is substituted or unsubstituted heteroaryl. In some embodiments, ring A is selected from 5- to 14-membered heteroaryl, 5- to 12-membered heteroaryl, 5- to 10-membered heteroaryl, 5- to 8-membered heteroaryl, 5- to 6-membered heteroaryl. In certain embodiments, ring A is selected from thienyl, imidazolyl, pyrazolyl, thiazolyl, oxazolyl, pyridinyl, pyrimidinyl, pyrazinyl, pyridazinyl, furanyl, pyrrolyl, triazolyl, triazinyl, imidazolyl, pyrazolyl, pyrrolyl, pyridinyl, pyrimidinyl, or pyrazinyl. In certain embodiments, ring A is pyridinyl.

In some embodiments, ring B is substituted or unsubstituted heterocyclyl. Ring B is selected from 5- to 14-membered heterocyclyl, 5- to 12-membered heterocyclyl, 5- to 10-membered heterocyclyl, 5- to 8-membered heterocyclyl, 5- to 6-membered heterocyclyl. In certain embodiments, ring B is selected from pyrrolidinyl, imidazolidinyl, tetrahydrofuranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl, pyranyl. In certain embodiments, ring B is piperidinyl.

In some embodiments, ring B is substituted or unsubstituted heteroaryl. In some embodiments, ring B is selected from 5- to 14-membered heteroaryl, 5- to 12-membered heteroaryl, 5- to 10-membered heteroaryl, 5- to 8-membered heteroaryl, 5- to 6-membered heteroaryl. In certain embodiments, ring B is selected from thienyl, imidazolyl, pyrazolyl, thiazolyl, oxazolyl, pyridinyl, pyrimidinyl, pyrazinyl, azinyl, furanyl, pyrrolyl, triazolyl, triazinyl, pyridonyl. In certain embodiments, ring B is pyrazolyl or pyridinyl.

In some embodiments, ring C is substituted or unsubstituted heteroaryl. In some embodiments, ring C is selected from 5- to 14-membered heteroaryl, 5- to 12-membered heteroaryl, 5- to 10-membered heteroaryl, 5- to 8-membered heteroaryl, 5- to 6-membered heteroaryl. In some embodiments, ring C is selected from thienyl, imidazolyl, pyrazolyl, thiazolyl, oxazolyl, pyridinyl, pyrimidinyl, pyrazinyl, azinyl, furanyl, pyrrolyl, triazolyl, triazinyl, pyridonyl. In certain embodiments, ring C is pyrazolyl.

In some embodiments, ring C is substituted or unsubstituted heterocyclyl. Ring C is selected from 5- to 14-membered heterocyclyl, 5- to 12-membered heterocyclyl, 5- to 10-membered heterocyclyl, 5- to 8-membered heterocyclyl, 5- to 6-membered heterocyclyl. In certain embodiments, ring C is selected from pyrrolidinyl, imidazolidinyl, tetrahydrofuranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl, pyranyl. In certain embodiments, ring C is pyrrolidinyl.

In some embodiments, ring D is substituted or unsubstituted heterocyclyl. Ring D is selected from 5- to 14-membered heterocyclyl, 5- to 12-membered heterocyclyl, 5- to 10-membered heterocyclyl, 5- to 8-membered heterocyclyl, 5- to 6-membered heterocyclyl. In certain embodiments, ring D is selected from pyrrolidinyl, imidazolidinyl, tetrahydrofuranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl, pyranyl or oxazacyclohexane. In certain embodiments, ring D is pyrrolidinyl or 1,3-oxazacyclohexyl. In certain embodiments, ring D is pyrrolidinyl. In certain embodiments, ring D is 1,3-oxazacyclohexyl or morpholinyl. In certain embodiments, ring D is 1,3-oxazacyclohexyl.

In some embodiments, R₁ is selected from H, halogen, substituted or unsubstituted C₂₋₆ alkynyl, substituted or unsubstituted C₂₋₆ alkenyl, substituted or unsubstituted C₁₋₆ alkoxy, cyano, substituted or unsubstituted 3- to 8-membered heterocyclyl spiro 3- to 8-membered heterocyclyl, wherein the heterocyclyl comprises 1, 2, or 3 heteroatoms selected from the group consisting of N, O, S. In certain embodiments, R₁ is selected from H, halogen, substituted or unsubstituted C₂₋₅ alkynyl, substituted or unsubstituted C₁₋₃ alkoxy, cyano, substituted or unsubstituted 5- to 6-membered heterocyclyl spiro 4- to 6-membered heterocyclyl, wherein the heterocyclyl comprises 1 or 2 heteroatoms selected from the group consisting of N, O, S. In certain embodiments, said C₂₋₅ alkynyl, C₁₋₃ alkoxy, and 5- to 6-membered heterocyclyl spiro 4- to 6-membered heterocyclyl are substituted with substituents selected from C₁₋₆ alkyl, 3- to 5-membered cycloalkyl, 4- to 6-membered heterocyclyl, hydroxyC₂₋₅ alkyl. In certain embodiments, said C₂₋₅ alkynyl, C₁₋₃ alkoxy, and 5- to 6-membered heterocyclyl spiro 4- to 6-membered heterocyclyl are substituted with substituents selected from cyclopropyl, pyranyl, hydroxyisopropyl.

In some embodiments, R₁ is selected from:
H, 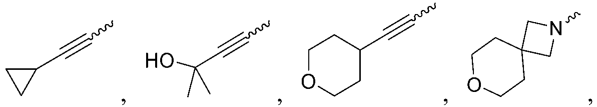 methoxy, cyano, chloro, and bromo.

In some embodiments, R₂ is selected from H, oxo (=O), substituted or unsubstituted C₁₋₆ alkoxy, halogen, substituted or unsubstituted C₁₋₆ alkyl, cyano, -NO₂, -OH, substituted or unsubstituted C₆₋₁₀ aryl, substituted or unsubstituted 5- to 14-membered heteroaryl, wherein the heteroaryl comprises 1, 2, or 3 heteroatoms selected from the group consisting of N, O, S, and P. In certain embodiments, said C₁₋₆ alkoxy and C₁₋₆ alkyl are each independently substituted with oxo (=O), C₆₋₁₀ aryl, -OH, cyano, or -NO₂. In certain embodiments, R₂ is selected from H, oxo (=O), substituted or unsubstituted C₁₋₆ alkoxy optionally substituted with C₆₋₁₀ ary, halogen, substituted or unsubstituted 5- to 10-membered heteroaryl, wherein the heteroaryl comprises 1, 2, or 3 heteroatoms selected from the group consisting of N, O, and S. In certain embodiments, R₂ is H, oxo, C₁₋₄ alkoxy substituted with C₆₋₁₀ aryl, benzopyrazolyl.

In some embodiments, R₃ is selected from: H, substituted or unsubstituted C₆₋₁₀ aryl, substituted or unsubstituted C₁₋₆ alkoxy, halogen, cyano, -NO₂, -OH. In certain embodiments, said C₁₋₆ alkoxy and C₁₋₆ alkyl are each independently substituted with oxo (=O), -OH, cyano, or -NO₂. In certain embodiments, R₃ is H or C₆₋₁₀ aryl.

In some embodiments, R₄ is selected from H, substituted or unsubstituted C₁₋₆ alkoxy, halogen, substituted or unsubstituted C₁₋₆ alkyl, cyano, -NO₂, -OH, substituted or unsubstituted 3- to 8-membered heterocyclyl, substituted or unsubstituted C₆₋₁₀ aryl, substituted or unsubstituted 5- to 10-membered heteroaryl, wherein the heterocyclyl or the heteroaryl comprises 1, 2, or 3 heteroatoms selected from the group consisting of N, O, and S. In certain embodiments, said C₁₋₆ alkoxy, C₁₋₆ alkyl, 3- to 8-membered heterocyclyl, C₆₋₁₀ aryl, 5- to 10-membered heteroaryl are each independently substituted with 1, 2, or 3 substituents selected from H, halogen, -OH, C₁₋₆ alkyl, halogenated C₁₋₆ alkyl, C₁₋₈ alkoxy. In certain embodiments, t is 1 or 2.

In certain embodiments, R₄ is selected from halogen, difluoropropyl, phenyl, benzyl, difluorophenyl, methylphenyl, methoxyphenyl, and pyridinyl. In certain embodiments, R₄ is selected from: 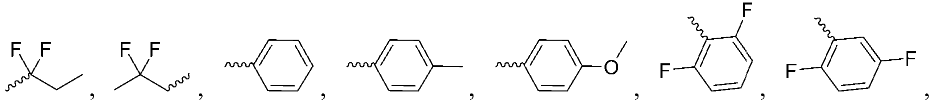 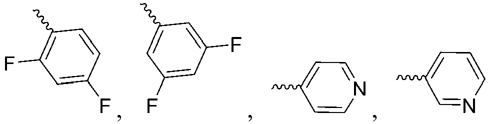 and halogen.

In certain embodiments, R₄ is selected from: 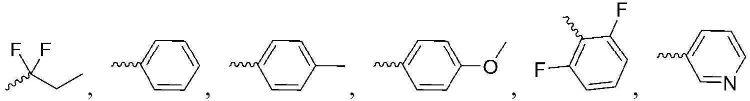 and F.

In some embodiments, R₅ is selected from H, substituted or unsubstituted C₁₋₆ alkoxy, halogen, substituted or unsubstituted C₁₋₆ alkyl. In certain embodiments, R₅ is selected from unsubstituted C₁₋₆ alkyl. In certain embodiments, R₅ is methyl.

In some embodiments, R₆ is selected from H, substituted or unsubstituted C₁₋₆ alkoxy, halogen, substituted or unsubstituted C₁₋₆ alkyl. In certain embodiments, R₆ is H.

In some embodiments, the compound of Formula A provided in the present disclosure, or a pharmaceutically acceptable salt thereof, or an enantiomer, diastereomer, tautomer, solvate, polymorph, prodrug or metabolite thereof, has the structure of Formula I below: 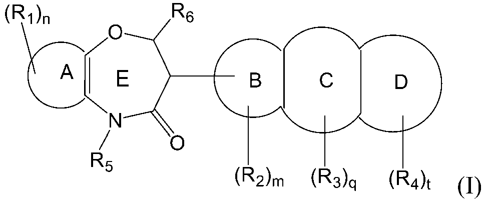 in Formula I:
ring A, ring B, ring C, and ring D are each independently selected from: substituted or unsubstituted C₃₋₈ cycloalkyl, substituted or unsubstituted 3- to 8-membered heterocyclyl, substituted or unsubstituted C₆₋₁₀ aryl, substituted or unsubstituted 5- to 10-membered heteroaryl, wherein the heterocyclyl or the heteroaryl comprises 1, 2, or 3 heteroatoms selected from the group consisting of N, O, S, and P;
R₁ is selected from: H, halogen, substituted or unsubstituted C₂₋₆ alkynyl, substituted or unsubstituted C₂₋₆ alkenyl, substituted or unsubstituted C₁₋₆ alkyl, substituted or unsubstituted C₁₋₆ alkoxy, cyano, -NO₂, -OH, substituted or unsubstituted C₃₋₈ cycloalkyl, substituted or unsubstituted 3- to 8- membered heterocyclyl, substituted or unsubstituted C₆₋₁₀ aryl, substituted or unsubstituted 5- to 10- membered heteroaryl, substituted or unsubstituted 3- to 8- membered heterocyclyl spiro 3- to 8- membered heterocyclyl, substituted or unsubstituted 3- to 8- membered heterocyclyl fused 3- to 8- membered heterocyclyl, substituted or unsubstituted C₃₋₈ cycloalkyl spiro C₃₋₈ cycloalkyl, substituted or unsubstituted C₃₋₈ cycloalkyl fused C₃₋₈ cycloalkyl, substituted or unsubstituted 3- to 8- membered heterocyclyl fused C₆₋₁₀ aryl, substituted or unsubstituted 3- to 8- membered heterocyclyl fused 5- to 10- membered heteroaryl, substituted or unsubstituted C₃₋₈ cycloalkyl fused C₆₋₁₀ aryl, substituted or unsubstituted C₃₋₈ cycloalkyl fused 5- to 10- membered heteroaryl, wherein the heterocyclyl or the heteroaryl comprises 1, 2 or 3 heteroatoms selected from the group consisting of N, O, S and P;
R₂, R₃, R₄, R₅, and R₆ are each independently selected from: H, oxo (=O), substituted or unsubstituted C₁₋₆ alkoxy, halogen, substituted or unsubstituted C₂₋₆ alkynyl, substituted or unsubstituted C₂₋₆ alkenyl, substituted or unsubstituted C₁₋₆ alkyl, cyano, -NO₂, -OH, substituted or unsubstituted C₃₋₈ cycloalkyl, substituted or unsubstituted 3- to 8-membered heterocyclyl, substituted or unsubstituted C₆₋₁₀ aryl, substituted or unsubstituted 5- to 10-membered heteroaryl, wherein the heterocyclyl or the heteroaryl comprises 1, 2, or 3 heteroatoms selected from the group consisting of N, O, S, and P;
n, m, q, and t are each independently any integer selected from 0, 1, 2, or 3;
the "substituted" means that one or more hydrogen atoms on the group are substituted with substituents selected from the group consisting of: halogen, -OH, -NO₂, -NH₂, -NH(unsubstituted or halogenated C₁₋₆ alkyl), -N(unsubstituted or halogenated C₁₋₆ alkyl)₂, -CN, unsubstituted C₁₋₈ alkyl or C₁₋₈ alkyl substituted with halogen or hydroxy, unsubstituted or halogenated C₁₋₈ alkoxy, unsubstituted or halogenated C₁₋₈ alkoxy-C₁₋₈ alkyl, unsubstituted or halogenated C₃₋₈ cycloalkyl-C₁₋₈ alkyl, unsubstituted or halogenated C₁₋₆ alkylcarbonyl, unsubstituted or halogenated C₁₋₆ alkoxycarbonyl, hydroxamic acid group, unsubstituted or halogenated C₁₋₆ alkylthio, - S(O)₂N(unsubstituted or halogenated C₁₋₆ alkyl)₂, -S(O)₂ unsubstituted or halogenated C₁₋₆ alkyl, - N(unsubstituted or halogenated C₁₋₆ alkyl)S(O)₂N(unsubstituted or halogenated C₁₋₆ alkyl)₂, - S(O)N(unsubstituted or halogenated C₁₋₆ alkyl)₂, -S(O)(unsubstituted or halogenated C₁₋₆ alkyl), - N(unsubstituted or halogenated C₁₋₆ alkyl)S(O)N(unsubstituted or halogenated C₁₋₆ alkyl)₂, - N(unsubstituted or halogenated C₁₋₆ alkyl)S(O)(unsubstituted or halogenated C₁₋₆ alkyl), unsubstituted or halogenated C₅₋₁₀ aryl, unsubstituted or halogenated 5- to 10- membered heteroaryl, unsubstituted or halogenated C₃₋₈ cycloalkyl and unsubstituted or halogenated 3- to 8- membered heterocyclyl, wherein the heterocyclyl and the heteroaryl comprise 1, 2, 3 or 4 heteroatoms selected from the group consisting of N, O and S.

In some embodiments, ring A is selected from: substituted or unsubstituted C₆₋₁₀ aryl, substituted or unsubstituted 5- to 10-membered heteroaryl, wherein the heteroaryl comprises 1, 2, or 3 heteroatoms selected from the group consisting of N, O, S, and P.

In some embodiments, ring B is selected from: substituted or unsubstituted C₃₋₈ cycloalkyl, substituted or unsubstituted 3- to 8-membered heterocyclyl, wherein the heterocyclyl comprises 1, 2, or 3 heteroatoms selected from the group consisting of N, O, S, and P.

In some embodiments, ring C is selected from: substituted or unsubstituted C₆₋₁₀ aryl, substituted or unsubstituted 5- to 10-membered heteroaryl, wherein the heteroaryl comprises 1, 2, or 3 heteroatoms selected from the group consisting of N, O, S, and P.

In some embodiments, ring D is selected from: substituted or unsubstituted C₃₋₈ cycloalkyl, substituted or unsubstituted 3- to 8-membered heterocyclyl, wherein the heterocyclyl comprises 1, 2, or 3 heteroatoms selected from the group consisting of N, O, S, and P.

In some embodiments, R₁ is selected from: H, halogen, substituted or unsubstituted C₂₋₆ alkynyl, substituted or unsubstituted C₂₋₆ alkenyl, substituted or unsubstituted C₁₋₆ alkyl, substituted or unsubstituted C₁₋₆ alkoxy, cyano, -NO₂, -OH, substituted or unsubstituted C₃₋₈ cycloalkyl, substituted or unsubstituted 3- to 8-membered heterocyclyl, substituted or unsubstituted C₆₋₁₀ aryl, substituted or unsubstituted 5- to 10-membered heteroaryl, substituted or unsubstituted 3- to 8-membered heterocyclyl spiro 3- to 8-membered heterocyclyl, substituted or unsubstituted 3- to 8-membered heterocyclyl fused 3- to 8-membered heterocyclyl, substituted or unsubstituted C₃₋₈ cycloalkyl spiro C₃₋₈ cycloalkyl, substituted or unsubstituted C₃₋₈ cycloalkyl fused C₃₋₈ cycloalkyl, wherein the heterocyclyl or the heteroaryl comprises 1, 2, or 3 heteroatoms selected from the group consisting of N, O, S, and P. In some embodiments, R₁ is selected from: H, halogen, substituted or unsubstituted C₂₋₆ alkynyl, substituted or unsubstituted C₂₋₆ alkenyl, substituted or unsubstituted C₁₋₆ alkyl, substituted or unsubstituted C₁₋₆ alkoxy, cyano, -NO₂, -OH, substituted or unsubstituted C₃₋₈ cycloalkyl, substituted or unsubstituted 3- to 8-membered heterocyclyl, substituted or unsubstituted 3- to 8-membered heterocyclyl spiro 3- to 8-membered heterocyclyl, substituted or unsubstituted 3- to 8-membered heterocyclyl fused 3- to 8-membered heterocyclyl, substituted or unsubstituted C₃₋₈ cycloalkyl spiro C₃₋₈ cycloalkyl, wherein the heterocyclyl comprises 1, 2, or 3 heteroatoms selected from the group consisting of N, O, S, and P. In certain embodiments, R₁ is selected from: H, halogen, substituted or unsubstituted C₂₋₆ alkynyl, substituted or unsubstituted C₂₋₆ alkenyl, substituted or unsubstituted C₁₋₆ alkyl, substituted or unsubstituted C₁₋₆ alkoxy, cyano, - NO₂, -OH, substituted or unsubstituted 3- to 8-membered heterocyclyl, substituted or unsubstituted 3- to 8-membered heterocyclyl spiro 3- to 8-membered heterocyclyl, wherein the heterocyclyl comprises 1, 2, or 3 heteroatoms selected from the group consisting of N, O, S, and P.

In some embodiments, R₂, R₃, R₄, R₅, and R₆ are each independently selected from: H, oxo (=O), substituted or unsubstituted C₁₋₆ alkoxy, halogen, substituted or unsubstituted C₁₋₆ alkyl, substituted or unsubstituted C₃₋₈ cycloalkyl, substituted or unsubstituted 3- to 8-membered heterocyclyl, substituted or unsubstituted C₆₋₁₀ aryl, substituted or unsubstituted 5- to 10-membered heteroaryl, wherein the heterocyclyl or the heteroaryl comprises 1, 2, or 3 heteroatoms selected from the group consisting of N, O, S, and P. In some embodiments, R₂, R₃, R₄, R₅, and R₆ are each independently selected from: H, oxo (=O), substituted or unsubstituted C₁₋₆ alkoxy, halogen, substituted or unsubstituted C₁₋₆ alkyl, substituted or unsubstituted C₆₋₁₀ aryl, substituted or unsubstituted 5- to 10-membered heteroaryl, wherein the heteroaryl comprises 1, 2, or 3 heteroatoms selected from the group consisting of N, O, S, and P. In certain embodiments, R₂, R₃, R₄, R₅, and R₆ are each independently selected from: H, oxo (=O), halogen, substituted or unsubstituted C₁₋₆ alkyl, substituted or unsubstituted C₆₋₁₀ aryl, substituted or unsubstituted 5- to 10-membered heteroaryl, wherein the heteroaryl comprises 1, 2, or 3 heteroatoms selected from the group consisting of N, O, S, and P.

In some embodiments, ring A is substituted or unsubstituted aryl. Ring A is 6- to 14-membered aryl, 6- to 12-membered aryl, 6- to 10-membered aryl, 6- to 8-membered aryl. In certain embodiments, ring A is phenyl or naphthyl. In certain embodiments, ring A is phenyl.

In some embodiments, ring A is substituted or unsubstituted heteroaryl. In some embodiments, ring A is selected from 5- to 14-membered heteroaryl, 5- to 12-membered heteroaryl, 5- to 10-membered heteroaryl, 5- to 8-membered heteroaryl, 5- to 6-membered heteroaryl. In certain embodiments, ring A is selected from thienyl, imidazolyl, pyrazolyl, thiazolyl, oxazolyl, pyridinyl, pyrimidinyl, pyrazinyl, pyridazinyl, furanyl, pyrrolyl, triazolyl, triazinyl, imidazolyl, pyrazolyl, pyrrolyl, pyridinyl, pyrimidinyl, or pyrazinyl. In certain embodiments, ring A is pyridinyl.

In some embodiments, ring B is substituted or unsubstituted heterocyclyl. Ring B is selected from 5- to 14-membered heterocyclyl, 5- to 12-membered heterocyclyl, 5- to 10-membered heterocyclyl, 5- to 8-membered heterocyclyl, 5- to 6-membered heterocyclyl. In certain embodiments, ring B is selected from pyrrolidinyl, imidazolidinyl, tetrahydrofuranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl, pyranyl. In certain embodiments, ring B is piperidinyl.

In some embodiments, ring C is substituted or unsubstituted heteroaryl. In some embodiments, ring C is 5- to 14-membered heteroaryl, 5- to 12-membered heteroaryl, 5- to 10-membered heteroaryl, 5- to 8-membered heteroaryl, 5- to 6-membered heteroaryl. In some embodiments, ring C is selected from thienyl, imidazolyl, pyrazolyl, thiazolyl, oxazolyl, pyridinyl, pyrimidinyl, pyrazinyl, azinyl, furanyl, pyrrolyl, triazolyl, triazinyl, pyridonyl. In certain embodiments, ring C is pyrazolyl.

In some embodiments, ring D is substituted or unsubstituted heterocyclyl. Ring D is 5- to 14-membered heterocyclyl, 5- to 12-membered heterocyclyl, 5- to 10-membered heterocyclyl, 5- to 8-membered heterocyclyl, 5- to 6-membered heterocyclyl. In certain embodiments, ring D is selected from pyrrolidinyl, imidazolidinyl, tetrahydrofuranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl, pyranyl or oxazacyclohexane. In certain embodiments, ring D is pyrrolidinyl or 1,3-oxazacyclohexyl. In certain embodiments, ring D is pyrrolidinyl. In certain embodiments, ring D is 1,3-oxazacyclohexyl or morpholinyl. In certain embodiments, ring D is 1,3-oxazacyclohexyl.

In some embodiments, R₁ is selected from H, halogen, substituted or unsubstituted C₂₋₆ alkynyl, substituted or unsubstituted C₂₋₆ alkenyl, substituted or unsubstituted C₁₋₆ alkoxy, cyano, substituted or unsubstituted 3- to 8-membered heterocyclyl spiro 3- to 8-membered heterocyclyl, wherein the heterocyclyl comprises 1, 2, or 3 heteroatoms selected from the group consisting of N, O, S. In certain embodiments, R₁ is selected from H, halogen, substituted or unsubstituted C₂₋₅ alkynyl, substituted or unsubstituted C₁₋₃ alkoxy, cyano, substituted or unsubstituted 5- to 6-membered heterocyclyl spiro 4- to 6-membered heterocyclyl, wherein the heterocyclyl comprises 1 or 2 heteroatoms selected from the group consisting of N, O, S. In certain embodiments, said C₂₋₅ alkynyl, C₁₋₃ alkoxy, and 5- to 6-membered heterocyclyl spiro 4- to 6-membered heterocyclyl are substituted with substituents selected from C₁₋₆ alkyl, 3- to 5-membered cycloalkyl, 4- to 6-membered heterocyclyl, hydroxyC₂₋₅ alkyl. In certain embodiments, said C₂₋₅ alkynyl, C₁₋₃ alkoxy, and 5- to 6-membered heterocyclyl spiro 4- to 6-membered heterocyclyl are substituted with substituents selected from cyclopropyl, pyranyl, hydroxyisopropyl.

In some embodiments, R₁ is selected from the group consisting of:
H, 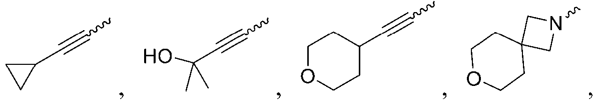 methoxy, cyano, chloro, and bromo.

In some embodiments, R₂ is selected from H, oxo (=O), substituted or unsubstituted C₁₋₆ alkoxy, halogen, substituted or unsubstituted C₁₋₆ alkyl, cyano, -NO₂, -OH. In certain embodiments, said C₁₋₆ alkoxy and C₁₋₆ alkyl are each independently substituted with oxo (=O), -OH, cyano, or - NO₂. In certain embodiments, R₂ is oxo.

In some embodiments, R₃ is selected from: H, substituted or unsubstituted C₁₋₆ alkoxy, halogen, cyano, -NO₂, -OH. In certain embodiments, said C₁₋₆ alkoxy and C₁₋₆ alkyl are each independently substituted with oxo (=O), -OH, cyano, or -NO₂. In certain embodiments, R₃ is H.

In some embodiments, R₄ is selected from H, substituted or unsubstituted C₁₋₆ alkoxy, halogen, substituted or unsubstituted C₁₋₆ alkyl, cyano, -NO₂, -OH, substituted or unsubstituted 3- to 8-membered heterocyclyl, substituted or unsubstituted C₆₋₁₀ aryl, substituted or unsubstituted 5- to 10-membered heteroaryl, wherein the heterocyclyl or the heteroaryl comprises 1, 2, or 3 heteroatoms selected from the group consisting of N, O, and S. In certain embodiments, said C₁₋₆ alkoxy, C₁₋₆ alkyl, 3- to 8-membered heterocyclyl, C₆₋₁₀ aryl, 5- to 10-membered heteroaryl are each independently substituted with 1, 2, or 3 substituents selected from H, halogen, -OH, C₁₋₆ alkyl, halogenated C₁₋₆ alkyl, and C₁₋₈ alkoxy. In certain embodiments, t is 1 or 2.

In certain embodiments, R₄ is selected from halogen, methylphenyl, difluoropropyl, phenyl, benzyl, difluorophenyl, methoxyphenyl, and pyridinyl. In certain embodiments, R₄ is selected from: 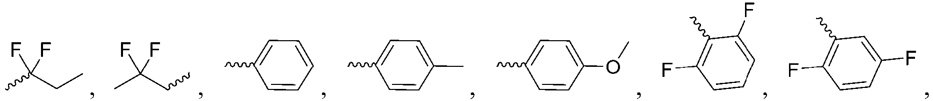 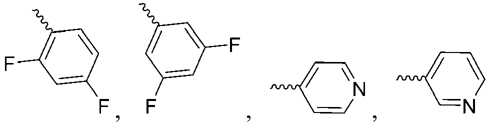 and halogen.

In certain embodiments, R₄ is selected from: 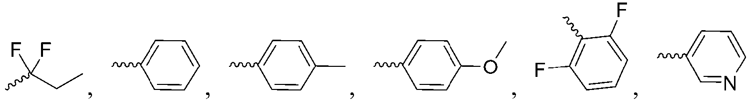 and F.

In some embodiments, R₅ is selected from H, substituted or unsubstituted C₁₋₆ alkoxy, halogen, substituted or unsubstituted C₁₋₆ alkyl. In certain embodiments, R₅ is selected from unsubstituted C₁₋₆ alkyl. In certain embodiments, R₅ is methyl.

In some embodiments, R₆ is selected from H, substituted or unsubstituted C₁₋₆ alkoxy, halogen, substituted or unsubstituted C₁₋₆ alkyl. In certain embodiments, R₆ is H.

In some embodiments, n, m, q, and t are each independently any integer selected from 0, 1, or 2. In some embodiments, n, m, q, and t are each independently any integer selected from 0 or 1.

In some embodiments, the compound of Formula A provided in the present disclosure, or a pharmaceutically acceptable salt thereof, or an enantiomer, diastereomer, tautomer, solvate, polymorph, prodrug or metabolite thereof, has the structure of Formula II below: 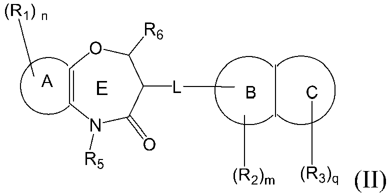 in Formula II:
ring A, ring B, and ring C are each independently selected from: substituted or unsubstituted C₃₋₈ cycloalkyl, substituted or unsubstituted 3- to 8-membered heterocyclyl, substituted or unsubstituted C₆₋₁₀ aryl, substituted or unsubstituted 5- to 10-membered heteroaryl, wherein the heterocyclyl or the heteroaryl comprises 1, 2, or 3 heteroatoms selected from the group consisting of N, O, S, and P;
L is selected from: 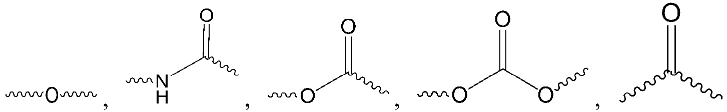 and C₁₋₁₀ alkylene;
R₁ is selected from: H, halogen, substituted or unsubstituted C₂₋₆ alkynyl, substituted or unsubstituted C₂₋₆ alkenyl, substituted or unsubstituted C₁₋₆ alkyl, substituted or unsubstituted C₁₋₆ alkoxy, cyano, -NO₂, -OH, substituted or unsubstituted C₃₋₈ cycloalkyl, substituted or unsubstituted 3- to 8- membered heterocyclyl, substituted or unsubstituted C₆₋₁₀ aryl, substituted or unsubstituted 5- to 10- membered heteroaryl, substituted or unsubstituted 3- to 8- membered heterocyclyl spiro 3- to 8- membered heterocyclyl, substituted or unsubstituted 3- to 8- membered heterocyclyl fused 3- to 8- membered heterocyclyl, substituted or unsubstituted C₃₋₈ cycloalkyl spiro C₃₋₈ cycloalkyl, substituted or unsubstituted C₃₋₈ cycloalkyl fused C₃₋₈ cycloalkyl, substituted or unsubstituted 3- to 8- membered heterocyclyl fused C₆₋₁₀ aryl, substituted or unsubstituted 3- to 8- membered heterocyclyl fused 5- to 10- membered heteroaryl, substituted or unsubstituted C₃₋₈ cycloalkyl fused C₆₋₁₀ aryl, substituted or unsubstituted C₃₋₈ cycloalkyl fused 5- to 10- membered heteroaryl, wherein the heterocyclyl or the heteroaryl comprises 1, 2 or 3 heteroatoms selected from the group consisting of N, O, S and P;
R₂, R₃, R₅, and R₆ are each independently selected from: H, oxo (=O), substituted or unsubstituted C₁₋₆ alkoxy, halogen, substituted or unsubstituted C₂₋₆ alkynyl, substituted or unsubstituted C₂₋₆ alkenyl, substituted or unsubstituted C₁₋₆ alkyl, cyano, -NO₂, -OH, substituted or unsubstituted C₃₋₈ cycloalkyl, substituted or unsubstituted 3- to 8-membered heterocyclyl, substituted or unsubstituted C₆₋₁₀ aryl, substituted or unsubstituted 5- to 10-membered heteroaryl, wherein the heterocyclyl or the heteroaryl comprises 1, 2, or 3 heteroatoms selected from the group consisting of N, O, S, and P;
n, m, and q are each independently any integer selected from 0, 1, 2, or 3;
the "substituted" means that one or more hydrogen atoms on the group are substituted with substituents selected from the group consisting of: halogen, -OH, -NO₂, -NH₂, -NH(unsubstituted or halogenated C₁₋₆ alkyl), -N(unsubstituted or halogenated C₁₋₆ alkyl)₂, -CN, unsubstituted C₁₋₈ alkyl or C₁₋₈ alkyl substituted with halogen or hydroxy, unsubstituted or halogenated C₁₋₈ alkoxy, unsubstituted or halogenated C₁₋₈ alkoxy-C₁₋₈ alkyl, unsubstituted or halogenated C₃₋₈ cycloalkyl-C₁₋₈ alkyl, unsubstituted or halogenated C₁₋₆ alkylcarbonyl, unsubstituted or halogenated C₁₋₆ alkoxycarbonyl, hydroxamic acid group, unsubstituted or halogenated C₁₋₆ alkylthio, - S(O)₂N(unsubstituted or halogenated C₁₋₆ alkyl)₂, -S(O)₂ unsubstituted or halogenated C₁₋₆ alkyl, - N(unsubstituted or halogenated C₁₋₆ alkyl)S(O)₂N(unsubstituted or halogenated C₁₋₆ alkyl)₂, - S(O)N(unsubstituted or halogenated C₁₋₆ alkyl)₂, -S(O)(unsubstituted or halogenated C₁₋₆ alkyl), - N(unsubstituted or halogenated C₁₋₆ alkyl)S(O)N(unsubstituted or halogenated C₁₋₆ alkyl)₂, - N(unsubstituted or halogenated C₁₋₆ alkyl)S(O)(unsubstituted or halogenated C₁₋₆ alkyl), unsubstituted or halogenated C₅₋₁₀ aryl, unsubstituted or halogenated 5- to 10- membered heteroaryl, unsubstituted or halogenated C₃₋₈ cycloalkyl, unsubstituted or halogenated 3- to 8- membered heterocyclyl, wherein the heterocyclyl and the heteroaryl comprise 1, 2, 3 or 4 heteroatoms selected from the group consisting of N, O and S.

In some embodiments, ring A is selected from: substituted or unsubstituted C₆₋₁₀ aryl, substituted or unsubstituted 5- to 10-membered heteroaryl, wherein the heteroaryl comprises 1, 2, or 3 heteroatoms selected from the group consisting of N, O, and S.

In some embodiments, ring B is selected from: substituted or unsubstituted C₆₋₁₀ aryl, substituted or unsubstituted 5- to 10-membered heteroaryl, wherein the heteroaryl comprises 1, 2, or 3 heteroatoms selected from the group consisting of N, O, and S.

In some embodiments, ring C is selected from: substituted or unsubstituted C₃₋₈ cycloalkyl, substituted or unsubstituted 3- to 8-membered heterocyclyl, wherein the heterocyclyl comprises 1, 2, or 3 heteroatoms selected from the group consisting of N, O, and S.

In some embodiments, L is selected from: 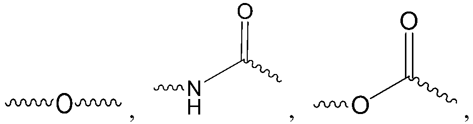 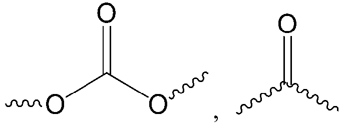 and C₁₋₁₀ alkylene. In some embodiments, L is selected from: 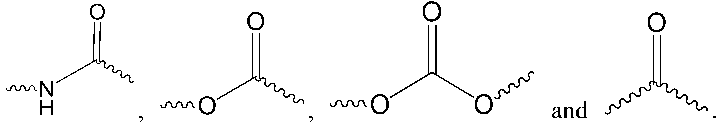 In certain embodiments, L is

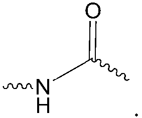

In some embodiments, R₁ is selected from: H, halogen, substituted or unsubstituted C₂₋₆ alkynyl, substituted or unsubstituted C₂₋₆ alkenyl, substituted or unsubstituted C₁₋₆ alkyl, substituted or unsubstituted C₁₋₆ alkoxy, cyano, -NO₂, -OH, substituted or unsubstituted C₃₋₈ cycloalkyl, substituted or unsubstituted 3- to 8-membered heterocyclyl, substituted or unsubstituted C₆₋₁₀ aryl, substituted or unsubstituted 5- to 10-membered heteroaryl, wherein the heterocyclyl or the heteroaryl comprises 1, 2, or 3 heteroatoms selected from the group consisting of N, O, S, and P. In some embodiments, R₁ is selected from: H, halogen, substituted or unsubstituted C₂₋₆ alkynyl, substituted or unsubstituted C₂₋₆ alkenyl, substituted or unsubstituted C₁₋₆ alkyl, substituted or unsubstituted C₁₋₆ alkoxy, cyano, -NO₂, -OH. In certain embodiments, R₁ is selected from: H, halogen, substituted or unsubstituted C₁₋₆ alkyl, substituted or unsubstituted C₁₋₆ alkoxy, cyano, - NO₂, -OH.

In some embodiments, R₂, R₃, R₅, and R₆ are each independently selected from: H, oxo (=O), substituted or unsubstituted C₁₋₆ alkoxy, halogen, substituted or unsubstituted C₂₋₆ alkynyl, substituted or unsubstituted C₂₋₆ alkenyl, substituted or unsubstituted C₁₋₆ alkyl, cyano, -NO₂, -OH, substituted or unsubstituted C₆₋₁₀ aryl, substituted or unsubstituted 5- to 10-membered heteroaryl, wherein the heteroaryl comprises 1, 2, or 3 heteroatoms selected from the group consisting of N, O, S, and P. In some embodiments, R₃, R₄, R₅, and R₆ are each independently selected from: H, oxo (=O), substituted or unsubstituted C₁₋₆ alkoxy, halogen, substituted or unsubstituted C₁₋₆ alkyl, cyano, -NO₂, -OH, substituted or unsubstituted C₆₋₁₀ aryl, substituted or unsubstituted 5- to 10-membered heteroaryl, wherein the heteroaryl comprises 1, 2, or 3 heteroatoms selected from the group consisting of N, O, S, and P. In certain embodiments, R₃, R₄, R₅, and R₆ are each independently selected from: H, oxo (=O), substituted or unsubstituted C₁₋₆ alkoxy, halogen, substituted or unsubstituted C₁₋₆ alkyl, substituted or unsubstituted C₆₋₁₀ aryl.

In some embodiments, n, m, and q are each independently any integer selected from 0, 1, or 2. In some embodiments, n, q, and t are each independently any integer selected from 0 or 1.

In some embodiments, the compound of Formula A provided in the present disclosure, or a pharmaceutically acceptable salt thereof, or an enantiomer, diastereomer, tautomer, solvate, polymorph, prodrug or metabolite thereof, has the structure of Formula III below: 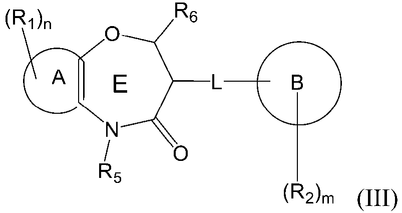 in Formula III:
ring A and ring B are each independently selected from: substituted or unsubstituted C₃₋₈ cycloalkyl, substituted or unsubstituted 3- to 8-membered heterocyclyl, substituted or unsubstituted C₆₋₁₀ aryl, substituted or unsubstituted 5- to 10-membered heteroaryl, wherein the heterocyclyl or the heteroaryl comprises 1, 2, or 3 heteroatoms selected from the group consisting of N, O, S, and P;
L is selected from: 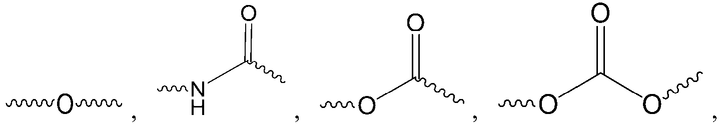 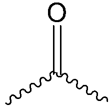 and C₁₋₁₀ alkylene;
R₁ is selected from: H, halogen, substituted or unsubstituted C₂₋₆ alkynyl, substituted or unsubstituted C₂₋₆ alkenyl, substituted or unsubstituted C₁₋₆ alkyl, substituted or unsubstituted C₁₋₆ alkoxy, cyano, -NO₂, -OH, substituted or unsubstituted C₃₋₈ cycloalkyl, substituted or unsubstituted 3- to 8- membered heterocyclyl, substituted or unsubstituted C₆₋₁₀ aryl, substituted or unsubstituted 5- to 10- membered heteroaryl, substituted or unsubstituted 3- to 8- membered heterocyclyl spiro 3- to 8- membered heterocyclyl, substituted or unsubstituted 3- to 8- membered heterocyclyl fused 3- to 8- membered heterocyclyl, substituted or unsubstituted C₃₋₈ cycloalkyl spiro C₃₋₈ cycloalkyl, substituted or unsubstituted C₃₋₈ cycloalkyl fused C₃₋₈ cycloalkyl, substituted or unsubstituted 3- to 8- membered heterocyclyl fused C₆₋₁₀ aryl, substituted or unsubstituted 3- to 8- membered heterocyclyl fused 5- to 10- membered heteroaryl, substituted or unsubstituted C₃₋₈ cycloalkyl fused C₆₋₁₀ aryl, substituted or unsubstituted C₃₋₈ cycloalkyl fused 5- to 10- membered heteroaryl, wherein the heterocyclyl or the heteroaryl comprises 1, 2 or 3 heteroatoms selected from the group consisting of N, O, S and P;
R₂, R₅, and R₆ are each independently selected from: H, oxo (=O), substituted or unsubstituted C₁₋₆ alkoxy, halogen, substituted or unsubstituted C₂₋₆ alkynyl, substituted or unsubstituted C₂₋₆ alkenyl, substituted or unsubstituted C₁₋₆ alkyl, cyano, -NO₂, -OH, substituted or unsubstituted C₃₋₈ cycloalkyl, substituted or unsubstituted 3- to 8-membered heterocyclyl, substituted or unsubstituted C₆₋₁₀ aryl, substituted or unsubstituted 5- to 10-membered heteroaryl, wherein the heterocyclyl or the heteroaryl comprises 1, 2, or 3 heteroatoms selected from the group consisting of N, O, S, and P;
n and m are each independently any integer selected from 0, 1, 2, or 3;
the "substituted" means that one or more hydrogen atoms on the group are substituted with substituents selected from the group consisting of: halogen, -OH, -NO₂, -NH₂, -NH(unsubstituted or halogenated C₁₋₆ alkyl), -N(unsubstituted or halogenated C₁₋₆ alkyl)₂, -CN, unsubstituted C₁₋₈ alkyl or C₁₋₈ alkyl substituted with halogen or hydroxy, unsubstituted or halogenated C₁₋₈ alkoxy, unsubstituted or halogenated C₁₋₈ alkoxy-C₁₋₈ alkyl, unsubstituted or halogenated C₃₋₈ cycloalkyl-C₁₋₈ alkyl, unsubstituted or halogenated C₁₋₆ alkylcarbonyl, unsubstituted or halogenated C₁₋₆ alkoxycarbonyl, hydroxamic acid group, unsubstituted or halogenated C₁₋₆ alkylthio, - S(O)₂N(unsubstituted or halogenated C₁₋₆ alkyl)₂, -S(O)₂ unsubstituted or halogenated C₁₋₆ alkyl, - N(unsubstituted or halogenated C₁₋₆ alkyl)S(O)₂N(unsubstituted or halogenated C₁₋₆ alkyl)₂, - S(O)N(unsubstituted or halogenated C₁₋₆ alkyl)₂, -S(O)(unsubstituted or halogenated C₁₋₆ alkyl), - N(unsubstituted or halogenated C₁₋₆ alkyl)S(O)N(unsubstituted or halogenated C₁₋₆ alkyl)₂, - N(unsubstituted or halogenated C₁₋₆ alkyl)S(O)(unsubstituted or halogenated C₁₋₆ alkyl), unsubstituted or halogenated C₅₋₁₀ aryl, unsubstituted or halogenated 5- to 10- membered heteroaryl, unsubstituted or halogenated C₃₋₈ cycloalkyl, unsubstituted or halogenated 3- to 8- membered heterocyclyl, wherein the heterocyclyl and the heteroaryl comprise 1, 2, 3 or 4 heteroatoms selected from the group consisting of N, O and S.

In some embodiments, ring A and ring B are each independently selected from: substituted or unsubstituted C₆₋₁₀ aryl, substituted or unsubstituted 5- to 10-membered heteroaryl, wherein the heteroaryl comprises 1, 2, or 3 heteroatoms selected from the group consisting of N, O, S, and P.

In some embodiments, L is selected from 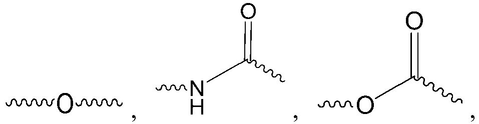 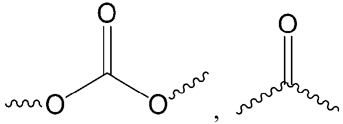 and C₁₋₁₀ alkylene. In some embodiments, L is selected from: 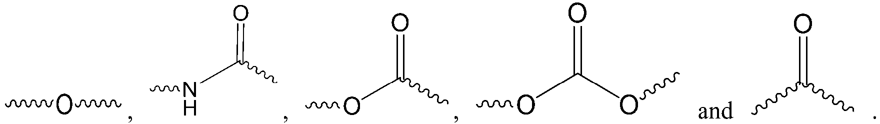 In certain embodiments, L is selected from:

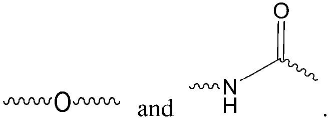

In some embodiments, R₁ is selected from: H, halogen, substituted or unsubstituted C₂₋₆ alkynyl, substituted or unsubstituted C₂₋₆ alkenyl, substituted or unsubstituted C₁₋₆ alkyl, substituted or unsubstituted C₁₋₆ alkoxy, cyano, -NO₂, -OH, substituted or unsubstituted C₃₋₈ cycloalkyl, substituted or unsubstituted 3- to 8-membered heterocyclyl, substituted or unsubstituted C₆₋₁₀ aryl, substituted or unsubstituted 5- to 10-membered heteroaryl, substituted or unsubstituted 3- to 8-membered heterocyclyl spiro 3- to 8-membered heterocyclyl, substituted or unsubstituted 3- to 8-membered heterocyclyl fused 3- to 8-membered heterocyclyl, wherein the heterocyclyl or the heteroaryl comprises 1, 2, or 3 heteroatoms selected from the group consisting of N, O, S, and P. In some embodiments, R₁ is selected from: H, halogen, substituted or unsubstituted C₂₋₆ alkynyl, substituted or unsubstituted C₂₋₆ alkenyl, substituted or unsubstituted C₁₋₆ alkyl, substituted or unsubstituted C₁₋₆ alkoxy, cyano, -NO₂, -OH, substituted or unsubstituted C₃₋₈ cycloalkyl, substituted or unsubstituted 3- to 8-membered heterocyclyl, substituted or unsubstituted 3- to 8-membered heterocyclyl spiro 3- to 8-membered heterocyclyl, substituted or unsubstituted 3- to 8-membered heterocyclyl fused 3- to 8-membered heterocyclyl, wherein the heterocyclyl comprises 1, 2, or 3 heteroatoms selected from the group consisting of N, O, S, and P. In certain embodiments, R₁ is selected from: H, halogen, substituted or unsubstituted C₂₋₆ alkynyl, substituted or unsubstituted C₂₋₆ alkenyl, substituted or unsubstituted C₁₋₆ alkyl, substituted or unsubstituted C₁₋₆ alkoxy, substituted or unsubstituted 3- to 8-membered heterocyclyl, substituted or unsubstituted 3-to 8-membered heterocyclyl spiro 3- to 8-membered heterocyclyl, wherein the heterocyclyl comprises 1, 2, or 3 heteroatoms selected from the group consisting of N, O, S, and P.

In some embodiments, R₂, R₅, and R₆ are each independently selected from: H, oxo (=O), substituted or unsubstituted C₁₋₆ alkoxy, halogen, substituted or unsubstituted C₂₋₆ alkynyl, substituted or unsubstituted C₂₋₆ alkenyl, substituted or unsubstituted C₁₋₆ alkyl, cyano, -NO₂, -OH, substituted or unsubstituted C₆₋₁₀ aryl, substituted or unsubstituted 5- to 10-membered heteroaryl, wherein the heteroaryl comprises 1, 2, or 3 heteroatoms selected from the group consisting of N, O, S, and P. In some embodiments, R₂, R₅, and R₆ are each independently selected from: H, oxo (=O), substituted or unsubstituted C₁₋₆ alkoxy, halogen, substituted or unsubstituted C₁₋₆ alkyl, cyano, - NO₂, -OH, substituted or unsubstituted C₆₋₁₀ aryl, substituted or unsubstituted 5- to 10-membered heteroaryl, wherein the heteroaryl comprises 1, 2, or 3 heteroatoms selected from the group consisting of N, O, S, and P. In certain embodiments, R₂, R₅, and R₆ are each independently selected from: H, oxo (=O), substituted or unsubstituted C₁₋₆ alkoxy, halogen, substituted or unsubstituted C₁₋₆ alkyl, substituted or unsubstituted 5- to 10-membered heteroaryl, wherein the heteroaryl comprises 1, 2, or 3 heteroatoms selected from the group consisting of N, O, S, and P.

In some embodiments, n and m are each independently any integer selected from 0, 1, or 2. In some embodiments, n and m are each independently any integer selected from 0 or 1.

In some embodiments, the compound of Formula A provided in the present disclosure, or a pharmaceutically acceptable salt thereof, or an enantiomer, diastereomer, tautomer, solvate, polymorph, prodrug or metabolite thereof, has the structure of Formula I-1 below: 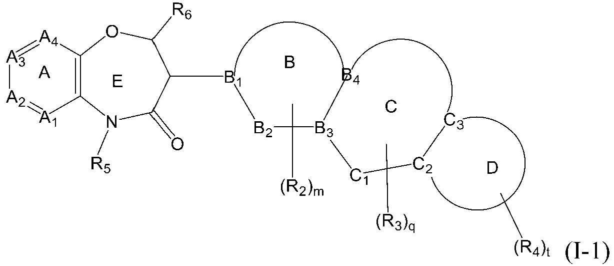 in Formula I-1:
A₁, A₂, A₃, and A₄ are each independently selected from: CR₁ or N;
B₁, B₂, B₃, and B₄ are each independently selected from: C(R₂)ₘ or N(R₂)ₘ;
C₁, C₂, and C₃ are each independently selected from: C(R₃)_{q} or N(R₃)_{q};
ring B, ring C, and ring D are each independently selected from: substituted or unsubstituted C₃₋₈ cycloalkyl, substituted or unsubstituted 3- to 8-membered heterocyclyl, substituted or unsubstituted C₆₋₁₀ aryl, substituted or unsubstituted 5- to 10-membered heteroaryl, wherein the heterocyclyl or the heteroaryl comprises 1, 2, or 3 heteroatoms selected from the group consisting of N, O, S, and P;
R₁ is selected from: H, halogen, substituted or unsubstituted C₂₋₆ alkynyl, substituted or unsubstituted C₂₋₆ alkenyl, substituted or unsubstituted C₁₋₆ alkyl, substituted or unsubstituted C₁₋₆ alkoxy, cyano, -NO₂, -OH, substituted or unsubstituted C₃₋₈ cycloalkyl, substituted or unsubstituted 3- to 8- membered heterocyclyl, substituted or unsubstituted C₆₋₁₀ aryl, substituted or unsubstituted 5- to 10- membered heteroaryl, substituted or unsubstituted 3- to 8- membered heterocyclyl spiro 3- to 8- membered heterocyclyl, substituted or unsubstituted 3- to 8- membered heterocyclyl fused 3- to 8- membered heterocyclyl, substituted or unsubstituted C₃₋₈ cycloalkyl spiro C₃₋₈ cycloalkyl, substituted or unsubstituted C₃₋₈ cycloalkyl fused C₃₋₈ cycloalkyl, substituted or unsubstituted 3- to 8- membered heterocyclyl fused C₆₋₁₀ aryl, substituted or unsubstituted 3- to 8- membered heterocyclyl fused 5- to 10- membered heteroaryl, substituted or unsubstituted C₃₋₈ cycloalkyl fused C₆₋₁₀ aryl, substituted or unsubstituted C₃₋₈ cycloalkyl fused 5- to 10- membered heteroaryl, wherein the heterocyclyl or heteroaryl comprises 1, 2 or 3 heteroatoms selected from the group consisting of N, O, S and P;
R₂, R₃, R₄, R₅, and R₆ are each independently selected from: H, oxo (=O), substituted or unsubstituted C₁₋₆ alkoxy, halogen, substituted or unsubstituted C₂₋₆ alkynyl, substituted or unsubstituted C₂₋₆ alkenyl, substituted or unsubstituted C₁₋₆ alkyl, cyano, -NO₂, -OH, substituted or unsubstituted C₃₋₈ cycloalkyl, substituted or unsubstituted 3- to 8-membered heterocyclyl, substituted or unsubstituted C₆₋₁₀ aryl, substituted or unsubstituted 5- to 10-membered heteroaryl, wherein the heterocyclyl or heteroaryl comprises 1, 2, or 3 heteroatoms selected from the group consisting of N, O, S, and P;
m, q, and t are each independently any integer selected from 0, 1, 2, or 3;
the "substituted" means that one or more hydrogen atoms on the group are substituted with substituents selected from the group consisting of: halogen, -OH, -NO₂, -NH₂, -NH(unsubstituted or halogenated C₁₋₆ alkyl), -N(unsubstituted or halogenated C₁₋₆ alkyl)₂, -CN, unsubstituted C₁₋₈ alkyl or C₁₋₈ alkyl substituted with halogen or hydroxy, unsubstituted or halogenated C₁₋₈ alkoxy, unsubstituted or halogenated C₁₋₈ alkoxy-C₁₋₈ alkyl, unsubstituted or halogenated C₃₋₈ cycloalkyl-C₁₋₈ alkyl, unsubstituted or halogenated C₁₋₆ alkylcarbonyl, unsubstituted or halogenated C₁₋₆ alkoxycarbonyl, hydroxamic acid group, unsubstituted or halogenated C₁₋₆ alkylthio, - S(O)₂N(unsubstituted or halogenated C₁₋₆ alkyl)₂, -S(O)₂ unsubstituted or halogenated C₁₋₆ alkyl, - N(unsubstituted or halogenated C₁₋₆ alkyl)S(O)₂N(unsubstituted or halogenated C₁₋₆ alkyl)₂, - S(O)N(unsubstituted or halogenated C₁₋₆ alkyl)₂, -S(O)(unsubstituted or halogenated C₁₋₆ alkyl), - N(unsubstituted or halogenated C₁₋₆ alkyl)S(O)N(unsubstituted or halogenated C₁₋₆ alkyl)₂, - N(unsubstituted or halogenated C₁₋₆ alkyl)S(O)(unsubstituted or halogenated C₁₋₆ alkyl), unsubstituted or halogenated C₅₋₁₀ aryl, unsubstituted or halogenated 5- to 10- membered heteroaryl, unsubstituted or halogenated C₃₋₈ cycloalkyl and unsubstituted or halogenated 3- to 8- membered heterocyclyl, wherein the heterocyclyl and heteroaryl comprise 1, 2, 3 or 4 heteroatoms selected from the group consisting of N, O and S.

In some embodiments, ring B is selected from: substituted or unsubstituted C₃₋₈ cycloalkyl, substituted or unsubstituted 3- to 8-membered heterocyclyl, wherein the heterocyclyl comprises 1, 2, or 3 heteroatoms selected from the group consisting of N, O, S, and P. In some embodiments, ring B is substituted or unsubstituted 3- to 8-membered heterocyclyl, wherein the heterocyclyl comprises 1, 2, or 3 heteroatoms selected from the group consisting of N, O, and S. In some embodiments, ring B is substituted or unsubstituted 3- to 7-membered heterocyclyl, substituted or unsubstituted 4- to 7-membered heterocyclyl, substituted or unsubstituted 4- to 6-membered heterocyclyl, substituted or unsubstituted 5- or 6-membered heterocyclyl. In some embodiments, ring B is substituted or unsubstituted 5- to 7-membered heterocyclyl. In some embodiments, ring B comprises 1 or 2 N atoms.

In certain embodiments, ring B is selected from pyrrolidinyl, imidazolidinyl, tetrahydrofuranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl, pyranyl. In certain embodiments, ring B is piperidinyl.

In some embodiments, ring C is selected from: substituted or unsubstituted C₆₋₁₀ aryl, substituted or unsubstituted 5- to 10-membered heteroaryl, wherein the heteroaryl comprises 1, 2, or 3 heteroatoms selected from the group consisting of N, O, S, and P.In some embodiments, ring C is substituted or unsubstituted 5- to 9-membered heteroaryl, 5- to 8-membered heteroaryl, 5- to 7-membered heteroaryl, 5- to 6-membered heteroaryl. In some embodiments, ring C is substituted or unsubstituted 5- to 10-membered heteroaryl. In certain embodiments, the heteroaryl comprises 1, 2, or 3 heteroatoms selected from the group consisting of N, O, S, and P. In some embodiments, ring C is substituted or unsubstituted 5- to 7-membered heteroaryl. In some embodiments, ring C comprises 1, 2, or 3 N atoms. In certain embodiments, ring C comprises 1 or 2 N atoms. In some embodiments, ring C is selected from thienyl, imidazolyl, pyrazolyl, thiazolyl, oxazolyl, pyridinyl, pyrimidinyl, pyrazinyl, azinyl, furanyl, pyrrolyl, triazolyl, triazinyl, pyridonyl. In certain embodiments, ring C is pyrazolyl.

In some embodiments, ring D is selected from: substituted or unsubstituted C₃₋₈ cycloalkyl, substituted or unsubstituted 3- to 8-membered heterocyclyl, wherein the heterocyclyl comprises 1, 2, or 3 heteroatoms selected from the group consisting of N, O, S, and P.In some embodiments, ring D is substituted or unsubstituted 3- to 8-membered heterocyclyl, wherein the heterocyclyl comprises 1, 2, or 3 heteroatoms selected from the group consisting of N, O, S, and P. In some embodiments, ring D is selected from: 5- to 10-membered heterocyclyl, 5- to 9-membered heterocyclyl, 5- to 8-membered heterocyclyl, 5- to 6-membered heterocyclyl. In some embodiments, ring D is substituted or unsubstituted 5- to 7-membered heterocyclyl. In some embodiments, ring D comprises 1 or 2 N atoms. In certain embodiments, ring D is selected from pyrrolidinyl, imidazolidinyl, tetrahydrofuranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl, pyranyl or oxazacyclohexane. In certain embodiments, ring D is pyrrolidinyl or 1,3-oxazacyclohexyl. In certain embodiments, ring D is pyrrolidinyl. In certain embodiments, ring D is 1,3-oxazacyclohexyl or morpholinyl. In certain embodiments, ring D is 1,3-oxazacyclohexyl.

In some embodiments, the structure formed by ring B, ring C and ring D is selected from the group consisting of:

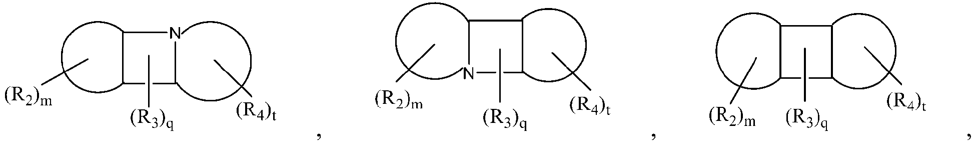

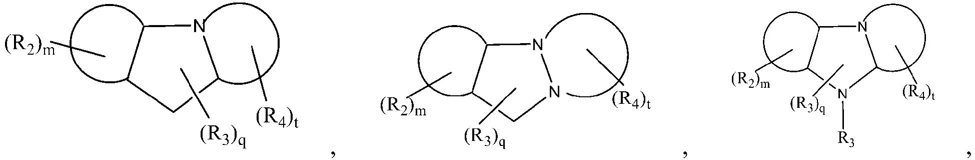

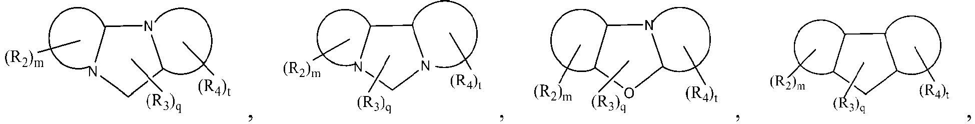

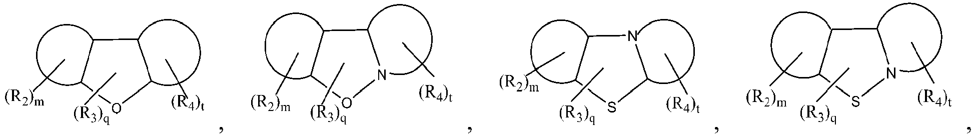

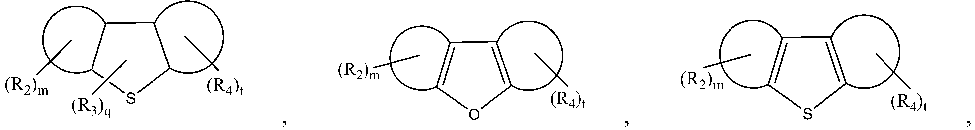

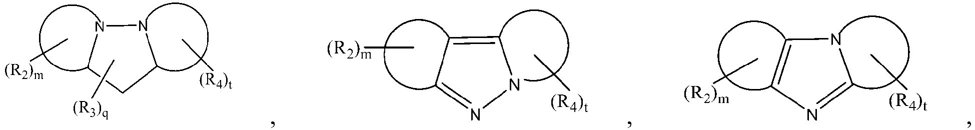

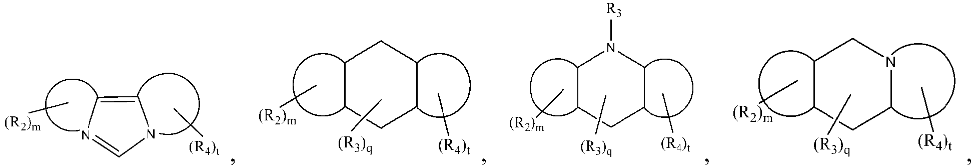

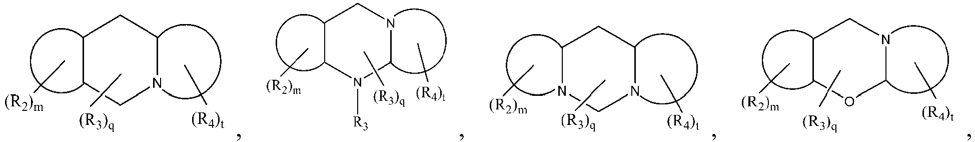

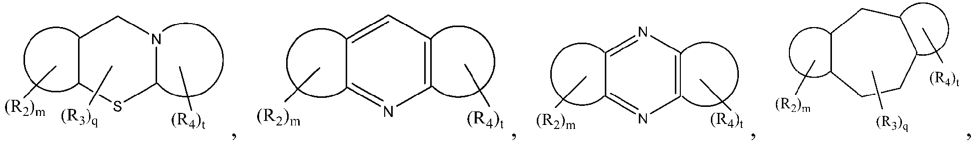

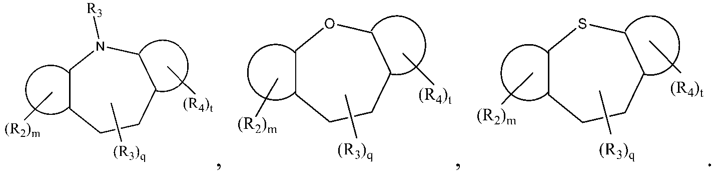

In some embodiments, the structure formed by ring B, ring C and ring D is selected from the group consisting of: 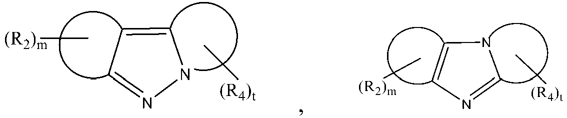 and

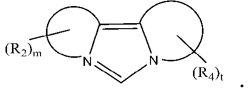

In some embodiments, the structure formed by ring B, ring C and ring D is selected from the group consisting of:

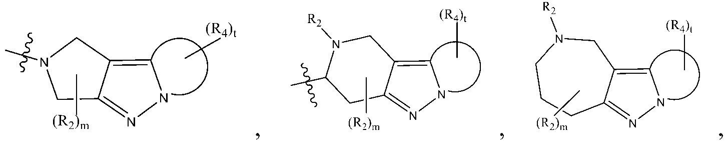

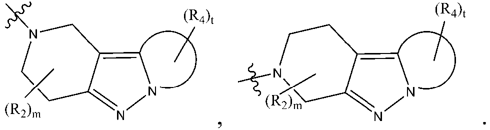

In some embodiments, the structure formed by ring B, ring C and ring D is selected from the group consisting of:

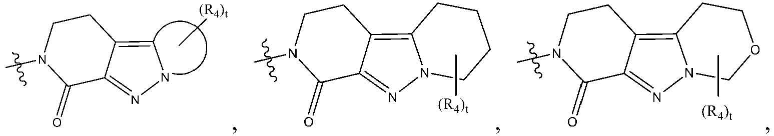

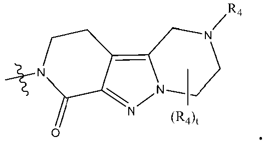

In some embodiments, A₁ and A₂ are each independently selected from CR₁ or N; A₃ and A₄ are C.

In some embodiments, B₁ is N(R₂)ₘ. In some embodiments, B₂ is C(R₂)ₘ. In some embodiments, B₃ is C(R₂)ₘ. In some embodiments, B₄ is C(R₂)ₘ.

In some embodiments, C₁ is N(R₃)_{q}. In some embodiments, C₂ is N(R₃)_{q}.

In some embodiments, R₁ is selected from: H, halogen, substituted or unsubstituted C₂₋₆ alkynyl, substituted or unsubstituted C₂₋₆ alkenyl, substituted or unsubstituted C₁₋₆ alkyl, substituted or unsubstituted C₁₋₆ alkoxy, cyano, -NO₂, -OH, substituted or unsubstituted C₃₋₈ cycloalkyl, substituted or unsubstituted 3- to 8-membered heterocyclyl, substituted or unsubstituted 3- to 8-membered heterocyclyl spiro 3- to 8-membered heterocyclyl, substituted or unsubstituted 3- to 8-membered heterocyclyl fused 3- to 8-membered heterocyclyl, substituted or unsubstituted C₃₋₈ cycloalkyl spiro C₃₋₈ cycloalkyl, substituted or unsubstituted C₃₋₈ cycloalkyl fused C₃₋₈ cycloalkyl, wherein the heterocyclyl comprises 1, 2, or 3 heteroatoms selected from the group consisting of N, O, S, and P. In some embodiments, R₁ is selected from: H, halogen, substituted or unsubstituted C₂₋₆ alkynyl, substituted or unsubstituted C₂₋₆ alkenyl, substituted or unsubstituted C₁₋₆ alkyl, substituted or unsubstituted C₁₋₆ alkoxy, cyano, -NO₂, -OH, substituted or unsubstituted 3- to 8-membered heterocyclyl, substituted or unsubstituted 3- to 8-membered heterocyclyl spiro 3- to 8-membered heterocyclyl, wherein the heterocyclyl comprises 1, 2, or 3 heteroatoms selected from the group consisting of N, O, S, and P. In some embodiments, R₁ is selected from: H, halogen, substituted or unsubstituted C₂₋₆ alkynyl, substituted or unsubstituted C₁₋₆ alkoxy, cyano, substituted or unsubstituted 3- to 8-membered heterocyclyl, substituted or unsubstituted 3- to 8-membered heterocyclyl spiro 3- to 8-membered heterocyclyl, wherein the heterocyclyl comprises 1, 2 or 3 heteroatoms selected from the group consisting of N, O, S and P. In some embodiments, R₁ is selected from: H, halogen, substituted or unsubstituted C₂₋₆ alkynyl, C₁₋₆ alkoxy, cyano, substituted or unsubstituted 3- to 8-membered heterocyclyl, substituted or unsubstituted 3- to 8-membered heterocyclyl spiro 3- to 8-membered heterocyclyl, wherein the C₂₋₆ alkynyl is optionally substituted with substituents selected from the group consisting of C₃₋₈ cycloalkyl, C₁₋₆ alkyl, C₁₋₆ alkyl substituted with hydroxyl, and 3- to 8-membered heterocyclyl, and said heterocyclyl comprises 1, 2 or 3 heteroatoms selected from the group consisting of N, O, S and P. In some embodiments, R₁ is selected from: H, halogen, substituted or unsubstituted C₂₋₅ alkynyl, substituted or unsubstituted C₁₋₃ alkoxy, cyano, substituted or unsubstituted 5- to 6-membered heterocyclyl spiro 4- to 6-membered heterocyclyl, wherein said heterocyclyl comprises 1 or 2 heteroatoms selected from the group consisting of N, O, S. In certain embodiments, said C₂₋₅ alkynyl, C₁₋₃ alkoxy, and 5- to 6-membered heterocyclyl spiro 4- to 6-membered heterocyclyl are substituted with substituents selected from C₁₋₆ alkyl, 3- to 5-membered cycloalkyl, 4- to 6-membered heterocyclyl, hydroxyC₂₋₅ alkyl. In certain embodiments, said C₂₋₅ alkynyl, C₁₋₃ alkoxy, and 5- to 6-membered heterocyclyl spiro 4- to 6-membered heterocyclyl are substituted with substituents selected from cyclopropyl, pyranyl, and hydroxyisopropyl.

In certain embodiments, R₁ is selected from the group consisting of: H, 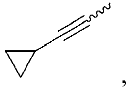 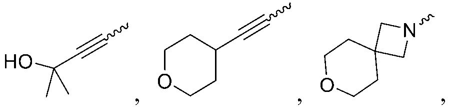 methoxy, cyano, chlorine, and bromine.

In some embodiments, R₂ and R₃ are each independently selected from: H, oxo (=O), substituted or unsubstituted C₁₋₆ alkoxy, halogen, substituted or unsubstituted C₂₋₆ alkynyl, substituted or unsubstituted C₂₋₆ alkenyl, substituted or unsubstituted C₁₋₆ alkyl, cyano, -NO₂, -OH. In some embodiments, R₂ and R₃ are each independently selected from: H, oxo, substituted or unsubstituted C₁₋₆ alkoxy, halogen, substituted or unsubstituted C₁₋₆ alkyl, cyano, -NO₂, -OH. In some embodiments, R₂ and R₃ are each independently selected from: H and oxo (=O).

In some embodiments, R₂ is selected from H, oxo (=O), substituted or unsubstituted C₁₋₆ alkoxy, halogen, substituted or unsubstituted C₁₋₆ alkyl, cyano, -NO₂, -OH. In certain embodiments, said C₁₋₆ alkoxy and C₁₋₆ alkyl are each independently substituted with oxo (=O), -OH, cyano, or - NO₂. In certain embodiments, R₂ is oxo.

In some embodiments, R₃ is selected from: H, substituted or unsubstituted C₁₋₆ alkoxy, substituted or unsubstituted C₁₋₆ alkyl, halogen, cyano, -NO₂, -OH. In certain embodiments, said C₁₋₆ alkoxy and C₁₋₆ alkyl are each independently substituted with oxo (=O), -OH, cyano or -NO₂. In certain embodiments, R₃ is H.

In some embodiments, R₄ is selected from: H, oxo (=O), substituted or unsubstituted C₁₋₆ alkoxy, halogen, substituted or unsubstituted C₁₋₆ alkyl, cyano, -NO₂, -OH, substituted or unsubstituted C₃₋₈ cycloalkyl, substituted or unsubstituted 3- to 8-membered heterocyclyl, substituted or unsubstituted C₆₋₁₀ aryl, substituted or unsubstituted 5- to 10-membered heteroaryl, wherein said heterocyclyl or heteroaryl comprises 1, 2 or 3 heteroatoms selected from the group consisting of N, O, S and P. In some embodiments, R₄ is selected from: H, substituted or unsubstituted C₁₋₆ alkoxy, halogen, substituted or unsubstituted C₁₋₆ alkyl, substituted or unsubstituted C₃₋₈ cycloalkyl, substituted or unsubstituted 3- to 8-membered heterocyclyl, substituted or unsubstituted C₆₋₁₀ aryl, substituted or unsubstituted 5- to 10-membered heteroaryl, wherein said heterocyclyl or heteroaryl comprises 1, 2 or 3 heteroatoms selected from the group consisting of N, O, S and P. In some embodiments, R₄ is selected from: H, halogen, substituted or unsubstituted C₁₋₆ alkyl, substituted or unsubstituted C₆₋₁₀ aryl, substituted or unsubstituted 5- to 10-membered heteroaryl, wherein said heteroaryl comprises 1, 2 or 3 heteroatoms selected from the group consisting of N, O, S and P. In some embodiments, the substitution means that one or more hydrogen atoms on the group are replaced by a substituent selected from the group consisting of halogen, C₁₋₈ alkyl and C₁₋₈ alkoxy. In some embodiments, R₄ is selected from H, substituted or unsubstituted C₁₋₆ alkoxy, halogen, substituted or unsubstituted C₁₋₆ alkyl, cyano, -NO₂, -OH, substituted or unsubstituted 3- to 8-membered heterocyclyl, substituted or unsubstituted C₆₋₁₀ aryl, substituted or unsubstituted 5- to 10-membered heteroaryl, wherein the heterocyclyl or heteroaryl comprises 1, 2, or 3 heteroatoms selected from the group consisting of N, O, and S. In certain embodiments, said C₁₋₆ alkoxy, C₁₋₆ alkyl, 3- to 8-membered heterocyclyl, C₆₋₁₀ aryl, and 5- to 10-membered heteroaryl are each independently substituted with 1, 2, or 3 substituents selected from H, halogen, -OH, C₁₋₆ alkyl, halogenated C₁₋₆ alkyl, and C₁₋₈ alkoxy. In certain embodiments, t is 1 or 2.

In certain embodiments, R₄ is selected from halogen, methylphenyl, difluoropropyl, phenyl, benzyl, difluorophenyl, methoxyphenyl, and pyridinyl. In certain embodiments, R₄ is selected from: 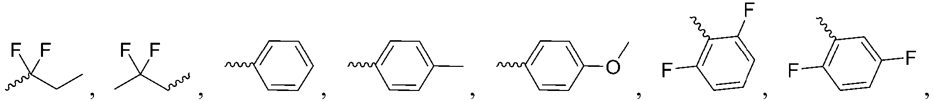 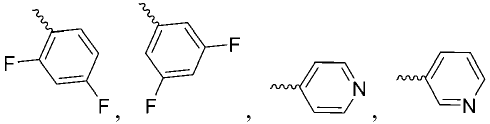 and halogen.

In certain embodiments, R₄ is selected from: 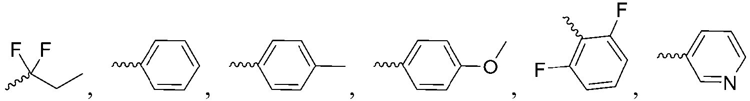 and F.

In some embodiments, R₅ and R₆ are each independently selected from: H, oxo (=O), substituted or unsubstituted C₁₋₆ alkoxy, halogen, substituted or unsubstituted C₂₋₆ alkynyl, substituted or unsubstituted C₂₋₆ alkenyl, substituted or unsubstituted C₁₋₆ alkyl, cyano, -NO₂, -OH. In some embodiments, R₅ and R₆ are each independently selected from: H, oxo (=O), substituted or unsubstituted C₁₋₆ alkoxy, halogen, substituted or unsubstituted C₁₋₆ alkyl, cyano, -NO₂, -OH. In some embodiments, R₅ and R₆ are each independently selected from: H, substituted or unsubstituted C₁₋₆ alkoxy, halogen, substituted or unsubstituted C₁₋₆ alkyl. In some embodiments, R₅ and R₆ are each independently selected from: H, C₁₋₆ alkyl. In some embodiments, R₅ is selected from H, substituted or unsubstituted C₁₋₆ alkoxy, halogen, and substituted or unsubstituted C₁₋₆ alkyl. In certain embodiments, R₅ is selected from unsubstituted C₁₋₆ alkyl. In certain embodiments, R₅ is methyl.

In some embodiments, R₆ is selected from H, substituted or unsubstituted C₁₋₆ alkoxy, halogen, and substituted or unsubstituted C₁₋₆ alkyl. In certain embodiments, R₆ is H.

In some embodiments, m is any integer of 0, 1 or 2. In some embodiments, m is 1.

In some embodiments, q is any integer of 0, 1 or 2. In some embodiments, q is any integer of 0-1. In some embodiments, q is 0.

In some embodiments, t is any integer of 0, 1 or 2. In some embodiments, t is 1 or 2.

In some embodiments, the compound of Formula A provided in the present disclosure, or a pharmaceutically acceptable salt thereof, or an enantiomer, diastereomer, tautomer, solvate, polymorph, prodrug or metabolite thereof, has the structure of Formula II-1 below: 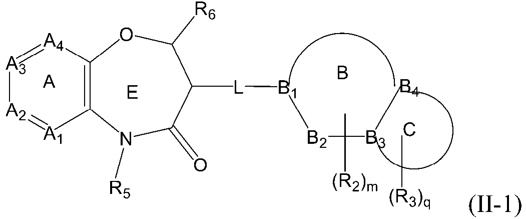 in formula II-1:
A₁, A₂, A₃, and A₄ are each independently selected from: CR₁ or N;
B₁, B₂, B₃, and B₄ are each independently selected from: C(R₂)ₘ or N(R₂)ₘ;
ring B, and ring C are each independently selected from: substituted or unsubstituted C₃₋₈ cycloalkyl, substituted or unsubstituted 3- to 8-membered heterocyclyl, substituted or unsubstituted C₆₋₁₀ aryl, substituted or unsubstituted 5- to 10-membered heteroaryl, wherein the heterocyclyl or the heteroaryl comprises 1, 2, or 3 heteroatoms selected from the group consisting of N, O, S, and P;
L is selected from: 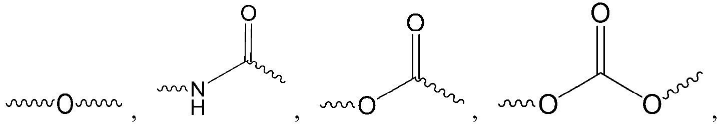 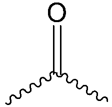 and C₁₋₁₀ alkylene;
R₁ is selected from: H, halogen, substituted or unsubstituted C₂₋₆ alkynyl, substituted or unsubstituted C₂₋₆ alkenyl, substituted or unsubstituted C₁₋₆ alkyl, substituted or unsubstituted C₁₋₆ alkoxy, cyano, -NO₂, -OH, substituted or unsubstituted C₃₋₈ cycloalkyl, substituted or unsubstituted 3- to 8- membered heterocyclyl, substituted or unsubstituted C₆₋₁₀ aryl, substituted or unsubstituted 5- to 10- membered heteroaryl, substituted or unsubstituted 3- to 8- membered heterocyclyl spiro 3- to 8- membered heterocyclyl, substituted or unsubstituted 3- to 8- membered heterocyclyl fused 3- to 8- membered heterocyclyl, substituted or unsubstituted C₃₋₈ cycloalkyl spiro C₃₋₈ cycloalkyl, substituted or unsubstituted C₃₋₈ cycloalkyl fused C₃₋₈ cycloalkyl, substituted or unsubstituted 3- to 8- membered heterocyclyl fused C₆₋₁₀ aryl, substituted or unsubstituted 3- to 8- membered heterocyclyl fused 5- to 10- membered heteroaryl, substituted or unsubstituted C₃₋₈ cycloalkyl fused C₆₋₁₀ aryl, substituted or unsubstituted C₃₋₈ cycloalkyl fused 5- to 10- membered heteroaryl, wherein the heterocyclyl or the heteroaryl comprises 1, 2 or 3 heteroatoms selected from the group consisting of N, O, S and P;
R₂, R₃, R₅, and R₆ are each independently selected from: H, oxo (=O), substituted or unsubstituted C₁₋₆ alkoxy, halogen, substituted or unsubstituted C₂₋₆ alkynyl, substituted or unsubstituted C₂₋₆ alkenyl, substituted or unsubstituted C₁₋₆ alkyl, cyano, -NO₂, -OH, substituted or unsubstituted C₃₋₈ cycloalkyl, substituted or unsubstituted 3- to 8-membered heterocyclyl, substituted or unsubstituted C₆₋₁₀ aryl, substituted or unsubstituted 5- to 10-membered heteroaryl, wherein the heterocyclyl or the heteroaryl comprises 1, 2, or 3 heteroatoms selected from the group consisting of N, O, S, and P;
m and q are each independently any integer selected from 0, 1, 2, or 3;
the "substituted" means that one or more hydrogen atoms on the group are substituted with substituents selected from the group consisting of: halogen, -OH, -NO₂, -NH₂, -NH(unsubstituted or halogenated C₁₋₆ alkyl), -N(unsubstituted or halogenated C₁₋₆ alkyl)₂, -CN, unsubstituted C₁₋₈ alkyl or C₁₋₈ alkyl substituted with halogen or hydroxy, unsubstituted or halogenated C₁₋₈ alkoxy, unsubstituted or halogenated C₁₋₈ alkoxy-C₁₋₈ alkyl, unsubstituted or halogenated C₃₋₈ cycloalkyl-C₁₋₈ alkyl, unsubstituted or halogenated C₁₋₆ alkylcarbonyl, unsubstituted or halogenated C₁₋₆ alkoxycarbonyl, hydroxamic acid group, unsubstituted or halogenated C₁₋₆ alkylthio, - S(O)₂N(unsubstituted or halogenated C₁₋₆ alkyl)₂, -S(O)₂ unsubstituted or halogenated C₁₋₆ alkyl, - N(unsubstituted or halogenated C₁₋₆ alkyl)S(O)₂N(unsubstituted or halogenated C₁₋₆ alkyl)₂, - S(O)N(unsubstituted or halogenated C₁₋₆ alkyl)₂, -S(O)(unsubstituted or halogenated C₁₋₆ alkyl), - N(unsubstituted or halogenated C₁₋₆ alkyl)S(O)N(unsubstituted or halogenated C₁₋₆ alkyl)₂, - N(unsubstituted or halogenated C₁₋₆ alkyl)S(O)(unsubstituted or halogenated C₁₋₆ alkyl), unsubstituted or halogenated C₅₋₁₀ aryl, unsubstituted or halogenated 5- to 10- membered heteroaryl, unsubstituted or halogenated C₃₋₈ cycloalkyl and unsubstituted or halogenated 3- to 8- membered heterocyclyl, wherein the heterocyclyl and the heteroaryl comprise 1, 2, 3 or 4 heteroatoms selected from the group consisting of N, O and S.

In some embodiments, A₁, A₂, A₃ and A₄ are C.

In some embodiments, B₁ and B₄ are C(R₂)ₘ, B₂ and B₃ are N(R₂)ₘ.

In some embodiments, ring B is substituted or unsubstituted C₆₋₁₀ aryl, substituted or unsubstituted 5- to 10-membered heteroaryl, wherein said heteroaryl comprises 1, 2 or 3 heteroatoms selected from the group consisting of N, O, S and P. In some embodiments, ring B is substituted or unsubstituted 5- to 10-membered heteroaryl, wherein said heteroaryl comprises 1, 2 or 3 heteroatoms selected from the group consisting of N, O, S and P. In some embodiments, ring B is substituted or unsubstituted 5- to 7-membered heteroaryl, wherein said heteroaryl comprises 1, 2 or 3 nitrogen atoms.

In some embodiments, ring C is substituted or unsubstituted C₃₋₈ cycloalkyl, substituted or unsubstituted 3- to 8-membered heterocyclyl, wherein said heterocyclyl comprises 1, 2 or 3 heteroatoms selected from the group consisting of N, O, S and P. In some embodiments, ring C is substituted or unsubstituted 3- to 8-membered heterocyclyl, wherein the heterocyclyl comprises 1, 2, or 3 heteroatoms selected from the group consisting of N, O, S, and P. In some embodiments, ring C is substituted or unsubstituted 5- to 7-membered heterocyclyl, wherein said heterocyclyl comprises 1, 2 or 3 nitrogen atoms.

In some embodiments, L is selected from: 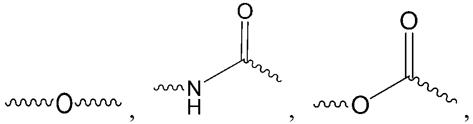 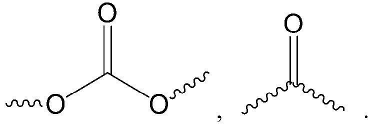 In some embodiments, L is selected from: 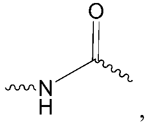 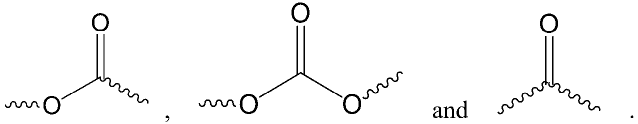 In some embodiments, L is

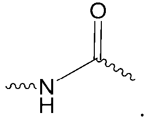

In some embodiments, R₁ is selected from: H, halogen, substituted or unsubstituted C₂₋₆ alkynyl, substituted or unsubstituted C₂₋₆ alkenyl, substituted or unsubstituted C₁₋₆ alkyl, substituted or unsubstituted C₁₋₆ alkoxy, cyano, -NO₂, -OH. In some embodiments, R₁ is selected from: H, halogen, substituted or unsubstituted C₁₋₆ alkyl, substituted or unsubstituted C₁₋₆ alkoxy, cyano, - NO₂, -OH. In some embodiments, R₁ is selected from: H, halogen, substituted or unsubstituted C₁₋₆ alkyl. In some embodiments, R₁ is selected from: H and halogen.

In some embodiments, R₂ and R₃ are each independently selected from: H, substituted or unsubstituted C₁₋₆ alkoxy, halogen, substituted or unsubstituted C₁₋₆ alkyl, cyano, -NO₂, -OH, substituted or unsubstituted C₃₋₈ cycloalkyl, substituted or unsubstituted 3- to 8-membered heterocyclyl, substituted or unsubstituted C₆₋₁₀ aryl, substituted or unsubstituted 5- to 10-membered heteroaryl, wherein said heterocyclyl or heteroaryl comprises 1, 2 or 3 heteroatoms selected from the group consisting of N, O, S and P. In some embodiments, R₂ and R₃ are each independently selected from: H, halogen, substituted or unsubstituted C₁₋₆ alkyl, cyano, -NO₂, substituted or unsubstituted C₆₋₁₀ aryl, substituted or unsubstituted 5- to 10-membered heteroaryl, wherein said heteroaryl comprises 1, 2 or 3 heteroatoms selected from the group consisting of N, O, S and P. In some embodiments, R₂ and R₃ are each independently selected from: H, halogen, substituted or unsubstituted C₁₋₆ alkyl, substituted or unsubstituted C₆₋₁₀ aryl. In some embodiments, R₂ and R₃ are each independently selected from: H, C₁₋₆ alkyl and C₆₋₁₀ aryl.

In some embodiments, R₅ and R₆ are each independently selected from: H, oxo (=O), substituted or unsubstituted C₁₋₆ alkoxy, halogen, substituted or unsubstituted C₂₋₆ alkynyl, substituted or unsubstituted C₂₋₆ alkenyl, substituted or unsubstituted C₁₋₆ alkyl, cyano, -NO₂, -OH. In some embodiments, R₅ and R₆ are each independently selected from: H, oxo (=O), substituted or unsubstituted C₁₋₆ alkoxy, halogen, substituted or unsubstituted C₁₋₆ alkyl, cyano, -NO₂, -OH. In some embodiments, R₅ and R₆ are each independently selected from: H, oxo (=O), substituted or unsubstituted C₁₋₆ alkyl. In some embodiments, R₅ and R₆ are each independently selected from: H and C₁₋₆ alkyl.

In some embodiments, m is any integer of 0, 1 or 2. In some embodiments, m is 0 or 1. In some embodiments, m is 0.

In some embodiments, q is any integer of 0, 1 or 2. In some embodiments, q is 0 or 1. In some embodiments, q is 1.

In some embodiments, the structure composed of ring B and ring C is : 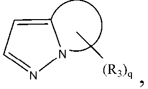 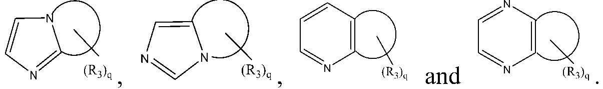 In some embodiments, the structure composed of ring B and ring C is 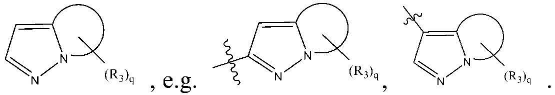 **In** some embodiments, the structure composed of ring B and ring C is selected from the group consisting of:

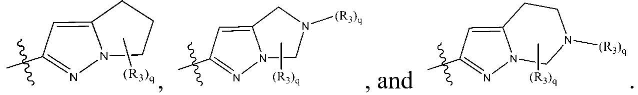

In some embodiments, the compound of Formula A provided in the present disclosure, or a pharmaceutically acceptable salt thereof, or an enantiomer, diastereomer, tautomer, solvate, polymorph, prodrug or metabolite thereof, has the structure of Formula III-1 below: 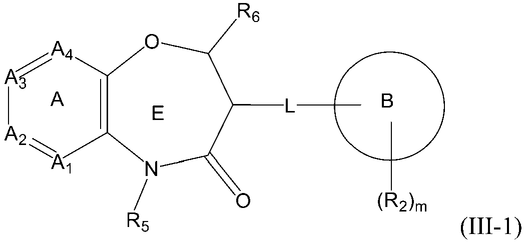 in formula III-1:
A₁, A₂, A₃, and A₄ are each independently selected from: CR₁ or N;
ring B is selected from: substituted or unsubstituted C₃₋₈ cycloalkyl, substituted or unsubstituted 3- to 8-membered heterocyclyl, substituted or unsubstituted C₆₋₁₀ aryl, substituted or unsubstituted 5- to 10-membered heteroaryl, wherein the heterocyclyl or heteroaryl comprises 1, 2, or 3 heteroatoms selected from the group consisting of N, O, S, and P;
L is selected from: 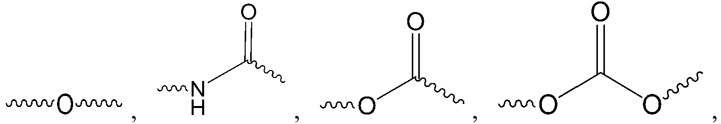 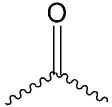 and C₁₋₁₀ alkylene;
R₁ is selected from: H, halogen, substituted or unsubstituted C₂₋₆ alkynyl, substituted or unsubstituted C₂₋₆ alkenyl, substituted or unsubstituted C₁₋₆ alkyl, substituted or unsubstituted C₁₋₆ alkoxy, cyano, -NO₂, -OH, substituted or unsubstituted C₃₋₈ cycloalkyl, substituted or unsubstituted 3- to 8- membered heterocyclyl, substituted or unsubstituted C₆₋₁₀ aryl, substituted or unsubstituted 5- to 10- membered heteroaryl, substituted or unsubstituted 3- to 8- membered heterocyclyl spiro 3- to 8- membered heterocyclyl, substituted or unsubstituted 3- to 8- membered heterocyclyl fused 3- to 8- membered heterocyclyl, substituted or unsubstituted C₃₋₈ cycloalkyl spiro C₃₋₈ cycloalkyl, substituted or unsubstituted C₃₋₈ cycloalkyl fused C₃₋₈ cycloalkyl, substituted or unsubstituted 3- to 8- membered heterocyclyl fused C₆₋₁₀ aryl, substituted or unsubstituted 3- to 8- membered heterocyclyl fused 5- to 10- membered heteroaryl, substituted or unsubstituted C₃₋₈ cycloalkyl fused C₆₋₁₀ aryl, substituted or unsubstituted C₃₋₈ cycloalkyl fused 5- to 10- membered heteroaryl, wherein the heterocyclyl or the heteroaryl comprises 1, 2 or 3 heteroatoms selected from the group consisting of N, O, S and P;
R₂, R₅, and R₆ are each independently selected from: H, oxo (=O), substituted or unsubstituted C₁₋₆ alkoxy, halogen, substituted or unsubstituted C₂₋₆ alkynyl, substituted or unsubstituted C₂₋₆ alkenyl, substituted or unsubstituted C₁₋₆ alkyl, cyano, -NO₂, -OH, substituted or unsubstituted C₃₋₈ cycloalkyl, substituted or unsubstituted 3- to 8-membered heterocyclyl, substituted or unsubstituted C₆₋₁₀ aryl, substituted or unsubstituted 5- to 10-membered heteroaryl, wherein the heterocyclyl or heteroaryl comprises 1, 2, or 3 heteroatoms selected from the group consisting of N, O, S, and P;
m is any integer of 0, 1, 2 or 3;
the "substituted" means that one or more hydrogen atoms on the group are substituted with substituents selected from the group consisting of: halogen, -OH, -NO₂, -NH₂, -NH(unsubstituted or halogenated C₁₋₆ alkyl), -N(unsubstituted or halogenated C₁₋₆ alkyl)₂, -CN, unsubstituted C₁₋₈ alkyl or C₁₋₈ alkyl substituted with halogen or hydroxy, unsubstituted or halogenated C₁₋₈ alkoxy, unsubstituted or halogenated C₁₋₈ alkoxy-C₁₋₈ alkyl, unsubstituted or halogenated C₃₋₈ cycloalkyl-C₁₋₈ alkyl, unsubstituted or halogenated C₁₋₆ alkylcarbonyl, unsubstituted or halogenated C₁₋₆ alkoxycarbonyl, hydroxamic acid group, unsubstituted or halogenated C₁₋₆ alkylthio, - S(O)₂N(unsubstituted or halogenated C₁₋₆ alkyl)₂, -S(O)₂ unsubstituted or halogenated C₁₋₆ alkyl, - N(unsubstituted or halogenated C₁₋₆ alkyl)S(O)₂N(unsubstituted or halogenated C₁₋₆ alkyl)₂, - S(O)N(unsubstituted or halogenated C₁₋₆ alkyl)₂, -S(O)(unsubstituted or halogenated C₁₋₆ alkyl), - N(unsubstituted or halogenated C₁₋₆ alkyl)S(O)N(unsubstituted or halogenated C₁₋₆ alkyl)₂, - N(unsubstituted or halogenated C₁₋₆ alkyl)S(O)(unsubstituted or halogenated C₁₋₆ alkyl), unsubstituted or halogenated C₅₋₁₀ aryl, unsubstituted or halogenated 5- to 10- membered heteroaryl, unsubstituted or halogenated C₃₋₈ cycloalkyl and unsubstituted or halogenated 3- to 8- membered heterocyclyl, wherein the heterocyclyl and the heteroaryl comprise 1, 2, 3 or 4 heteroatoms selected from the group consisting of N, O and S.

In some embodiments, A₁, A₂, A₃ and A₄ are C.

In some embodiments, ring B is substituted or unsubstituted C₆₋₁₀ aryl, substituted or unsubstituted 5- to 10-membered heteroaryl, wherein said heteroaryl comprises 1, 2 or 3 heteroatoms selected from the group consisting of N, O, S and P. In some embodiments, ring B is substituted or unsubstituted 5- to 10-membered heteroaryl, wherein said heteroaryl comprises 1, 2 or 3 heteroatoms selected from the group consisting of N, O, S and P. In some embodiments, ring B is substituted or unsubstituted 5- to 7-membered heteroaryl, wherein said heteroaryl comprises 1, 2 or 3 nitrogen atoms. In some embodiments, ring B is selected from: pyridinyl, imidazolyl, pyrrolyl, pyrazolyl, pyrimidinyl, pyrazinyl, triazolyl and triazinyl. In some embodiments, ring B is pyridinyl.

In some embodiments, L is selected from 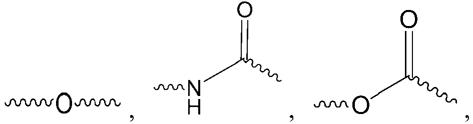 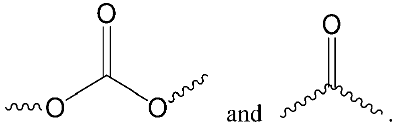 In some embodiments, L is selected from: 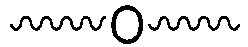 and

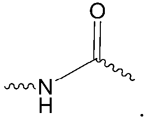

In some embodiments, R₁ is selected from: H, halogen, substituted or unsubstituted C₂₋₆ alkynyl, substituted or unsubstituted C₂₋₆ alkenyl, substituted or unsubstituted C₁₋₆ alkyl, substituted or unsubstituted C₁₋₆ alkoxy, cyano, -NO₂, -OH, substituted or unsubstituted C₃₋₈ cycloalkyl, substituted or unsubstituted 3- to 8-membered heterocyclyl, substituted or unsubstituted 3- to 8-membered heterocyclyl spiro 3- to 8-membered heterocyclyl, substituted or unsubstituted 3- to 8-membered heterocyclyl fused 3- to 8-membered heterocyclyl, substituted or unsubstituted C₃₋₈ cycloalkyl spiro C₃₋₈ cycloalkyl, substituted or unsubstituted C₃₋₈ cycloalkyl fused C₃₋₈ cycloalkyl, wherein the heterocyclyl comprises 1, 2, or 3 heteroatoms selected from the group consisting of N, O, S, and P. In some embodiments, R₁ is selected from: H, halogen, substituted or unsubstituted C₂₋₆ alkynyl, substituted or unsubstituted C₂₋₆ alkenyl, substituted or unsubstituted C₁₋₆ alkyl, substituted or unsubstituted C₁₋₆ alkoxy, substituted or unsubstituted 3- to 8-membered heterocyclyl, substituted or unsubstituted 3- to 8-membered heterocyclyl spiro 3- to 8-membered heterocyclyl, wherein the heterocyclyl comprises 1, 2, or 3 heteroatoms selected from the group consisting of N, O, S, and P. In some embodiments, R₁ is selected from: H, halogen, unsubstituted C₂₋₆ alkynyl or C₂₋₆ alkynyl substituted with C₃₋₈ cycloalkyl, substituted or unsubstituted 3- to 8-membered heterocyclyl, substituted or unsubstituted 3- to 8-membered heterocyclyl spiro 3- to 8-membered heterocyclyl, wherein said heterocyclyl comprises 1, 2 or 3 heteroatoms selected from the group consisting of N, O, S and P. In some embodiments, R₁ is selected from: H, halogen, substituted or unsubstituted C₂₋₆ alkynyl, C₁₋₆ alkoxy, cyano, substituted or unsubstituted 3- to 8-membered heterocyclyl, substituted or unsubstituted 3- to 8-membered heterocyclyl spiro 3- to 8-membered heterocyclyl, wherein the C₂₋₆ alkynyl is optionally substituted with substituents selected from the group consisting of C₃₋₈ cycloalkyl, C₁₋₆ alkyl, C₁₋₆ alkyl substituted with hydroxyl, and 3- to 8-membered heterocyclyl, and said heterocyclyl comprises 1, 2 or 3 heteroatoms selected from the group consisting of N, O, S and P.

In some embodiments, R₂ is selected from: H, oxo (=O), substituted or unsubstituted C₁₋₆ alkoxy, halogen, substituted or unsubstituted C₁₋₆ alkyl, cyano, -NO₂, -OH, substituted or unsubstituted C₆₋₁₀ aryl, substituted or unsubstituted 5- to 10-membered heteroaryl, wherein the heteroaryl comprises 1, 2, or 3 heteroatoms selected from the group consisting of N, O, S, and P. In some embodiments, R₂ is selected from: H, substituted or unsubstituted C₁₋₆ alkoxy, halogen, substituted or unsubstituted C₁₋₆ alkyl, substituted or unsubstituted C₆₋₁₀ aryl, substituted or unsubstituted 5- to 10-membered heteroaryl, wherein said heteroaryl comprises 1, 2 or 3 heteroatoms selected from the group consisting of N, O, S and P. In some embodiments, R₂ is selected from: H, C₁₋₆ alkoxy optionally substituted with C₆₋₁₀ aryl, substituted or unsubstituted 7-to 10-membered heteroaryl, wherein said heteroaryl comprises 1, 2 or 3 nitrogen atoms.

In some embodiments, R₅ and R₆ are each independently selected from: H, oxo (=O), substituted or unsubstituted C₁₋₆ alkoxy, halogen, substituted or unsubstituted C₂₋₆ alkynyl, substituted or unsubstituted C₂₋₆ alkenyl, substituted or unsubstituted C₁₋₆ alkyl, cyano, -NO₂, -OH. In some embodiments, R₅ and R₆ are each independently selected from: H, oxo (=O), substituted or unsubstituted C₁₋₆ alkoxy, halogen, substituted or unsubstituted C₁₋₆ alkyl, cyano, -NO₂, -OH. In some embodiments, R₅ and R₆ are each independently selected from: H, oxo (=O), substituted or unsubstituted C₁₋₆ alkyl.

In some embodiments, m is any integer of 0, 1 or 2. In some embodiments, m is 0 or 1. In some embodiments, m is 1.

In some embodiments, the compound of formula A has the structure shown in formula A' below: 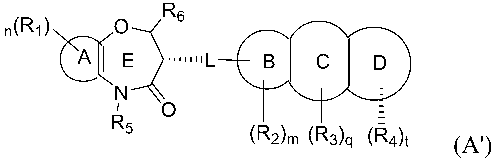 wherein R₁-R₆, ring A-ring E, L, n, m, q and t are as described in any preceding embodiment.

In some embodiments, the compound of formula A has the structure shown in formula A" below: 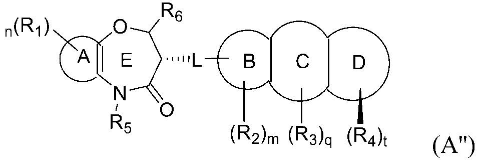 wherein R₁-R₆, ring A-ring E, L, n, m, q and t are as described in any preceding embodiment.

In some embodiments, the compound of formula A has the structure shown in formula I' below: 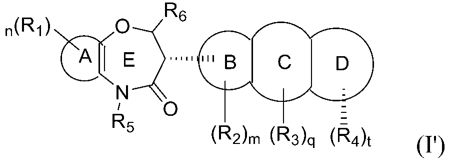 wherein R₁-R₆, ring A-ring E, L, n, m, q and t are as described in any preceding embodiment.

In some embodiments, the compound of formula A has the structure shown in formula I" below: 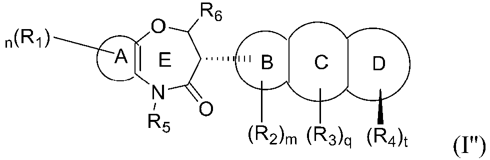 wherein R₁-R₆, ring A-ring E, L, n, m, q and t are as described in any preceding embodiment.

In some embodiments, the compound of formula A has the structure shown in formula II' below: 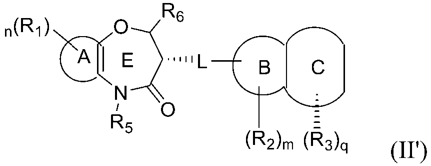 wherein R₁-R₃, R₅-R₆, ring A-ring C, ring E, L, n, m and q are as described in any preceding embodiment.

In some embodiments, the compound of formula A has the structure shown in formula II" below: 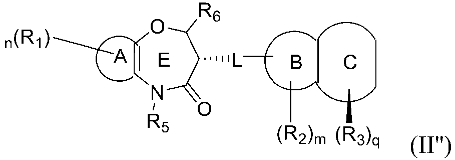 wherein R₁-R₃, R₅-R₆, ring A-ring C, ring E, L, n, m and q are as described in any preceding embodiment.

In some embodiments, the compound of formula A has the structure shown in formula III' below: 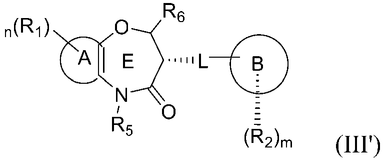 wherein R₁-R₂, R₅-R₆, ring A, ring B, ring E, L, n and m are as described in any preceding embodiment.

In some embodiments, the compound of formula A has the structure shown in formula III" below: 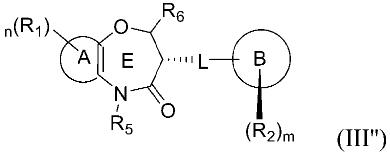 wherein R₁-R₂, R₅-R₆, ring A, ring B, ring E, L, n and m are as described in any preceding embodiment.

In some embodiments, the compound of formula A has the structure shown in formula I'-1 below: 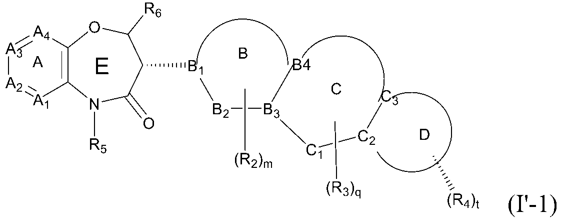 wherein A₁-A₄, B₁-B₄, C₁-C₃, R₂-R₆, ring A-ring E, m, q and t are as described in any preceding embodiment.

In some embodiments, the compound of formula A has the structure shown in formula I"-1 below: 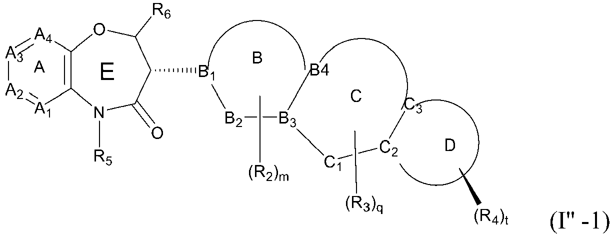 wherein A₁-A₄, B₁-B₄, C₁-C₃, R₂-R₆, ring A-ring E, m, q and t are as described in any preceding embodiment.

In some embodiments, the compound of formula A has the structure shown in formula I"-1 below: 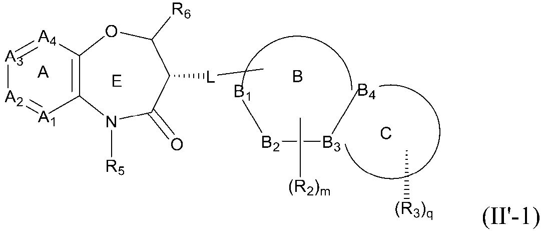 wherein A₁-A₄, B₁-B₄, R₂-R₃, R₅-R₆, ring A, ring B, ring C, ring E, L, m and q are as described in any preceding embodiment.

In some embodiments, the compound of formula A has the structure shown in formula II'-2 below: 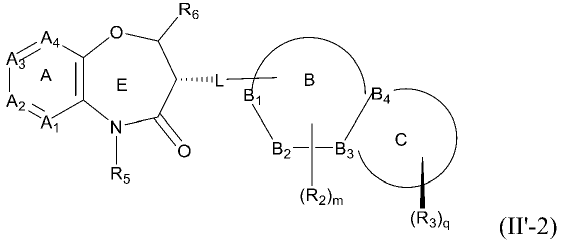 wherein A₁-A₄, B₁-B₄, R₂-R₃, R₅-R₆, ring A, ring B, ring C, ring E, L, m and q are as described in any preceding embodiment.

In some embodiments, the compound of formula A has the structure shown in formula III'-1 below: 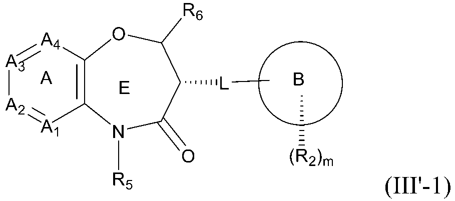 wherein A₁-A₄, R₂, R₅-R₆, ring A, ring B, ring E, L and m are as described in any preceding embodiment.

In some embodiments, the compound of formula A has the structure shown in formula III'-2 below: 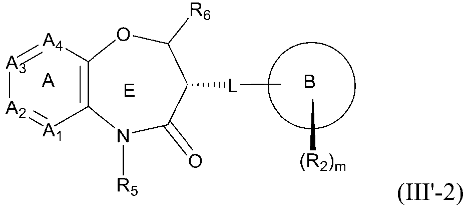 wherein A₁-A₄, R₂, R₅-R₆, ring A, ring B, ring E, L and m are as described in any preceding embodiment.

In some embodiments, the compound of formula A provided herein, or a pharmaceutically acceptable salt thereof, or a solvate, isotopically substituted derivative, polymorph, prodrug or metabolite thereof, is selected from the following structures: 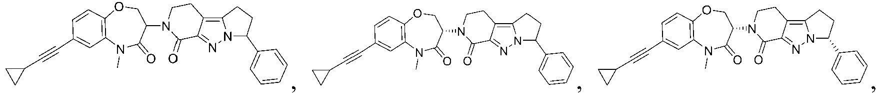 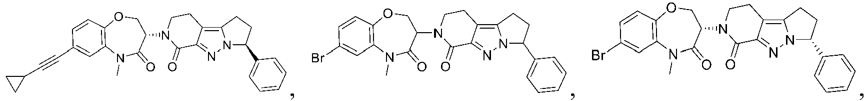 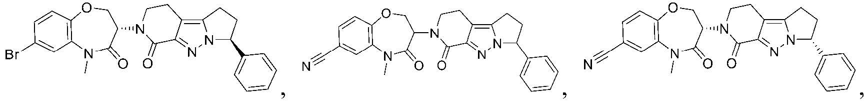 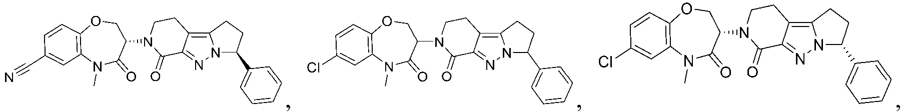 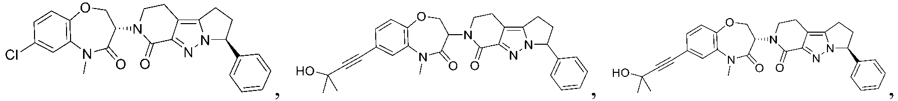 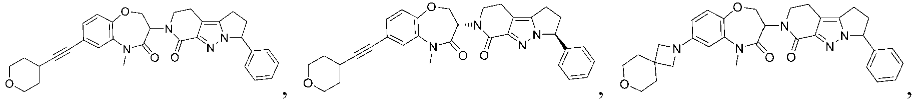 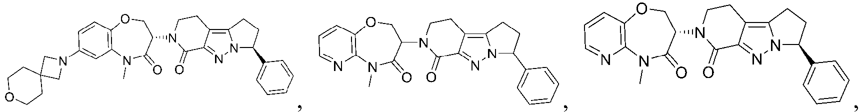 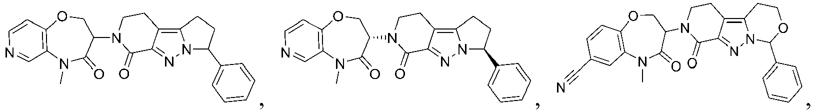 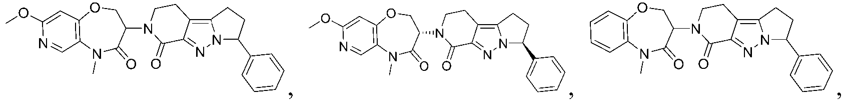 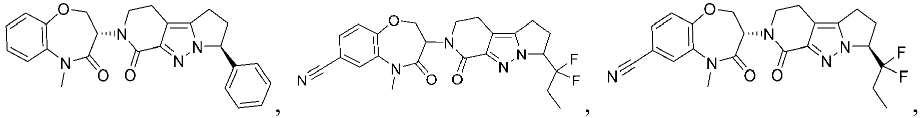 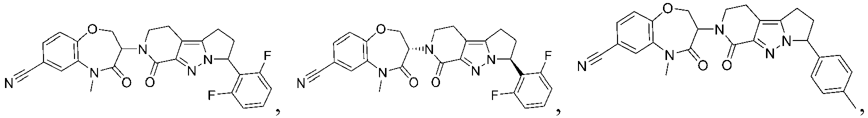 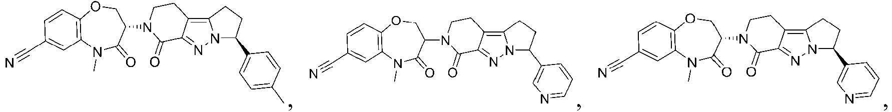 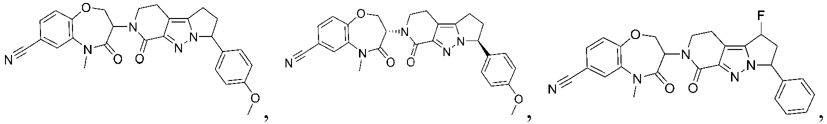 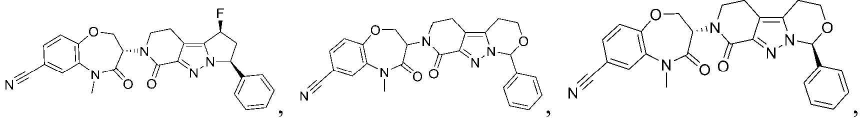 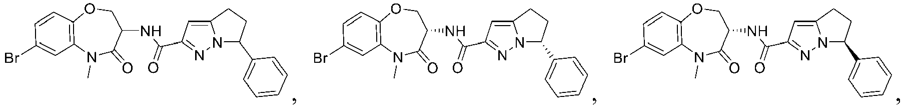 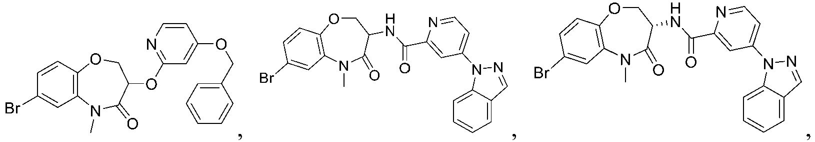 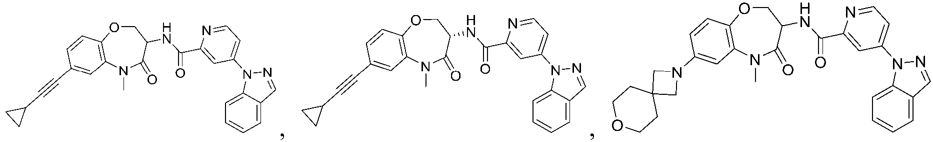 and

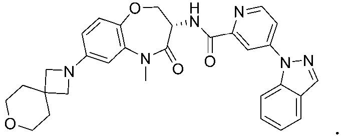

### Pharmaceutical composition

The present disclosure provides a pharmaceutical composition comprising a compound of formula A or any sub-formula thereof (including but not limited to the compounds of formula I, formula II, formula III) as described in any embodiment herein, or a pharmaceutically acceptable salt thereof, or an enantiomer, diastereomer, tautomer, solvate, polymorph, prodrug or metabolite thereof, and a pharmaceutically acceptable excipient or carrier.

The pharmaceutical composition described herein may comprise 0.01-99% w (weight percentage), 0.05-80% w, 0.10-70% w, and/or even 0.10-50% w of the compound of formula A or any sub-formula thereof (including but not limited to the compounds of formula I, formula II, formula III) or a pharmaceutically acceptable salt thereof, or an enantiomer, diastereomer, tautomer, solvate, polymorph, prodrug or metabolite thereof as an active ingredient (e.g., the sole active ingredient), depending on the mode of administration.

In another aspect, the present disclosure further provides a method for preparing the pharmaceutical composition of the present disclosure, said pharmaceutical composition comprising mixing the compound of formula A or any sub-formula thereof (including but not limited to the compound of formula I, formula II, formula III) or a pharmaceutically acceptable salt thereof, or an enantiomer, diastereomer, tautomer, solvate, polymorph, prodrug or metabolite thereof as described in any embodiment herein with a pharmaceutically acceptable excipient or carrier.

Herein, the pharmaceutical composition may be administered topically (e.g., to the skin or lung and/or airway), for example in the forms of creams, solutions, suspensions, hexafluoroalkane aerosols and dry powder formulations; or systemically, for example by oral administration in the form of tablets, capsules, syrups, powders or granules; or by gastrointestinal administration in the form of solutions or suspensions; or subcutaneously; or rectally in the form of suppositories; or transdermally.

The compositions of the present disclosure may be obtained by conventional methods using conventional pharmaceutical excipients well known in the art. Thus, the pharmaceutical composition intended for oral use may comprise, for example, one or more coloring agents, sweetening agents, flavoring agents and/or preservatives.

Suitable pharmaceutically acceptable excipients for tablet preparation comprise, for example, inert diluents such as lactose, sodium carbonate, calcium phosphate or calcium carbonate; granulating and disintegrating agents such as corn starch or algenic acid; binding agents such as starch; lubricating agents such as magnesium stearate, stearic acid or talc; preservatives such as ethyl paraben or propyl paraben; and antioxidants such as ascorbic acid. Tablets may be uncoated or coated using conventional coating materials and methods known in the art to improve disintegration of the tablets and subsequent absorption of the active ingredient in the gastrointestinal tract, or to improve stability and/or appearance of the tablets.

Compositions for oral use may be in the form of hard gelatin capsules wherein the active ingredient is mixed with an inert solid diluent, for example, calcium carbonate, calcium phosphate or kaolin; or as soft gelatin capsules wherein the active ingredient is mixed with water or an oil such as peanut oil, liquid paraffin or olive oil.

Aqueous suspensions usually comprise the active ingredient in the form of fine powder together with one or more suspending agents such as sodium carboxymethylcellulose, methylcellulose, hydroxypropylmethylcellulose, sodium alginate, polyvinylpyrrolidone, tragacanth gum and acacia gum; dispersing or wetting agents such as lecithin or condensation products of 1, 2-alkylene oxides with fatty acids (e.g., polyoxyethylene stearate), or condensation products of ethylene oxide with long-chain fatty alcohols (e.g., heptadecaethyleneoxycetanol), or condensation products of ethylene oxide with partial esters derived from fatty acids and hexitol (e.g., polyoxyethylene sorbitol monooleate), or condensation products of ethylene oxide with partial esters derived from fatty acids and hexitol anhydrides (e.g., polyoxyethylene sorbitan monooleate). The aqueous suspensions may also comprise one or more preservatives (e.g., ethyl paraben or propyl paraben), antioxidants (e.g., ascorbic acid), coloring agents, flavoring agents and/or sweetening agents (e.g., sucrose, saccharin or aspartame).

Oily suspensions may be prepared by suspending the active ingredient in a vegetable oil (e.g., peanut oil, olive oil, sesame oil or coconut oil) or in a mineral oil (e.g., liquid paraffin). Oily suspensions may further comprise a thickening agent such as beeswax, hard paraffin or cetyl alcohol. Sweetening agents and flavoring agents such as those listed above may be added to provide a palatable oral preparation. These compositions may be preserved by addition of an antioxidant such as ascorbic acid.

Dispersible powders and granules suitable for preparing aqueous suspensions upon addition of water generally comprise the active ingredient, dispersing/wetting agents, suspending agents and one or more preservatives. The applicable dispersing agents or wetting agents, and suspending agents are as exemplified above. Additional excipients such as sweetening agents, flavoring agents and coloring agents may also be present.

In some embodiments, the pharmaceutical composition of the present disclosure can also be in the form of oil-in-water emulsions. The oily phase may be a vegetable oil such as olive oil or peanut oil, a mineral oil such as liquid paraffin, or a mixture thereof. Suitable emulsifiers may be natural-occurring gums such as acacia gum or tragacanth gum, natural-occuring phospholipids such as soybean phospholipid and lecithin, esters or partial esters derived from fatty acids and hexitol anhydrides (e.g., sorbitan monooleate), and condensation products of these partial esters with ethylene oxide such as polyoxyethylene sorbitan monooleate. The emulsions may further comprise sweetening agents, flavoring agents and preservatives.

Syrups and elixirs may be formulated with sweetening agents such as glycerol, propylene glycol, sorbitol, aspartame or sucrose, and may also comprise analgesics, preservatives, flavoring agents and/or coloring agents.

The pharmaceutical compositions described herein may also be in the form of sterile injectable aqueous or oily suspensions, which may be prepared according to known methods using one or more of the suitable dispersing or wetting agents and suspending agents mentioned above. The sterile injectable formulations may also be a sterile injectable solution or suspension in a non-toxic, parenterally acceptable diluent or solvent (e.g., a solution in 1,3-butanediol).

Suppositories may be prepared by mixing the active ingredient with a suitable non-irritating excipient which is solid at ordinary temperature but liquid at rectal temperature and will therefor melt in the rectum to release the drug. Suitable excipients comprise, for example, cocoa butter and polyethylene glycols.

Topical formulations such as creams, ointments, gels or aqueous or oily solutions or suspensions may generally be prepared by formulating the active ingredient with a conventional, topically acceptable excipient or diluent using conventional methods known in the art.

Compositions for insufflation can be administered as a fine and dispersed powder having an average particle size of 30 µm or less. The powder comprises the active ingredient alone or is diluted with one or more physiologically acceptable carriers such as lactose. The powder for insufflation is then conveniently placed in a capsule containing, for example, 1-50 mg of the active ingredient, for use with a turbo-inhaler device, e.g., for insufflation of the known drug sodium cromoglycate.

Compositions for administration by inhalation may be in the form of a conventional pressurized aerosol arranged to dispense the active ingredient as an aerosol containing fine and dispersed solid or liquid droplets. Conventional aerosol propellants such as volatile fluorinated hydrocarbons or hydrocarbons may be used, and the aerosol device is conveniently arranged to dispense a metered quantity of the active ingredient.

According to well-known principles of medicine, the size of the dose of the compounds of the present disclosure for therapeutic purposes will naturally vary according to the nature and severity of the disease, the age and gender of the animal or patient, and the route of administration.

In general, the compound of formula A or any sub-formula thereof (including but not limited to the compounds of formula I, formula II, formula III) or a pharmaceutically acceptable salt thereof, or an enantiomer, diastereomer, tautomer, solvate, polymorph, prodrug or metabolite thereof described herein is administered so as to obtain a daily dose ranging from, for example, 0.1 mg to 1000 mg of the active ingredient per kg body weight, and if desired, may be administered in divided doses. However, the daily dose must be varied depending on the host being treated, the particular route of administration, and the severity of the disease being treated. Therefore, the optimum dose may be determined by the physician treating any particular patient. Generally, lower doses are used when the parenteral route is employed. Thus, for example, for intravenous administration, a dose ranging from, e.g., 0.1 mg to 30 mg of the active ingredient per kg body weight will generally be used. Similarly, for administration by inhalation, a dose ranging from, e.g., 0.1 mg to 25 mg of the active ingredient per kg body weight will generally be used. However, oral administration is preferred. For example, formulations intended for oral administration to humans will generally comprise from 0.1 mg to 2 g of the active ingredient.

For information on other formulations, routes of administration and dosage regimens, reference may be made to the disclosures in Comprehensive Medicinal Chemistry (Corwin Hansch; Chairman of Editorial Board).

The pharmaceutical composition of the present disclosure may be administered alone or in combination with other pharmaceutically acceptable compounds (e.g., Ripk1 inhibitors).

The therapeutic method of the present disclosure may be administered alone or in combination with other therapeutic measures or therapeutic drugs.

### Use

The compound of formula A or any sub-formula thereof (including but not limited to the compounds of formula I, formula II, formula III) of the present disclosure, or a pharmaceutically acceptable salt thereof, or an enantiomer, diastereomer, tautomer, solvate, polymorph, prodrug or metabolite thereof, is a Ripk1 inhibitor. Therefore, the compound of formula A or any sub-formula thereof (including but not limited to the compounds of formula I, formula II, formula III) or a pharmaceutically acceptable salt thereof, or an enantiomer, diastereomer, tautomer, solvate, polymorph, prodrug or metabolite thereof can be used for treating or preventing a Ripk1-related disease or disorder, or for preparing a medicament for treating or preventing a Ripk1-related disease or disorder.

Accordingly, the present disclosure provides a method for treating or preventing a Ripk1-related disease or disorder in a subject, said method comprising administering to the subject a therapeutically effective amount of the compound of formula A or any sub-formula thereof (including but not limited to the compounds of formula I, formula II, formula III) or a pharmaceutically acceptable salt thereof, or an enantiomer, diastereomer, tautomer, solvate, polymorph, prodrug or metabolite thereof, or the pharmaceutical composition of the present disclosure.

The present disclosure also provides use of the compound of formula A or any sub-formula thereof (including but not limited to the compounds of formula I, formula II, formula III) or a pharmaceutically acceptable salt thereof, or an enantiomer, diastereomer, tautomer, solvate, polymorph, prodrug or metabolite thereof, or the pharmaceutical composition of the present disclosure, in the manufacture of a medicament for treating or preventing a Ripk1-related disease or disorder.

The present disclosure also provides the compound of formula A or any sub-formula thereof (including but not limited to the compounds of formula I, formula II, formula III) or a pharmaceutically acceptable salt thereof, or an enantiomer, diastereomer, tautomer, solvate, polymorph, prodrug or metabolite thereof, or the pharmaceutical composition, of the present disclosure, for use in treating or preventing a Ripkl-related disease or disorder.

As used herein, "Ripk1-related" means that Ripk1 plays a role in the occurrence and/or development of the disease. The Ripk1-related disease or disorder described herein may also be referred to as Ripk1-mediated disease or disorder.

In some embodiments, the Ripk1-mediated disease described herein comprise inflammatory diseases and diseases associated with necrotic cell death.

In some embodiments, the Ripk1-mediated disease comprise stroke, inflammatory bowel disease, ulcerative colitis, Crohn's disease, psoriasis, rheumatoid arthritis, NASH and heart failure.

In some embodiments, the Ripk1-related disease or disorder include, but are not limited to, inflammatory diseases (e.g., Crohn's disease and ulcerative colitis, inflammatory bowel disease, asthma, graft-versus-host disease, chronic obstructive pulmonary disease), inflammatory skin diseases (e.g., psoriasis, eczema), autoimmune diseases (e.g., Graves' disease, rheumatoid arthritis, systemic lupus erythematosus, psoriasis; destructive bone diseases such as bone resorption diseases, osteoarthritis, osteoporosis, multiple myeloma-related bone disease); proliferative diseases (leukemia, such as myeloid leukemia, comprising granulocytic leukemia, lymphocytic leukemia, monocytic leukemia, erythroleukemia, further comprising acute myeloid leukemia, chronic myelogenous leukemia), angiogenesis disorders (e.g., angiogenesis disorders comprising solid tumors, ocular neovascularization and infantile hemangioma), infectious diseases (e.g., sepsis, septic shock and shigellosis); neurodegenerative diseases (e.g., Alzheimer's disease, Parkinson's disease, amyotrophic lateral sclerosis, cerebral ischemia or neurodegenerative diseases caused by traumatic injury), tumors and viral diseases (e.g., metastatic melanoma, Kaposi's sarcoma, multiple myeloma, fibrosarcoma, lymphoma, HIV infection and CMV retinitis, AIDS).

In some embodiments, the Ripkl-related disease or disorder includes, but is not limited to, pancreatitis (acute or chronic), asthma, allergies, adult respiratory distress syndrome, chronic obstructive pulmonary disease, glomerulonephritis, rheumatoid arthritis, systemic lupus erythematosus, scleroderma, chronic thyroiditis, Graves' disease, autoimmune gastritis, diabetes, autoimmune hemolytic anemia, autoimmune neutropenia, thrombocytopenia, atopic dermatitis, chronic active hepatitis, myasthenia gravis, amyotrophic lateral sclerosis, multiple sclerosis, inflammatory bowel disease, ulcerative colitis, Crohn's disease, psoriasis, graft-versus-host disease, endotoxin-induced inflammatory response, tuberculosis, atherosclerosis, muscle degeneration, cachexia, psoriatic arthritis, Reiter's syndrome, gout, traumatic arthritis, rubella arthritis, acute synovitis, pancreatic β cell disease; diseases characterized by massive neutrophil infiltration; rheumatoid spondylitis, gouty arthritis and other arthritic conditions, cerebral malaria, chronic pulmonary inflammation, silicosis, pulmonary sarcoidosis, bone resorption diseases, allograft rejection, infection-induced fever and myalgia, cachexia secondary to infection, corpus luteum formation, scar tissue formation, ulcerative colitis, fever, influenza, osteoporosis, osteoarthritis, acute myeloid leukemia, chronic myeloid leukemia, metastatic melanoma, Kaposi's sarcoma, multiple myeloma, sepsis, septic shock and shigellosis; Alzheimer's disease, Parkinson's disease, cerebral ischemia or neurodegenerative diseases caused by traumatic injury; angiogenesis disorders comprising solid tumors, ocular neovascularization and infantile hemangioma; viral diseases comprising acute hepatitis infection (comprising hepatitis A, hepatitis B and hepatitis C), HIV infection and CMV retinitis, Acquired Immunodeficiency Syndrome, ARC or malignant tumors and herpes; stroke, myocardial ischemia, stroke heart attack, organ ischemia, vascular proliferation, cardiac and renal reperfusion injury, thrombosis, cardiac hypertrophy, thrombin-induced platelet aggregation, endotoxemia and/or toxic shock syndrome, disorders associated with prostaglandin endoperoxide synthase-2, and pemphigus vulgaris.

In some embodiments, the Ripk1-related disease or disorder are selected from: stroke, inflammatory bowel disease, Crohn's disease and ulcerative colitis, allograft rejection, rheumatoid arthritis, psoriasis, ankylosing spondylitis, psoriatic arthritis and pemphigus vulgaris. Alternatively, the preferred disorder is selected from ischemia-reperfusion injury, comprising cerebral ischemia-reperfusion injury caused by stroke and myocardial ischemia-reperfusion injury caused by myocardial infarction.

In some embodiments, the present disclosure also provides a method for treating an inflammatory skin disease in a subject, said method comprising administering to the subject a therapeutically effective amount of the compound of formula A or any sub-formula thereof (including but not limited to the compounds of formula I, formula II, formula III) or a pharmaceutically acceptable salt thereof, or an enantiomer, diastereomer, tautomer, solvate, polymorph, prodrug or metabolite thereof, or the pharmaceutical composition, of the present disclosure. The present disclosure also provides use of the compound of formula A or any sub-formula thereof (including but not limited to the compounds of formula I, formula II, formula III) or a pharmaceutically acceptable salt thereof, or an enantiomer, diastereomer, tautomer, solvate, polymorph, prodrug or metabolite thereof, or the pharmaceutical composition of the present disclosure, in the manufacture of a medicament for treating or preventing an inflammatory skin disease.

In some embodiments, the present disclosure also provides a method for treating eczema, atopic eczema, hand eczema or radiodermatitis in a subject, said method comprising administering to the subject a therapeutically effective amount of the compound of formula A or any sub-formula thereof (including but not limited to the compounds of formula I, formula II, formula III) or a pharmaceutically acceptable salt thereof, or an enantiomer, diastereomer, tautomer, solvate, polymorph, prodrug or metabolite thereof, or the pharmaceutical composition, of the present disclosure. The present disclosure also provides use of the compound of formula A or any sub-formula thereof (including but not limited to the compounds of formula I, formula II, formula III) or a pharmaceutically acceptable salt thereof, or an enantiomer, diastereomer, tautomer, solvate, polymorph, prodrug or metabolite thereof, or the pharmaceutical composition, of the present disclosure, in the manufacture of a medicament for treating, preventing and/or alleviating contact eczema, atopic eczema, hand eczema and radiodermatitis.

In the above methods and uses, the drug can be administered in a suitable manner, for example topically (e.g., to the skin or lung and/or airway), such as creams, solutions, suspensions, hexafluoroalkane aerosols and dry powder formulations; or systemically, for example by oral administration in the form of tablets, capsules, syrups, powders or granules; or by gastrointestinal administration as solutions or suspensions; or subcutaneously; or rectally as suppositories; or transdermally.

The disclosure is further described below in conjunction with specific examples. It is understood that these examples are merely for illustrative purposes rather than intended to limit the scope of the disclosure. For the experimental methods without specific conditions indicated in the following examples, the conventional conditions or the conditions suggested by the manufacturer are usually followed. Percentages and parts are by weight unless otherwise indicated.

The starting materials used in the following examples are commercially available from chemical suppliers such as Aldrich, TCI, Alfa Aesar, Bide, Energy, or can be synthesized by known methods.

| | | | |
|---|---|---|---|
| Ti(OEt)₄ | Tetraethyl titanate | DMF | N,N-Dimethylformamide |
| LiBH₄ | Lithium borohydride | Na₂CO₃ | Sodium carbonate |
| TFA | Trifluoroacetic acid | EtOH | Ethanol |
| LDA | Lithium diisopropylamide | CBr₄ | Carbon tetrabromide |
| Pd(AmPhos)₂Cl₂ | Dichlorobis[di-tert-butyl(4-dimethylaminophenyl)phosphine]palladium(II) | Ph₃P | Triphenylphosphine |
| Cs₂CO₃ | Cesium carbonate | NBS | N-Bromosuccinimide |
| DMAc or DMA | N,N-Dimethylacetamide | BPO | Benzoyl peroxide |
| THF | Tetrahydrofuran | TMP | 2,2,6,6-Tetramethylpiperidine |
| DCM | Dichloromethane | PBr₃ | Phosphorus tribromide |
| Ms₂O | Methanesulfonic anhydride | CCl₄ | Carbon tetrachloride |
| n-BuLi | n-Butyllithium | N₂H₄ | Hydrazine |
| Dibal-H | Diisobutylaluminium hydride | H₂SO₄ | Sulfuric acid |
| Dioxane | 1,4-Dioxane | POCl₃ | Phosphorus oxychloride |
| PPA | Polyphosphoric acid | Pd(OAc)₂ | Palladium(II) acetate |
| NaBH₄ | Sodium borohydride | EtONa | Sodium ethoxide |
| MeOH | Methanol | Pd₂(dba)₃ | Tris(dibenzylideneacetone) dipalladium(0) |
| Et₃N or TEA | Triethylamine | XantPhos | 4,5-Bis(diphenylphosphino)- |
| | | | 9,9-dimethylxanthene |
| DIPEA | N,N-Diisopropylethylamine | CH₃CN | Acetonitrile |
| HCl | Hydrogen chloride | Boc₂O | Di-tert-butyl dicarbonate |
| PMB-Br | 4-Methoxybenzyl bromide | K₂CO₃ | Potassium carbonate |
| DMAP | 4-Dimethylaminopyridine | DMF-DMA | N,N-Dimethylformamide dimethyl acetal |
| DMSO | Dimethyl sulfoxide | RuPhos | 2-Dicyclohexylphosphino-2',6'-diisopropoxy-1,1'-biphenyl |
| XPhos Pd G2 | Methanesulfonato(2-di-cyclohexylphosphino-2',4',6'-triisopropyl-1,1'-biphenylyl)(2'-amino-1,1'-biphenyl-2-yl)palladium(II) | t-BuXPhos Pd G3 | Methanesulfonato(2-di-tert-butylphosphino-2',4',6'-triisopropyl-1,1'-biphenylyl)(2'-amino-1,1'-biphenyl-2-yl)palladium(II) |

### Example 1: Synthesis of Compound 1'

### (3S)-7-(Cyclopropylethynyl)-5-methyl-3-(1-oxo-7-phenyl-4,5,6,7-tetrahydro-1H-pyrrolo[1', 2':1,5]pyrazolo[3,4-c]pyridin-2(3H)-yl)-2,3-dihydrobenzo[b][1,4]oxepin-4(5H)-one

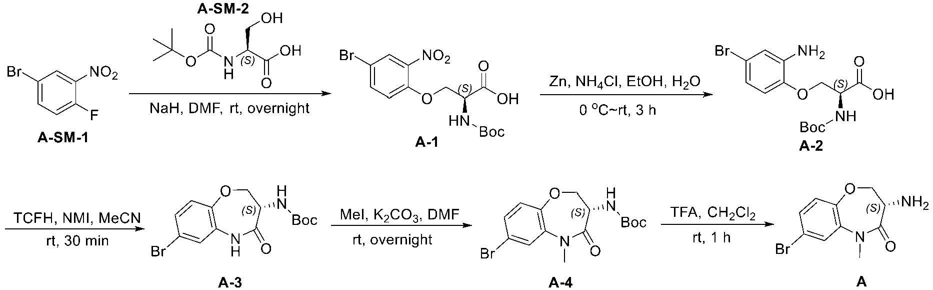

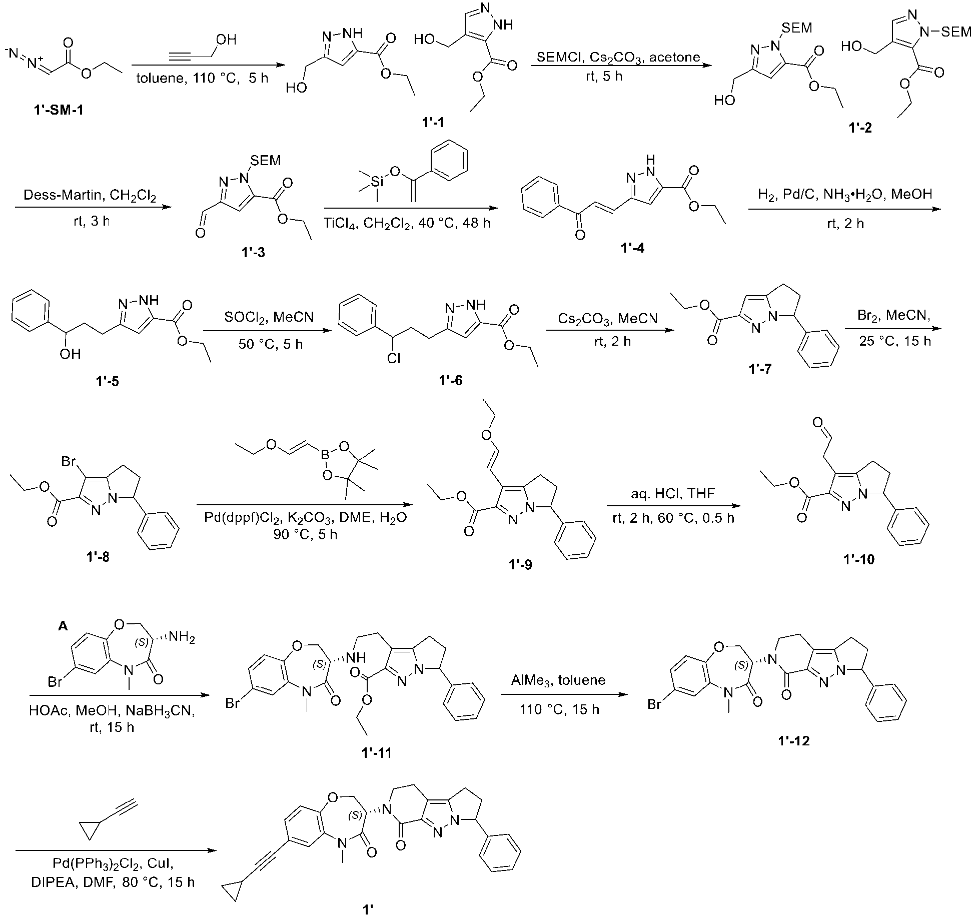

Step 1: Under argon protection at 0 °C, a solution of A-SM-2 (46.6 g, 227.3 mmol) in DMF (100 mL) was added dropwise to a suspension of sodium hydride (60% dispersed in mineral oil, 18.2 g, 454.5 mmol) in DMF (400 mL). The mixture was stirred at 0 °C for 1 hour. A solution of A-SM-1 (50.0 g, 227.3 mmol) in DMF (50 mL) was added dropwise. The reaction was carried out overnight at room temperature. The reaction mixture was poured into saturated aqueous solution of ammonium chloride and extracted with ethyl acetate (3 × 800 mL). The organic phase was combined, washed with water (3 × 1.5 L) and saturated brine (2 × 1.5 L), dried over anhydrous sodium sulfate, filtered and concentrated. The crude product was purified by column chromatography (dichloromethane/methanol = 10/1) to afford compound A-1 as a yellow oil (35.0 g, yield: 38.0%). LCMS: m/z = 304.7 [M-100+H]⁺.

Step 2: Under an ice-water bath, zinc powder (28.1 g, 432.0 mmol) was added portionwise to a suspension of A-1 (35.0 g, 86.4 mmol) and ammonium chloride (46.2 g, 863.7 mmol) in ethanol (350 mL) and water (70 mL), with the temperature not greater than 30 °C. The mixture was stirred at room temperature for 3 hours. After filtration, ethanol was removed from the filtrate under reduced pressure. Water (100 mL) and ethyl acetate (300 mL) were added. The layers were separated, and the aqueous phase was extracted with ethyl acetate (2 × 250 mL). The organic phase was combined, washed with saturated brine, dried over anhydrous sodium sulfate, filtered and concentrated to give compound A-2 as a yellow oil (25.0 g, yield: 77.1%). The crude product was used directly in the next step. LCMS: m/z = 376.8 [M+H]⁺.

Step 3: Under argon protection, N,N,N',N'-tetramethylchloroformamidinium hexafluorophosphate (20.6 g, 73.4 mmol) was added to a solution of A-2 (25.0 g, 66.6 mmol) and N-methylimidazole (11.4 g, 140.0 mmol) in acetonitrile (400 mL). The reaction was carried out at room temperature for 30 minutes. The reaction solution was poured into water and extracted with ethyl acetate (3 × 300 mL). The organic phase was combined, washed with saturated brine (500 mL), dried over anhydrous sodium sulfate, filtered and concentrated. The crude product was purified by column chromatography (petroleum ether/ethyl acetate = 10/1) to afford compound A-3 as a white solid (13.0 g, yield: 54.6%). LCMS: m/z=300.7[M+H-56]⁺.

¹H NMR (400 MHz, DMSO-d6) δ ppm: 10.04 (s, 1H), 7.30 ~7.26 (m, 2H), 7.15 (d, J = 8.0 Hz, 1H), 7.09 (d, J = 8.4 Hz, 1H), 4.37~4.27 (m, 3H), 1.37 (s, 9H).

Step 4: Under argon protection at 0 °C, potassium carbonate (10.7 g, 77.27 mmol) and iodomethane (11.0 g, 77.27 mmol) were added to a solution of A-3 (9.2 g, 25.76 mmol) in dry DMF (100 mL). The reaction was carried out overnight at room temperature. The reaction solution was poured into water and extracted with ethyl acetate (3 × 150 mL). The organic phase was combined, washed with water (3 × 300 mL) and saturated brine (2 × 300 mL), dried over anhydrous sodium sulfate, filtered and concentrated. The crude product was purified by column chromatography (petroleum ether/ethyl acetate = 20/1) to afford compound A-4 as a white solid (8.4 g, yield: 87.9%). LCMS: m/z=315.0[M+H-56]⁺.

¹H NMR (400 MHz, DMSO-d6) δ ppm: 7.74 (d, J = 2.4 Hz, 1H), 7.46 ~7.43 (m, 1H), 7.20 (d, J = 8.4 Hz, 1H), 7.17 (d, J = 8.4 Hz, 1H), 4.40~4.26 (m, 3H), 3.28 (s, 3H), 1.35 (s, 9H).

Step 5: Trifluoroacetic acid (7 mL) was added dropwise to a solution of A-4 (3.5 g, 9.43 mmol) in dichloromethane (35 mL) at 0 °C. The mixture was stirred at room temperature for 1 hour. The reaction solution was slowly poured into a saturated aqueous solution of sodium bicarbonate and extracted with ethyl acetate (3 × 200 mL). The organic phase was combined, washed with saturated brine (400 mL), dried over anhydrous sodium sulfate, filtered and concentrated. The crude product was purified by column chromatography (dichloromethane/methanol = 20/1) to afford compound A as a white solid (2.5 g, yield: 97.8%). LCMS: m/z=271.1 [M+H]⁺.

¹H NMR (400 MHz, DMSO-d6) δ ppm: 7.67 (d, J = 2.4 Hz, 1H), 7.41 ~7.38 (m, 1H), 7.13 (d, J = 8.4 Hz, 1H), 4.26~4.24 (m, 1H), 4.03~3.98 (m, 1H), 3.65~3.60 (m, 1H), 3.29 (s, 3H), 1.73 (s, 2H).

ee% = 100%.

Step 6: Propargyl alcohol (45 g, 0.79 mol) was added to a solution of 1'-SM-1 (100 g, 0.87 mol) in toluene. The mixture was stirred at 110 °C for 5 hours. The reaction solution was cooled to room temperature and concentrated under reduced pressure. Petroleum ether and ethyl acetate were added to the residue, and the mixture was filtered to give compound 1'-1 as a white solid, which is a mixture (82 g, yield: 60.0%). LCMS: m/z 171.1[M+H]⁺.

Step 7: 2-(trimethylsilyl)ethoxymethyl chloride (132 g, 0.79 mol) was added to a solution of 1'-1 (68 g, 0.40 mol) and cesium carbonate (390 g, 1.20 mol) in acetone. The mixture was stirred at room temperature for 5 hours. The reaction solution was filtered, and the filtrate was concentrated to dryness under reduced pressure. The crude product was purified by silica gel column chromatography to afford compound 1'-2 as a yellow oily liquid, which is a mixture (85 g, yield: 70.8%). LCMS: m/z 300.2[M+H]⁺.

Step 8: Dess-Martin periodinane (212 g, 0.50 mol) was added to a solution of 1'-2 (75 g, 0.25 mol) in dichloromethane. The mixture was stirred at room temperature for 3 hours. The reaction solution was filtered, and the filtrate was concentrated to dryness under reduced pressure. The crude product was purified by silica gel column chromatography to afford compound 1'-3 as a yellow oily liquid (12 g, yield: 16.1%). LCMS: m/z 298.3[M+H]⁺.

¹H NMR (400 MHz, CDCl₃) δ ppm: 10.01(s, 1H), 7.51(s, 1H), 5.92(s, 2H), 4.48(q, J = 7.2 Hz, 2H), 3.66 (t, J = 8.0 Hz, 2H), 1.46 (t, J = 7.2 Hz, 3H), 0.93 (t, J = 8.0 Hz, 2H), 0.01(s, 9H).

Step 9: Titanium tetrachloride (3.84 g, 20.2 mmol) was added to a solution of 1'-3 (2.98 g, 10.0 mmol) and trimethyl((1-phenylvinyl)oxy)silane (2.59 g, 13.5 mmol) in dichloromethane. The mixture was allowed to react at 40 °C for 48 hours. The reaction solution was diluted with water and extracted with ethyl acetate. The organic phase was combined, washed with saturated brine, dried over anhydrous sodium sulfate, filtered and concentrated under reduced pressure. The crude product was purified by silica gel column chromatography (petroleum ether/ethyl acetate = 3:1) to afford compound 1'-4 as a yellow solid (1.0 g, yield: 37.0%). LCMS: m/z 271.1[M+H]⁺.

Step 10: Palladium/carbon (10%, 50 mg) and aqueous ammonia (2 drops) were added to a solution of 1'-4 (270 mg, 1.0 mmol) in methanol. The atmosphere was replaced with hydrogen three times. The mixture was stirred at room temperature for 2 hours. After filtration, the filtrate was concentrated to dryness under reduced pressure. The crude product was purified by silica gel column chromatography to afford compound 1'-5 as a yellow oil (168 mg, yield: 61.3%). LCMS: m/z 275.1 [M+H]⁺.

Step 11: Thionyl chloride (357 mg, 9.3 mmol) was added to a solution of 1'-5 (850 mg, 3.1 mmol) in acetonitrile. The mixture was stirred at 50 °C for 5 hours. The reaction solution was concentrated to dryness under reduced pressure. Ethyl acetate and a saturated aqueous solution of sodium bicarbonate were added to the residue for dilution. The organic phase was collected and concentrated to dryness under reduced pressure. The crude product was purified by silica gel column chromatography to afford compound 1'-6 as a yellow solid (680 mg, yield: 75.0%). LCMS: m/z 293.1 [M+H]⁺.

Step 12: Cesium carbonate (978 mg, 3.0 mmol) was added to a solution of 1'-6 (292 mg, 1.0 mmol) in acetonitrile. The mixture was stirred at room temperature for 2 hours. After filtration, the filtrate was concentrated to dryness under reduced pressure. The crude product was purified by silica gel column chromatography to afford compound 1'-7 as a yellow solid (155 mg, yield: 60.5 %). LCMS: m/z 257.2 [M+H]⁺.

Step 13: Liquid bromine (3.2 g, 20.0 mmol) was added to a solution of 1'-7 (2.56 g, 10.0 mmol) in acetonitrile. The reaction mixture was stirred at 25 °C for 15 hours. Ethyl acetate and a saturated aqueous solution of sodium sulfite were added to the reaction solution for dilution. The organic phase was collected, dried over anhydrous sodium sulfate and concentrated to dryness under reduced pressure. The crude product was purified by silica gel column chromatography to afford compound 1'-8 as a yellow solid (2.8 g, yield: 83.6 %). LCMS: m/z 335.1/337.1 [M+H]⁺.

¹H NMR (400 MHz, CDCl₃) δ ppm: 7.36~7.29 (m, 3H), 7.03~7.01 (m, 2H), 5.54~5.50 (m, 1H), 4.42-4.35 (m, 2H), 3.14-2.91 (m, 3H), 2.57-2.51 (m, 1H), 1.37 (t, J = 7.2 Hz, 3H).

Step 14: Potassium carbonate (2.14 g, 15.5 mmol) and [1,1'-bis(diphenylphosphino)ferrocene]dichloropalladium (568 mg, 0.78 mmol) were added to a solution of 1'-8 (2.6 g, 7.8 mmol) and (E)-2-(2-ethoxyvinyl)-4,4,5,5-tetramethyl-1,3,2-dioxaborolane (3.1 g, 15.5 mmol) in ethylene glycol dimethyl ether and water. Under argon protection, the reaction was stirred at 90 °C for 15 hours. The reaction solution was diluted with ethyl acetate and water. The organic phase was collected, dried over anhydrous sodium sulfate and concentrated to dryness under reduced pressure. The crude product was purified by silica gel column chromatography to afford compound 1'-9 as a white solid (1.62 g, yield: 64.0%). LCMS: m/z 327.2 [M+H]⁺.

Step 15: An aqueous solution of hydrochloric acid (6 M, 7 mL) was added to a solution of ethyl 1'-9 (2.4 g, 7.36 mmol) in tetrahydrofuran. The mixture was stirred at room temperature for 1 hour. An additional aqueous solution of hydrochloric acid (6 M, 8 mL) was added. After stirring at room temperature for 1 hour, the mixture was allowed to react at 60 °C for 0.5 hours. Ethyl acetate and a saturated aqueous solution of sodium bicarbonate were added to the reaction solution for dilution. The organic phase was collected, dried over anhydrous sodium sulfate and concentrated to dryness under reduced pressure. The crude product was purified by silica gel column chromatography to afford compound 1'-10 as a yellow oil (1.3 g, yield: 59.1 %). LCMS: m/z 299.1 [M+H]⁺.

Step 16: Compound A (406 mg, 1.5 mmol) and one drop of acetic acid were added to a solution of 1'-10 (298 mg, 1.0 mmol) in methanol. The mixture was stirred at room temperature for 0.1 hours. Sodium cyanoborohydride (125 mg, 2 mmol) was added. The mixture was stirred at room temperature for 15 hours. Ethyl acetate and a saturated aqueous solution of sodium bicarbonate were added to the reaction solution for dilution. The organic phase was collected, dried over anhydrous sodium sulfate and concentrated to dryness under reduced pressure. The crude product was purified by silica gel column chromatography to afford compound 1'-11 as a colorless oil (380 mg, yield: 68.8 %). LCMS: m/z 553.1/555.1 [M+H]⁺.

Step 17: A solution of trimethylaluminum in toluene (1.0 M, 0.54 mL, 0.54 mmol) was added to a solution of 1'-11 (100 mg, 0.18 mmol) in toluene. The mixture was stirred at 110 °C for 15 hours. The reaction solution was diluted with ethyl acetate and water. The organic phase was collected, dried over anhydrous sodium sulfate and concentrated to dryness under reduced pressure. The crude product was purified by silica gel column chromatography to afford compound 1'-12 as a white solid (50 mg, yield: 54.5 %). LCMS: m/z 507.1/509.1 [M+H]⁺.

Step 18: Bis(triphenylphosphine)palladium(II) dichloride (14 mg, 0.02 mmol) was added to a solution of 1'-12 (50.7 mg, 0.1 mmol), cyclopropylacetylene (66 mg, 1.0 mmol), copper(I) iodide (4 mg, 0.02 mmol) and N,N-diisopropylethylamine (38 mg, 0.3 mmol) in N,N-dimethylformamide. Under argon protection, the mixture was stirred at 80 °C for 15 hours. The reaction solution was diluted with ethyl acetate and water. The organic phase was collected, dried over anhydrous sodium sulfate and concentrated to dryness under reduced pressure. The crude product was purified by Prep-HPLC to afford compound 1' as a white solid (2 mg, yield: 24.4%). LCMS: m/z 493.2 [M+H]⁺.

¹H NMR (400 MHz, MeOD-d4) δ ppm: 7.37~7.21 (m, 5H), 7.12~7.07 (m, 3H), 5.73~5.68 (m, 1H), 5.47~5.43 (m, 1H), 4.84~4.81 (m, 1H), 4.41~4.36 (m, 1H), 4.18~4.12 (m, 1H), 3.66~3.61 (m, 1H), 3.36 (s, 3H), 3.13~3.10 (m, 1H), 3.04~2.93 (m, 3H), 2.82~2.81 (m, 1H), 2.55~2.51 (m, 1 H), 1.48~1.44 (m, 1H), 0.90~0.86 (m, 2H), 0.75~0.71 (m, 2H).

### Example 2: Synthesis of Compound 1" and 1‴

### (S)-7-(Cyclopropylethynyl)-5-methyl-3-((R)-1-oxo-7-phenyl-4,5,6,7-tetrahydro-1H-pyrrolo[1', 2':1,5]pyrazolo[3,4-c]pyridin-2(3H)-yl)-2,3-dihydrobenzo[b][1,4]oxepin-4(5H)-one and

### (S)-7-(Cyclopropylethynyl)-5-methyl-3-((S)-1-oxo-7-phenyl-4,5,6,7-tetrahydro-1H-pyrrolo[1',2':1,5]pyrazolo[3,4-c]pyridin-2(3H)-yl)-2,3-dihydrobenzo[b][1,4]oxepin-4(5H)-one

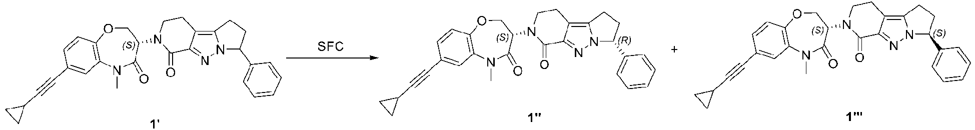

Step 1: Compound 1' (35 mg, 0.07 mmol) was purified by SFC to give two white solids: 1" (Peak 1, 8 mg, yield: 22.9%) and 1‴ (Peak 2, 8 mg, yield: 22.9%).

### 1" (peak 1):

LCMS: m/z 493.3 [M+H]⁺;
¹H NMR (400 MHz, MeOD-d₄) δ ppm: 7.37~7.28 (m, 4H), 7.23~7.21 (m, 1H), 7.12~7.07 (m, 3H), 5.71~5.68 (m, 1H), 5.47~5.43 (m, 1H), 4.84~4.81 (m, 1H), 4.41~4.36 (m, 1H), 4.18~4.12 (m, 1H), 3.66~3.61 (m, 1H), 3.35 (s, 3H), 3.14~3.10 (m, 1H), 3.04~2.93 (m, 3H), 2.82~2.81 (m, 1H), 2.55~2.49 (m, 1H), 1.48~1.44 (m, 1H), 0.90~0.86 (m, 2H), 0.75~0.71 (m, 2H).

de%= 100%.

### 1‴ (peak 2):

LCMS: m/z 493.3 [M+H]⁺;
¹H NMR (400 MHz, DMSO-d₆) δ ppm: 7.48 (d, J = 1.6 Hz, 1H), 7.36~7.29 (m, 3H), 7.26~7.24 (m, 1H), 7.18~7.16 (m, 1H), 7.11 (s, 1H), 7.09 (s, 1H), 5.56~5.46 (m, 2H), 4.83 (t, J = 10.4 Hz, 1H), 4.32 (t, J = 10.4 Hz, 1H), 3.99~3.96 (m, 1H), 3.60~3.56 (m, 1H), 3.27 (s, 3H), 3.08~3.04 (m, 1H), 2.96~2.81 (m, 3H), 2.74~2.72 (m, 1H), 2.44~2.39 (m, 1H), 1.58~1.52 (m, 1H), 0.91~0.86 (m, 2H), 0.75~0.71 (m, 2H).

de%= 100%.

### Example 3: Synthesis of Compound 2' and 2"

### (S)-7-Bromo-5-methyl-3-((R)-1-oxo-7-phenyl-4,5,6,7-tetrahydro-1H-pyrrolo[1',2':1,5]pyrazolo[3,4-c]pyridin-2(3H)-yl)-2,3-dihydrobenzo[b][1,4]oxepin-4(5H)-one and

### (S)-7-Bromo-5-methyl-3-((S)-1-oxo-7-phenyl-4,5,6,7-tetrahydro-1H-pyrrolo[1',2':1,5]pyrazolo[3,4-c]pyridin-2(3H)-yl)-2,3-dihydrobenzo[b][1,4]oxepin-4(5H)-one

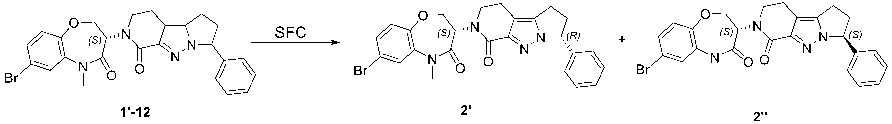

Step 1: Compound 1'-12 (25 mg, 0.05 mmol) was purified by SFC to give two white solids: 2' (Peak 1, 7 mg, yield: 28.0%) and 2" (Peak 2, 7 mg, yield: 28.0%).

### 2' (peak 1):

LCMS: m/z 509.2 [M+H]⁺;
¹H NMR (400 MHz, MeOD-d₄) δ ppm: 7.60 (d, J = 2.4 Hz, 1H), 7.42~7.39 (m, 1H), 7.36~7.26 (m, 3H), 7.15~7.06 (m, 3H), 5.74~5.68 (m, 1H), 5.47~5.43 (m, 1H), 4.87~4.82 (m, 1H), 4.42~4.38 (m, 1H), 4.18~4.12 (m, 1H), 3.66~3.61 (m, 1H), 3.35 (s, 3H), 3.15~3.11 (m, 1H), 3.04~2.93 (m, 3H), 2.82~2.81 (m, 1H), 2.55~2.49 (m, 1H).

de%= 100%.

### 2" (peak 2):

LCMS: m/z 509.2 [M+H]⁺;
¹H NMR (400 MHz, MeOD-d₄) δ ppm: 7.60 (d, J = 2.4 Hz, 1H), 7.42~7.38 (m, 1H), 7.35~7.26 (m, 3H), 7.15~7.06 (m, 3H), 5.74~5.68 (m, 1H), 5.49~5.46 (m, 1H), 4.87~4.82 (m, 1H), 4.43~4.39 (m, 1H), 4.18~4.11(m, 1H), 3.66~3.62 (m, 1H), 3.35 (s, 3H), 3.15~3.11 (m, 1H), 3.04~2.93 (m, 3H), 2.81~2.79 (m, 1H), 2.54~2.51 (m, 1H).

de%= 98.8%.

### Example 4: Synthesis of compound 3' and 3"

### (S)-5-Methyl-4-oxo-3-((R)-1-oxo-7-phenyl-4,5,6,7-tetrahydro-1 H-pyrrolo[1',2':1,5]pyrazolo[3,4-c]pyridin-2(3H)-yl)-2,3,4,5-tetrahydrobenzo[b][1,4]oxirane-7-carbonitrile and

### ((S)-5-Methyl-4-oxo-3-((S)-1-oxo-7-phenyl-4,5,6,7-tetrahydro-1H-pyrrolo[1',2':1,5]pyrazolo[3,4-c]pyridin-2(3H)-yl)-2,3,4,5-tetrahydrobenzo[b][1,4]oxirane-7-carbonitrile

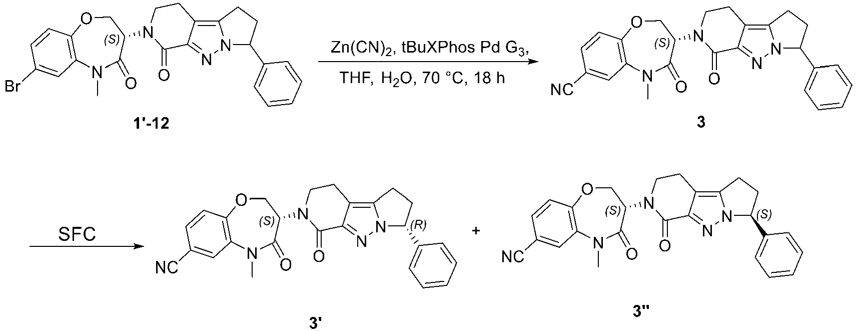

Step 1: Zinc cyanide (9.1 mg, 0.08 mmol) was added to a solution of 1'-12 (30 mg, 0.06 mmol) in tetrahydrofuran (5.0 mL) and water (2.0 mL). The atmosphere was replaced with argon three times. Methanesulfonato(2-di-tert-butylphosphino-2',4',6'-triisopropyl-1,1'-biphenylyl)(2'-amino-1,1'-biphenyl-2-yl)palladium(II) (5 mg, 0.006 mmol) was added. The atmosphere was additionally evacuated and replaced with argon three times. The mixture was allowed to react at 70 °C for 18 hours. The reaction solution was slowly poured into ice-water (10 mL) and extracted with ethyl acetate (3 × 10 mL). The insoluble matter was removed by filtration. The organic phase was combined, washed with saturated brine (2 × 50 mL), dried over anhydrous sodium sulfate and concentrated. The crude product was purified by Prep-HPLC to afford compound 3 as a pale yellow solid (18 mg, yield: 67.1%).

LCMS: m/z 454.2 [M+H]⁺.

Step 2: Compound 3 (18 mg) was purified by SFC to give 3' (Peak 1, 5 mg, yield: 27.8%) and 3" (Peak 2, 8 mg, yield: 44.4%).

### 3' (peak 1):

LCMS: m/z 454.2 [M+H]⁺;
¹H NMR (400 MHz, MeOD-d₄) δ ppm: 7.85 (d, J = 2.4 Hz, 1H), 7.64~7.62 (m, 1H), 7.37~7.27 (m, 4H), 7.10~7.08 (m, 2H), 5.71-5.66 (m, 1H), 5.47~5.43 (m, 1H), 4.89~4.84 (m, 1H), 4.51~4.47 (m, 1H), 4.15~4.10 (m, 1H), 3.66~3.61 (m, 1H), 3.35 (s, 3H), 3.14~3.11 (m, 1H), 3.04~2.93 (m, 3H), 2.82~2.81 (m, 1H), 2.55~2.49 (m, 1H).

de%=100%.

### 3" (peak 2):

LCMS: m/z 454.2 [M+H]⁺;
¹H NMR (400 MHz, MeOD-d₄) δ ppm: 7.85 (d, J = 2.4 Hz, 1 H), 7.65~7.62 (m, 1 H), 7.37~7.27 (m, 4 H), 7.08~7.06 (m,2 H), 5.71~5.66 (m, 1 H), 5.49~5.46 (m, 1 H), 4.99~4.94 (m, 1 H), 4.51~4.47 (m, 1 H), 4.15~4.10 (m, 1 H), 3.68~3.63 (m, 1 H), 3.35 (s, 3H), 3.17~3.12 (m, 1 H), 3.04~2.94 (m, 3 H), 2.85~2.82 (m, 1 H), 2.55~2.51 (m, 1 H).

de%=100%.

### Example 5: Synthesis of Compound 4' and 4"

### (S)-7-Chloro-5-methyl-3-((R)-1-oxo-7-phenyl-4,5,6,7-tetrahydro-1H-pyrrolo[1',2':1,5]pyrazolo[3,4-c]pyridin-2(3H)-yl)-2,3-dihydrobenzo[b][1,4]oxepin-4(5H)-one and

### ((S)-5-Methyl-4-oxo-3-((S)-1-oxo-7-phenyl-4,5,6,7-tetrahydro-1H-pyrrolo[1',2':1,5]pyrazolo[3,4-c]pyridin-2(3H)-yl)-2,3,4,5-tetrahydrobenzo[b][1,4]oxirane-7-carbonitrile

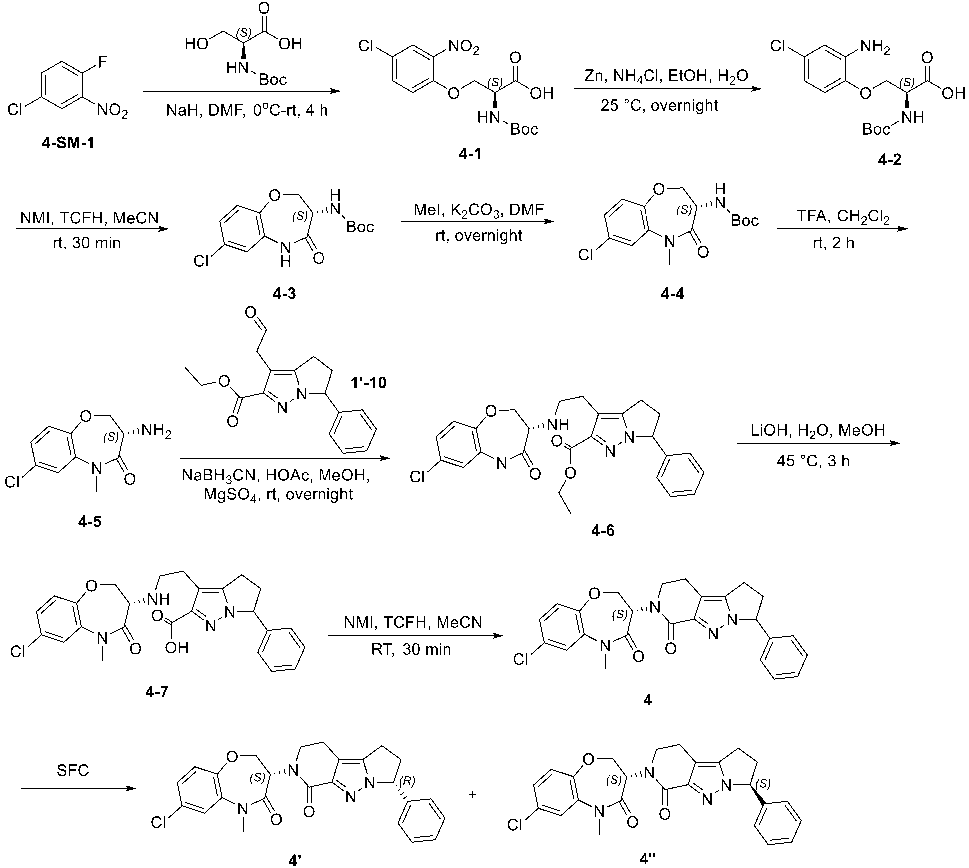

Step 1: Under nitrogen protection at 0 °C, a solution of Boc-L-serine (11.7 g, 56.97 mmol) in DMF (30 mL) was added dropwise to a solution of sodium hydride (60% dispersed in oil, 2.9 g, 72.5 mmol) in DMF (70 mL). The mixture was stirred at 0 °C for 1.5 hours. A solution of 4-SM-1 (10.0 g, 56.97 mmol) in DMF (20 mL) was added dropwise. The mixture was stirred at room temperature for 4 hours. The reaction solution was poured into water (600 mL), and the pH value was adjusted to 6 with 1 M hydrochloric acid. The mixture was extracted with ethyl acetate (3 × 150 mL). The organic phase was combined, washed with saturated brine, dried over anhydrous sodium sulfate, filtered and concentrated. The crude product was separated by column chromatography (dichloromethane:methanol = 10:1) to afford compound 4-1 as a gray solid (5.2 g, yield: 25.2%). LCMS: m/z 302.1 [M-56+H]⁺.

Step 2: Ammonium chloride (8.6 g, 160.7 mmol) was added to a solution of 4-1 (5.8 g, 16.1 mmol) in ethanol (30 mL) and water (6 mL) at 0 °C. Zinc powder (5.3 g, 80.4 mmol) was added under nitrogen protection. The reaction solution was warmed to 25 °C and stirred overnight. The reaction solution was filtered, and the filtrate was extracted with ethyl acetate (2 × 200 mL). The organic phase was combined, washed with water and brine, dried and concentrated to give compound 4-2 (4.8 g, yield: 90.3%). The crude product was used directly in the next step. LCMS: m/z 275.0 [M-56+H]⁺.

Step 3: Under nitrogen protection, N-methylimidazole (2.5 g, 30.5 mmol) and N,N,N',N'-tetramethylchloroformamidinium hexafluorophosphate (4.5 g, 16.0 mmol) were added to a solution of 4-2 (4.8 g, 14.5 mmol) in acetonitrile (20 mL). The reaction was carried out at room temperature for 30 minutes. The reaction solution was poured into water and extracted with ethyl acetate (2 × 200 mL). The organic phase was combined, washed with water and brine, then concentrated. The residue was purified by column chromatography (petroleum ether/ethyl acetate = 5/1) to afford compound 4-3 (2.3 g, yield: 50.7%). LCMS: m/z 257.0 [M-56+H]⁺.

Step 4: Iodomethane (5.2 g, 36.77 mmol) and potassium carbonate (4.1 g, 29.42 mmol) were added to a solution of 4-3 (2.3 g, 7.35 mmol) in dry N,N-dimethylformamide (10 mL) at 0 °C. The reaction was carried out overnight at room temperature. The reaction solution was poured into water and filtered. The filter cake was purified by column chromatography (dichloromethane/methanol = 20/1) to afford compound 4-4 (1.4 g, yield: 58.3%). LCMS: m/z = 271.1 [M-56+H]⁺.

Step 5: Trifluoroacetic acid (3 mL) was added to a solution of 4-4 (1.4 g, 4.28 mmol) in dry dichloromethane (10 mL) at 0 °C. The mixture was allowed to react at room temperature for 2 hours. After cooling to 0 °C, the reaction solution was slowly poured into saturated aqueous solution of sodium bicarbonate. The mixture was extracted with dichloromethane (2 × 50 mL). The organic phase was combined, washed with water and brine, dried and concentrated to give compound 4-5 (800 mg, yield: 82.4%). LCMS: m/z 227.3 [M+H]⁺.

Step 6: Acetic acid (543 mg, 9.05 mmol) and anhydrous magnesium sulfate (545 mg, 4.53 mmol) were added to a solution of 4-5 (410 mg, 1.81 mmol) and ethyl 3-(2-oxoethyl)-6-phenyl-5,6-dihydro-4H-pyrrolo[1,2-b]pyrazole-2-carboxylate (270 mg, 0.91 mmol) in methanol (10 mL). The mixture was allowed to react at room temperature for 30 minutes. Sodium cyanoborohydride (114 mg, 1.81 mmol) was then added. The reaction was carried out overnight at room temperature. Dichloromethane/methanol (V/V = 10/1, 10 mL) was added. The mixture was filtered, and the filtrate was concentrated. The residue was purified by C18 chromatography to afford compound 4-6 (120 mg, yield: 13.0%). LCMS: m/z 509.3 [M+H]⁺.

Step 7: Lithium hydroxide (47 mg, 2.0 mmol) was added to a solution of 4-6 (100 mg, 0.20 mmol) in methanol (10 mL) and water (4 mL). The mixture was allowed to react at 45 °C for 3 hours. The reaction solution was poured into water, and the pH value was adjusted to 4 with 1 M hydrochloric acid. The mixture was extracted with dichloromethane (2 × 50 mL). The organic phase was combined, washed with water and brine, dried and concentrated to give compound 4-7 (85 mg, yield: 90.0%). LCMS: m/z 481.5 [M+H]⁺.

Step 8: Under nitrogen protection, N-methylimidazole (31 mg, 0.37 mmol) and N,N,N',N'-tetramethylchloroformamidinium hexafluorophosphate (55 mg, 0.19 mmol) were added to a solution of 4-7 (85 mg, 0.18 mmol) in acetonitrile (8 mL). The reaction was carried out at room temperature for 30 minutes. The reaction solution was poured into water and extracted with ethyl acetate (2 × 50 mL). The organic phase was combined, washed with water and brine, then concentrated. The residue was purified by column chromatography (petroleum ether/ethyl acetate = 1/1) to afford compound 4 (82 mg, yield: 95%). LCMS: m/z 463.1 [M+H]⁺.

Step 9: Compound 4 (82 mg, 0.18 mmol) was purified by SFC to give two white solids: 4' (Peak 1, 26.16 mg, yield: 31.9%) and 4" (Peak 2, 19.53 mg, yield: 23.8%).

4' (1):
¹H NMR (400 MHz, CDCl₃) δ ppm: 7.31~7.26 (m, 3H), 7.21 (d, J = 2.0 Hz, 1H), 7.18 ~7.15 (m, 1H), 7.10 ~7.08 (m, 1H), 7.00 (d, J = 6.8 Hz, 2H), 5.97 (dd, J = 11.2, 8.0 Hz, 1H), 5.45~5.43 (m, 1H), 4.65~4.60 (m, 1H), 4.41 (t, J = 8.8 Hz, 1H), 4.26~4.21 (m, 1H), 3.60~3.55 (m, 1H), 3.36 (s, 3H), 3.14~3.07 (m, 2H), 3.00 ~ 2.92 (m, 1H), 2.89 ~2.83 (m, 1H), 2.73~2.69 (m, 1H), 2.55~2.50 (m, 1H).

de% = 100%.

### 4" (peak 2):

¹H NMR (400 MHz, CDCl₃) δ ppm: 7.29~7.26 (m, 3H), 7.21~7.15 (m, 2H), 7.09 ~7.07 (m, 1H), 7.04 (d, J = 6.0 Hz, 2H), 5.96 (dd, J = 10.8, 8.0 Hz, 1H), 5.45~5.43 (m, 1H), 4.65~4.60 (m, 1H), 4.39 (t, J = 8.4 Hz, 1H), 4.24~4.21 (m, 1H), 3.60~3.55 (m, 1H), 3.36 (s, 3H), 3.14~3.09 (m, 2H), 2.97~2.89 (m, 2H), 2.74~2.70 (m, 1H), 2.56~2.51 (m, 1H).

de% = 100%.

Comparative Compound AA: Comparative Compound AA was synthesized according to the method described in patent (WO2014125444, Example 45).

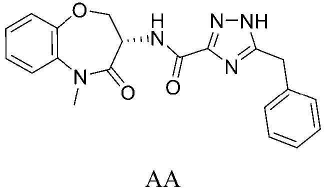

### Testing Example 1: Binding Assay Based on Fluorescence Polarization

Mouse fibroblast L929 cells were cultured in DMEM medium comprising 10% fetal bovine serum, 100 units/mL penicillin and 100 µg/mL streptomycin. Human histiocytic lymphoma cells U937 cells and human T lymphocyte leukemia cells FADD⁻/⁻ Jurkat cells were cultured in RPMI1640 medium comprising 10% fetal bovine serum, 100 units/mL penicillin and 100 µg/mL streptomycin.

Cells in the logarithmic growth phase were digested and centrifuged, and then resuspended in fresh cell culture medium to prepare cell suspensions. After counting, the cells were seeded into 384-well plates at a volume of 45 µL medium per well. Each well contained 5000 L929 cells, 10000 U937 cells or 10000 FADD⁻/⁻ Jurkat cells. The plates were then incubated for 24 hours in an incubator at 37 °C with 5% CO₂.

Test compounds were dissolved in 100% DMSO to prepare 20 mM stock solutions. In 384-well plates, the test compounds were serially diluted from 10 mM with 100% DMSO at a ratio of 1:3, giving a total of 12 concentrations. A volume of 100 nL diluted compound was added to the 384-well plate containing the seeded cells.

A 10× solution of TNFα/SM1164 or 10× solution of TNFα/Z-vAD was prepared in culture medium. A volume of 5 µL drug-containing culture medium was added to each well to achieve final concentrations of 10 ng/mL for TNFα and 40 nM for SM-164 in L929 cells; 40 ng/mL for TNFα and 25 nM for SM-164 in FADD⁻/⁻ Jurkat cells; and 100 ng/mL for TNFα and 25 µM for Z-VAD in U937 cells. The plates were then incubated for 24 hours in an incubator at 37 °C with 5% CO₂. A volume of 15 µL CTG was added to each well, and the plates were incubated at room temperature for 10 minutes in the dark. Fluorescence signals were detected using a PerkinElmer EnVision plate reader. Then, the half-maximal inhibitory activity (IC50) for each test compound was fitted using the professional graphing and analysis software GraphPad Prism. The results are shown in Table 1.

**Table 1. Inhibitory Activity against Proliferation of L929 and U937 Cells induced by TNFα**

| Compound No. | TNFα+Z L929-CTG-recoveny | TNFα+Z U937-CTG-recoveny |
|---|---|---|
| | IC₅₀ (µmol) | IC₅₀ (µmol) |
| Comparative Compound AA | 2 | 0.003 |
| Compound 1' | - | - |
| Compound 1" | 1.65 | - |
| Compound 1‴ | 0.007 | - |
| Compound 2' | 2.52 | - |
| Compound 2" | 0.009 | 0.004 |
| Compound 3' | 4.75 | - |
| Compound 3" | 0.0088 | 0.0045 |
| Compound 4' | - | - |
| Compound 4" | - | - |

The experimental results indicated that this series of compounds exhibited good inhibitory activity against proliferation of L929 and U937 cells.

### Testing Example 2: Testing Assay for Enzyme Activity

The Promega ADP-Glo kinase assay kit was used to evaluate the inhibition of the test compounds against Ripk1 enzymatic activity.

Each test compound was dissolved in 100 % DMSO to prepare a 20 mM stock solution. The compound was further diluted with DMSO to 100 times the desired test concentration. 100 nL of the diluted test compound was added to a 384-well plate. Subsequently, diluted human Ripk1, substrate MBP, and ATP were sequentially added to the 348-well plate containing the test compound to reach final concentrations of 1.25 ng/µL, 0.125 ng/mL, and 12.5 µM, respectively, in the reaction wells. The 384-well plate containing the test compounds was incubated at room temperature for 3 hours. After incubation, 10 µL of Promega ADP-Glo reagent was added to terminate the reaction and eliminate residual ATP. The mixture after terminating the reaction was incubated at room temperature for 10 minutes. Subsequently, 20 µL of Promega ADP-Glo kinase detection reagent was added to convert ADP generated in the kinase reaction into ATP. The mixture was allowed to react at room temperature for 30-60 minutes, and then fluorescence signals were detected using a PerkinElmer EnVision plate reader. Then, the half-maximal inhibitory activity (IC50) for each test compound was fitted using the professional graphing and analysis software GraphPad Prism. The results are shown in Table 2.

**Table 2: Enzyme Activity of ADP-Glo Assay**

| Compound No. | ADP GLO |
|---|---|
| | IC₅₀ (µmol) |
| Comparative Compound AA | 0.007 |
| Compound 1' | 0.35 |
| Compound 1" | - |
| Compound 1‴ | 0.13 |
| Compound 2' | 1.34 |
| Compound 2" | 0.021 |
| Compound 3' | 3.22 |
| Compound 3" | 0.014 |
| Compound 4' | - |
| Compound 4" | - |

The experimental results demonstrated that this series of compounds exhibited good inhibitory effect against Ripk1 activity.

## Claims

1. A compound of Formula A, or a pharmaceutically acceptable salt thereof, or an enantiomer, diastereomer, tautomer, solvate, polymorph, prodrug or metabolite thereof, having the structure shown below: 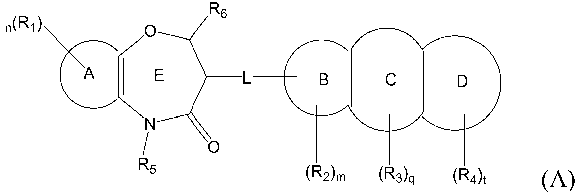 wherein:
ring A is selected from: substituted or unsubstituted C₃₋₈ cycloalkyl, substituted or unsubstituted 3- to 8- membered heterocyclyl, substituted or unsubstituted aryl, substituted or unsubstituted heteroaryl, wherein the heterocyclyl or heteroaryl comprises 1, 2, or 3 heteroatoms selected from the group consisting of N, O, S, and P;
L is selected from: 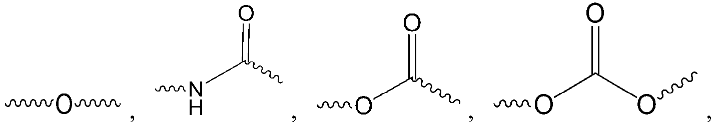 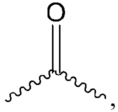 C₁₋₁₀ alkylene, or a chemical bond;
when L is 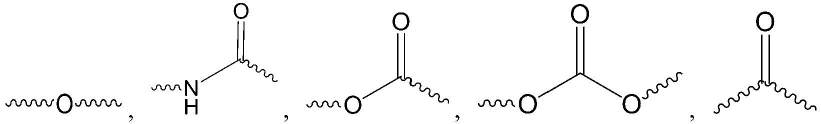 or C₁₋₁₀ alkylene, ring B is selected from: substituted or unsubstituted C₃₋₈ cycloalkyl, substituted or unsubstituted 3- to 8- membered heterocyclyl, substituted or unsubstituted aryl, substituted or unsubstituted heteroaryl, wherein the heterocyclyl or the heteroaryl comprises 1, 2, or 3 heteroatoms selected from the group consisting of N, O, S, and P; ring C and ring D are each independently selected from: substituted or unsubstituted C₃₋₈ cycloalkyl, substituted or unsubstituted 3- to 8-membered heterocyclyl, substituted or unsubstituted C₆₋₁₀ aryl, substituted or unsubstituted 5- to 10- membered heteroaryl, or absent, wherein the heterocyclyl or heteroaryl comprises 1, 2, or 3 heteroatoms selected from the group consisting of N, O, S, and P;
when L is a chemical bond, ring E is directly connected to ring B via the chemical bond, and ring B, ring C and ring D are each independently selected from: substituted or unsubstituted C₃₋₈ cycloalkyl, substituted or unsubstituted heterocyclyl, substituted or unsubstituted aryl, substituted or unsubstituted heteroaryl, wherein the heterocyclyl or the heteroaryl comprises 1, 2 or 3 heteroatoms selected from the group consisting of N, O, S and P;
R₁ is selected from H, halogen, substituted or unsubstituted C₂₋₆ alkynyl, substituted or unsubstituted C₂₋₆ alkenyl, substituted or unsubstituted C₁₋₆ alkyl, substituted or unsubstituted C₁₋₆ alkoxy, cyano, -NO₂, -OH, substituted or unsubstituted C₃₋₈ cycloalkyl, substituted or unsubstituted 3- to 8- membered heterocyclyl, substituted or unsubstituted C₆₋₁₀ aryl, substituted or unsubstituted 5- to 10- membered heteroaryl, substituted or unsubstituted 3- to 8- membered heterocyclyl spiro 3- to 8- membered heterocyclyl, substituted or unsubstituted 3- to 8- membered heterocyclyl fused 3- to 8- membered heterocyclyl, substituted or unsubstituted C₃₋₈ cycloalkyl spiro C₃₋₈ cycloalkyl, substituted or unsubstituted C₃₋₈ cycloalkyl fused C₃₋₈ cycloalkyl, substituted or unsubstituted 3- to 8- membered heterocyclyl fused C₆₋₁₀ aryl, substituted or unsubstituted 3- to 8- membered heterocyclyl fused 5- to 10- membered heteroaryl, substituted or unsubstituted C₃₋₈ cycloalkyl fused C₆₋₁₀ aryl, substituted or unsubstituted C₃₋₈ cycloalkyl fused 5- to 10- membered heteroaryl, wherein the heterocyclyl or the heteroaryl comprises 1, 2 or 3 heteroatoms selected from the group consisting of N, O, S and P;
R₂ is selected from H, oxo (=O), substituted or unsubstituted C₁₋₆ alkoxy, halogen, substituted or unsubstituted C₂₋₆ alkynyl, substituted or unsubstituted C₂₋₆ alkenyl, substituted or unsubstituted C₁₋₆ alkyl, cyano, -NO₂, -OH, substituted or unsubstituted C₃₋₈ cycloalkyl, substituted or unsubstituted 3- to 8- membered heterocyclyl, substituted or unsubstituted C₆₋₁₀ aryl, substituted or unsubstituted 5- to 10- membered heteroaryl, wherein the heterocyclyl or the heteroaryl comprises 1, 2 or 3 heteroatoms selected from the group consisting of N, O, S and P;
R₃ is selected from H, oxo, substituted or unsubstituted C₁₋₆ alkoxy, halogen, substituted or unsubstituted C₂₋₆ alkynyl, substituted or unsubstituted C₂₋₆ alkenyl, substituted or unsubstituted C₁₋₆ alkyl, cyano, -NO₂, -OH, substituted or unsubstituted C₃₋₈ cycloalkyl, substituted or unsubstituted 3- to 8- membered heterocyclyl, substituted or unsubstituted C₆₋₁₀ aryl, substituted or unsubstituted 5- to 10- membered heteroaryl, wherein the heterocyclyl or the heteroaryl comprises 1, 2 or 3 heteroatoms selected from the group consisting of N, O, S and P;
R₄ is selected from H, oxo, substituted or unsubstituted C₁₋₆ alkoxy, halogen, substituted or unsubstituted C₂₋₆ alkynyl, substituted or unsubstituted C₂₋₆ alkenyl, substituted or unsubstituted C₁₋₆ alkyl, cyano, -NO₂, -OH, substituted or unsubstituted C₃₋₈ cycloalkyl, substituted or unsubstituted 3- to 8- membered heterocyclyl, substituted or unsubstituted C₆₋₁₀ aryl, substituted or unsubstituted 5- to 10- membered heteroaryl, wherein the heterocyclyl or the heteroaryl comprises 1, 2 or 3 heteroatoms selected from the group consisting of N, O, S and P;
R₅ is selected from H, halogen, substituted or unsubstituted C₂₋₆ alkynyl, substituted or unsubstituted C₂₋₆ alkenyl, substituted or unsubstituted C₁₋₆ alkyl, substituted or unsubstituted C₁₋₆ alkoxy, cyano, -NO₂, -OH, substituted or unsubstituted C₃₋₈ cycloalkyl, substituted or unsubstituted 3- to 8- membered heterocyclyl, substituted or unsubstituted C₆₋₁₀ aryl, substituted or unsubstituted 5- to 10- membered heteroaryl, wherein the heterocyclyl or the heteroaryl comprises 1, 2 or 3 heteroatoms selected from the group consisting of N, O, S and P;
R₆ is selected from H, halogen, substituted or unsubstituted C₂₋₆ alkynyl, substituted or unsubstituted C₂₋₆ alkenyl, substituted or unsubstituted C₁₋₆ alkyl, substituted or unsubstituted C₁₋₆ alkoxy, cyano, -NO₂, -OH, substituted or unsubstituted C₃₋₈ cycloalkyl, substituted or unsubstituted 3- to 8- membered heterocyclyl, substituted or unsubstituted C₆₋₁₀ aryl, substituted or unsubstituted 5- to 10- membered heteroaryl, wherein the heterocyclyl or the heteroaryl comprises 1, 2 or 3 heteroatoms selected from the group consisting of N, O, S and P;
n, m, q and t are each independently any integer selected from 0, 1, 2 and 3;
the "substituted" means that one or more hydrogen atoms on the group are substituted with substituents selected from the group consisting of: halogen, -OH, -NO₂, -NH₂, -NH(unsubstituted or halogenated C₁₋₆ alkyl), -N(unsubstituted or halogenated C₁₋₆ alkyl)₂, -CN, unsubstituted C₁₋₈ alkyl or C₁₋₈ alkyl substituted with halogen or hydroxy, unsubstituted or halogenated C₁₋₈ alkoxy, unsubstituted or halogenated C₁₋₈ alkoxy-C₁₋₈ alkyl, unsubstituted or halogenated C₃₋₈ cycloalkyl-C₁₋₈ alkyl, unsubstituted or halogenated C₁₋₆ alkylcarbonyl, unsubstituted or halogenated C₁₋₆ alkoxycarbonyl, hydroxamic acid group, unsubstituted or halogenated C₁₋₆ alkylthio, - S(O)₂N(unsubstituted or halogenated C₁₋₆ alkyl)₂, -S(O)₂ unsubstituted or halogenated C₁₋₆ alkyl, - N(unsubstituted or halogenated C₁₋₆ alkyl)S(O)₂N(unsubstituted or halogenated C₁₋₆ alkyl)₂, - S(O)N(unsubstituted or halogenated C₁₋₆ alkyl)₂, -S(O)(unsubstituted or halogenated C₁₋₆ alkyl), - N(unsubstituted or halogenated C₁₋₆ alkyl)S(O)N(unsubstituted or halogenated C₁₋₆ alkyl)₂, - N(unsubstituted or halogenated C₁₋₆ alkyl)S(O)(unsubstituted or halogenated C₁₋₆ alkyl), unsubstituted or halogenated C₅₋₁₀ aryl, unsubstituted or halogenated 5- to 10- membered heteroaryl, unsubstituted or halogenated C₃₋₈ cycloalkyl and unsubstituted or halogenated 3- to 8- membered heterocyclyl, wherein the heterocyclyl and the heteroaryl comprise 1, 2, 3 or 4 heteroatoms selected from the group consisting of N, O and S;
" 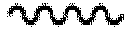 " indicates the point of attachment to the rest of the molecule via a chemical bond.

2. The compound of formula A, or a pharmaceutically acceptable salt thereof, or an enantiomer, diastereomer, tautomer, solvate, polymorph, prodrug, or metabolite thereof of claim 1, wherein:
ring A is substituted or unsubstituted C₆₋₁₄ aryl or substituted or unsubstituted 5- to 14-membered heteroaryl; ring A is substituted or unsubstituted C₆₋₁₀ aryl or substituted or unsubstituted 5- to 10- membered heteroaryl; preferably, ring A is pyridyl or phenyl;
ring B is substituted or unsubstituted 5- to 14- membered heterocyclyl or substituted or unsubstituted 5- to 14- membered heteroaryl; ring B is substituted or unsubstituted 5- to 10-membered heterocyclyl or substituted or unsubstituted 5- to 10- membered heteroaryl; preferably, ring B is piperidinyl, pyrazolyl, or pyridyl;
ring C is substituted or unsubstituted 5- to 14- membered heterocyclyl or substituted or unsubstituted 5- to 14- membered heteroaryl; ring C is substituted or unsubstituted 5- to 10-membered heterocyclyl or substituted or unsubstituted 5- to 10- membered heteroaryl; preferably, ring C is pyrazolyl or pyrrolidinyl;
ring D is substituted or unsubstituted 5- to 14- membered heterocyclyl; preferably, ring D is selected from the following groups: pyrrolidinyl, imidazolidinyl, tetrahydrofuranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl, pyranyl or oxazacyclohexane; preferably, ring D is pyrrolidinyl or 1,3-oxazacyclohexyl;
R₁ is selected from H, halogen, substituted or unsubstituted C₂₋₆ alkynyl, substituted or unsubstituted C₂₋₆ alkenyl, substituted or unsubstituted C₁₋₆ alkoxy, cyano, substituted or unsubstituted 3- to 8-membered heterocyclyl spiro 3- to 8-membered heterocyclyl, wherein the heterocyclyl comprises 1, 2, or 3 heteroatoms selected from the group consisting of N, O, S; preferably, R₁ is selected from H, halogen, substituted or unsubstituted C₂₋₅ alkynyl, substituted or unsubstituted C₁₋₃ alkoxy, cyano, substituted or unsubstituted 5- to 6- membered heterocyclyl spiro 4- to 6- membered heterocyclyl, wherein said C₂₋₅ alkynyl, C₁₋₃ alkoxy, and 5- to 6-membered heterocyclyl spiro 4- to 6-membered heterocyclyl are substituted with substituents selected from C₁₋₆ alkyl, 3- to 5-membered cycloalkyl, 4- to 6-membered heterocyclyl, hydroxyC₂₋₅ alkyl; preferably, said C₂₋₅ alkynyl, C₁₋₃ alkoxy, and 5- to 6-membered heterocyclyl spiro 4- to 6-membered heterocyclyl are substituted with substituents selected from cyclopropyl, pyranyl, hydroxyisopropyl; preferably, R₁ is selected from the group consisting of: H, 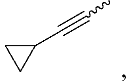 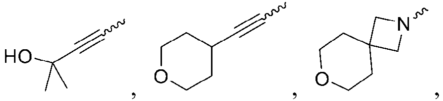 methoxy, cyano, chloro, and bromo;
R₂ is selected from H, oxo (=O), substituted or unsubstituted C₁₋₆ alkoxy, halogen, substituted or unsubstituted C₁₋₆ alkyl, cyano, -NO₂, -OH, substituted or unsubstituted C₆₋₁₀ aryl, substituted or unsubstituted 5- to 14- membered heteroaryl, wherein said heteroaryl comprises 1, 2, or 3 heteroatoms selected from the group consisting of N, O, S, and P, and said C₁₋₆ alkoxy and C₁₋₆ alkyl are each independently substituted with oxo (=O), C₆₋₁₀ aryl, -OH, cyano, or -NO₂; preferably, R₂ is selected from H, oxo (=O), C₁₋₆ alkoxy optionally substituted with C₆₋₁₀ aryl, halogen, substituted or unsubstituted 5- to 10- membered heteroaryl; preferably, R₂ is H, oxo, C₁₋₄ alkoxy substituted with C₆₋₁₀ aryl, benzopyrazolyl;
R₃ is selected from: H, substituted or unsubstituted C₆₋₁₀ aryl, substituted or unsubstituted C₁₋₆ alkoxy, halogen, cyano, -NO₂, -OH, wherein said C₁₋₆ alkoxy is substituted with oxo (=O), -OH, cyano, or -NO₂; preferably, R₃ is H or C₆₋₁₀ aryl;
R₄ is selected from H, substituted or unsubstituted C₁₋₆ alkoxy, halogen, substituted or unsubstituted C₁₋₆ alkyl, cyano, -NO₂, -OH, substituted or unsubstituted 3- to 8- membered heterocyclyl, substituted or unsubstituted C₆₋₁₀ aryl, substituted or unsubstituted 5- to 10- membered heteroaryl, wherein said heterocyclyl or the heteroaryl comprises 1, 2, or 3 heteroatoms selected from the group consisting of N, O, and S, wherein said C₁₋₆ alkoxy, C₁₋₆ alkyl, 3- to 8- membered heterocyclyl, C₆₋₁₀ aryl, 5- to 10- membered heteroaryl are each independently substituted with 1, 2, or 3 substituents selected from H, halogen, -OH, C₁₋₆ alkyl, halogenated C₁₋₆ alkyl, C₁₋₈ alkoxy; preferably, R₄ is selected from halogen, difluoropropyl, phenyl, benzyl, difluorophenyl, methylphenyl, methoxyphenyl, and pyridyl; preferably, R₄ is selected from: 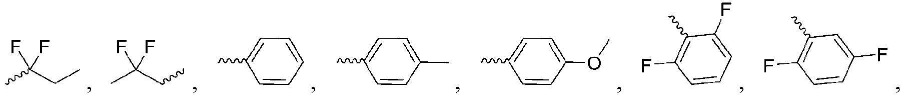 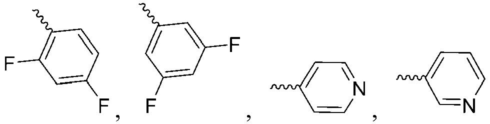 and halogen;
preferably, R₄ is selected from: 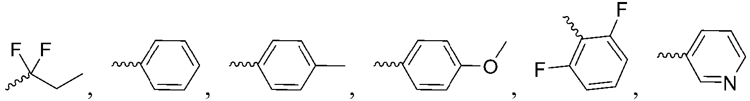 and F;
t is 1 or 2;
Rs is selected from H, substituted or unsubstituted C₁₋₆ alkoxy, halogen, substituted or unsubstituted C₁₋₆ alkyl; preferably, R₅ is unsubstituted C₁₋₆ alkyl; preferably, R₅ is methyl;
R₆ is selected from H, substituted or unsubstituted C₁₋₆ alkoxy, halogen, substituted or unsubstituted C₁₋₆ alkyl; preferably, R₆ is H.

3. The compound of formula A, or a pharmaceutically acceptable salt thereof, or an enantiomer, diastereomer, tautomer, solvate, polymorph, prodrug, or metabolite thereof of claim 1, having the structure of formula I below: 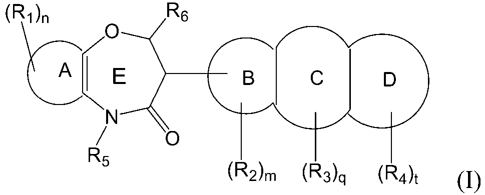 in formula I:
ring A, ring B, ring C, and ring D are each independently selected from: substituted or unsubstituted C₃₋₈ cycloalkyl, substituted or unsubstituted 3- to 8-membered heterocyclyl, substituted or unsubstituted C₆₋₁₀ aryl, substituted or unsubstituted 5- to 10-membered heteroaryl, wherein the heterocyclyl or the heteroaryl comprises 1, 2, or 3 heteroatoms selected from the group consisting of N, O, S, and P;
R₁ is selected from: H, halogen, substituted or unsubstituted C₂₋₆ alkynyl, substituted or unsubstituted C₂₋₆ alkenyl, substituted or unsubstituted C₁₋₆ alkyl, substituted or unsubstituted C₁₋₆ alkoxy, cyano, -NO₂, -OH, substituted or unsubstituted C₃₋₈ cycloalkyl, substituted or unsubstituted 3- to 8- membered heterocyclyl, substituted or unsubstituted C₆₋₁₀ aryl, substituted or unsubstituted 5- to 10- membered heteroaryl, substituted or unsubstituted 3- to 8- membered heterocyclyl spiro 3- to 8- membered heterocyclyl, substituted or unsubstituted 3- to 8- membered heterocyclyl fused 3- to 8- membered heterocyclyl, substituted or unsubstituted C₃₋₈ cycloalkyl spiro C₃₋₈ cycloalkyl, substituted or unsubstituted C₃₋₈ cycloalkyl fused C₃₋₈ cycloalkyl, substituted or unsubstituted 3- to 8- membered heterocyclyl fused C₆₋₁₀ aryl, substituted or unsubstituted 3- to 8- membered heterocyclyl fused 5- to 10- membered heteroaryl, substituted or unsubstituted C₃₋₈ cycloalkyl fused C₆₋₁₀ aryl, substituted or unsubstituted C₃₋₈ cycloalkyl fused 5- to 10- membered heteroaryl, wherein the heterocyclyl or the heteroaryl comprises 1, 2 or 3 heteroatoms selected from the group consisting of N, O, S and P;
R₂, R₃, R₄, R₅, and R₆ are each independently selected from: H, oxo (=O), substituted or unsubstituted C₁₋₆ alkoxy, halogen, substituted or unsubstituted C₂₋₆ alkynyl, substituted or unsubstituted C₂₋₆ alkenyl, substituted or unsubstituted C₁₋₆ alkyl, cyano, -NO₂, -OH, substituted or unsubstituted C₃₋₈ cycloalkyl, substituted or unsubstituted 3- to 8-membered heterocyclyl, substituted or unsubstituted C₆₋₁₀ aryl, substituted or unsubstituted 5- to 10-membered heteroaryl, wherein the heterocyclyl or the heteroaryl comprises 1, 2, or 3 heteroatoms selected from the group consisting of N, O, S, and P;
n, m, q and t are each independently any integer selected from 0, 1, 2 and 3;
the "substituted" means that one or more hydrogen atoms on the group are substituted with substituents selected from the group consisting of: halogen, -OH, -NO₂, -NH₂, -NH(unsubstituted or halogenated C₁₋₆ alkyl), -N(unsubstituted or halogenated C₁₋₆ alkyl)₂, -CN, unsubstituted C₁₋₈ alkyl or C₁₋₈ alkyl substituted with halogen or hydroxy, unsubstituted or halogenated C₁₋₈ alkoxy, unsubstituted or halogenated C₁₋₈ alkoxy-C₁₋₈ alkyl, unsubstituted or halogenated C₃₋₈ cycloalkyl-C₁₋₈ alkyl, unsubstituted or halogenated C₁₋₆ alkylcarbonyl, unsubstituted or halogenated C₁₋₆ alkoxycarbonyl, hydroxamic acid group, unsubstituted or halogenated C₁₋₆ alkylthio, - S(O)₂N(unsubstituted or halogenated C₁₋₆ alkyl)₂, -S(O)₂ unsubstituted or halogenated C₁₋₆ alkyl, - N(unsubstituted or halogenated C₁₋₆ alkyl)S(O)₂N(unsubstituted or halogenated C₁₋₆ alkyl)₂, - S(O)N(unsubstituted or halogenated C₁₋₆ alkyl)₂, -S(O)(unsubstituted or halogenated C₁₋₆ alkyl), - N(unsubstituted or halogenated C₁₋₆ alkyl)S(O)N(unsubstituted or halogenated C₁₋₆ alkyl)₂, - N(unsubstituted or halogenated C₁₋₆ alkyl)S(O)(unsubstituted or halogenated C₁₋₆ alkyl), unsubstituted or halogenated C₅₋₁₀ aryl, unsubstituted or halogenated 5- to 10- membered heteroaryl, unsubstituted or halogenated C₃₋₈ cycloalkyl and unsubstituted or halogenated 3- to 8- membered heterocyclyl, wherein the heterocyclyl and the heteroaryl comprise 1, 2, 3 or 4 heteroatoms selected from the group consisting of N, O and S.

4. The compound of formula A, or a pharmaceutically acceptable salt thereof, or an enantiomer, diastereomer, tautomer, solvate, polymorph, prodrug, or metabolite thereof of claim 3, wherein in formula I:
ring A is substituted or unsubstituted C₆₋₁₄ aryl or substituted or unsubstituted 5- to 14-membered heteroaryl; ring A is substituted or unsubstituted C₆₋₁₀ aryl or substituted or unsubstituted 5- to 10- membered heteroaryl; preferably, ring A is pyridyl or phenyl;
ring B is substituted or unsubstituted 5- to 14- membered heterocyclyl; ring B is substituted or unsubstituted 5- to 10- membered heterocyclyl; preferably, ring B is piperidinyl;
ring C is substituted or unsubstituted 5- to 14- membered heteroaryl; ring C is substituted or unsubstituted 5- to 10- membered heteroaryl; preferably, ring C is pyrazolyl;
ring D is substituted or unsubstituted 5- to 14- membered heterocyclyl; preferably, ring D is selected from pyrrolidinyl, imidazolidinyl, tetrahydrofuranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl, pyranyl or oxazacyclohexyl; preferably, ring D is pyrrolidinyl or 1,3-oxazacyclohexyl;
R₁ is selected from H, halogen, substituted or unsubstituted C₂₋₆ alkynyl, substituted or unsubstituted C₂₋₆ alkenyl, substituted or unsubstituted C₁₋₆ alkoxy, cyano, substituted or unsubstituted 3- to 8-membered heterocyclyl spiro 3- to 8-membered heterocyclyl, wherein the heterocyclyl comprises 1, 2, or 3 heteroatoms selected from the group consisting of N, O, S; preferably, R₁ is selected from H, halogen, substituted or unsubstituted C₂₋₅ alkynyl, substituted or unsubstituted C₁₋₃ alkoxy, cyano, substituted or unsubstituted 5- to 6- membered heterocyclyl spiro 4- to 6- membered heterocyclyl, wherein said C₂₋₅ alkynyl, C₁₋₃ alkoxy, and 5- to 6-membered heterocyclyl spiro 4- to 6-membered heterocyclyl are substituted with substituents selected from C₁₋₆ alkyl, 3- to 5-membered cycloalkyl, 4- to 6-membered heterocyclyl, hydroxyC₂₋₅ alkyl; preferably, said C₂₋₅ alkynyl, C₁₋₃ alkoxy, and 5- to 6-membered heterocyclyl spiro 4- to 6-membered heterocyclyl are substituted with substituents selected from cyclopropyl, pyranyl, hydroxyisopropyl; preferably, R₁ is selected from the group consisting of: H, 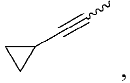 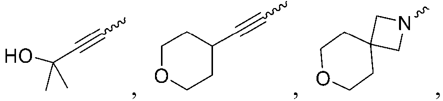 methoxy, cyano, chloro, and bromo;
R₂ is selected from H, oxo (=O), substituted or unsubstituted C₁₋₆ alkoxy, halogen, substituted or unsubstituted C₁₋₆ alkyl, cyano, -NO₂, -OH, wherein said C₁₋₆ alkoxy and C₁₋₆ alkyl are each independently substituted with oxo (=O), -OH, cyano, or -NO₂; preferably, R₂ is oxo;
R₃ is selected from: H, substituted or unsubstituted C₁₋₆ alkoxy, halogen, cyano, -NO₂, -OH, wherein said C₁₋₆ alkoxy is substituted with oxo (=O), -OH, cyano, or -NO₂; preferably, R₃ is H;
R₄ is selected from H, substituted or unsubstituted C₁₋₆ alkoxy, halogen, substituted or unsubstituted C₁₋₆ alkyl, cyano, -NO₂, -OH, substituted or unsubstituted 3- to 8- membered heterocyclyl, substituted or unsubstituted C₆₋₁₀ aryl, substituted or unsubstituted 5- to 10- membered heteroaryl, wherein said heterocyclyl or heteroaryl comprises 1, 2, or 3 heteroatoms selected from the group consisting of N, O, and S, wherein said C₁₋₆ alkoxy, C₁₋₆ alkyl, 3- to 8- membered heterocyclyl, C₆₋₁₀ aryl, 5- to 10- membered heteroaryl are each independently substituted with 1, 2, or 3 substituents selected from H, halogen, -OH, C₁₋₆ alkyl, halogenated C₁₋₆ alkyl, C₁₋₈ alkoxy; preferably, R₄ is selected from halogen, difluoropropyl, phenyl, benzyl, difluorophenyl, methylphenyl, methoxyphenyl, and pyridyl; preferably, R₄ is selected from: 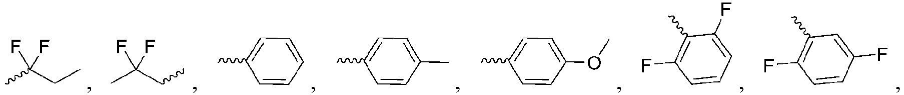 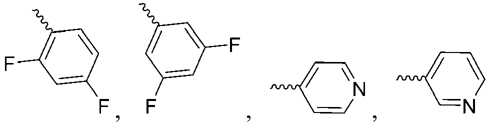 and halogen;
preferably, R₄ is selected from: 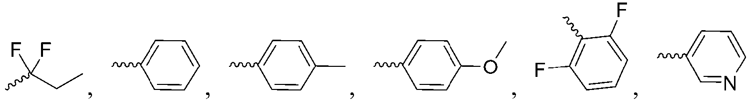 and F;
R₅ is selected from H, substituted or unsubstituted C₁₋₆ alkoxy, halogen, substituted or unsubstituted C₁₋₆ alkyl; preferably, R₅ is unsubstituted C₁₋₆ alkyl; preferably, R₅ is methyl;
R₆ is selected from H, substituted or unsubstituted C₁₋₆ alkoxy, halogen, substituted or unsubstituted C₁₋₆ alkyl; preferably, R₆ is H;
n, m, q, and t are each independently any integer selected from 0, 1, or 2; preferably, n, m, q, and t are each independently any integer selected from 0 or 1.

5. The compound of formula A, or a pharmaceutically acceptable salt thereof, or an enantiomer, diastereomer, tautomer, solvate, polymorph, prodrug, or metabolite thereof of claim 3, having the structure of formula I-1 below: 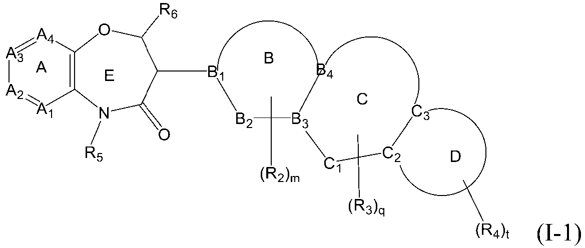 in formula I-1:
A₁, A₂, A₃, and A₄ are each independently selected from: CR₁ or N; preferably, A₁ and A₂ are each independently selected from: CR₁ or N; A₃ and A₄ are C;
B₁, B₂, B₃, and B₄ are each independently selected from: C(R₂)ₘ or N(R₂)ₘ; preferably, B₁ is N(R₂)ₘ; preferably, B₂ is C(R₂)ₘ; preferably, B₃ is C(R₂)ₘ; preferably, B₄ is C(R₂)ₘ;
C₁, C₂, and C₃ are each independently selected from: C(R₃)_{q} or N(R₃)_{q}; preferably, C₁ is N(R₃)_{q}; preferably, C₂ is N(R₃)_{q}; preferably, C₃ is C(R₃)_{q}.

6. The compound of formula A, or a pharmaceutically acceptable salt thereof, or an enantiomer, diastereomer, tautomer, solvate, polymorph, prodrug, or metabolite thereof of claim 3 or 5, wherein the structure formed by ring B, ring C and ring D is selected from the group consisting of: 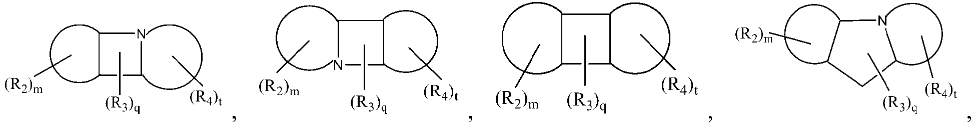 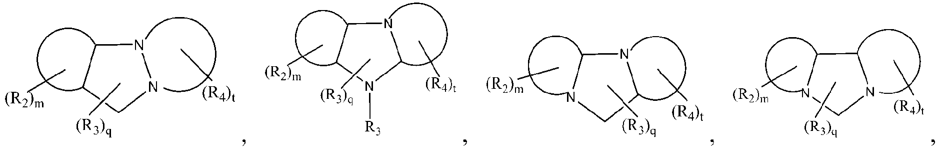 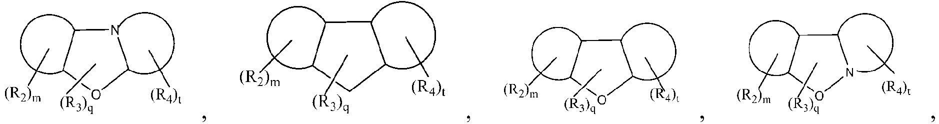 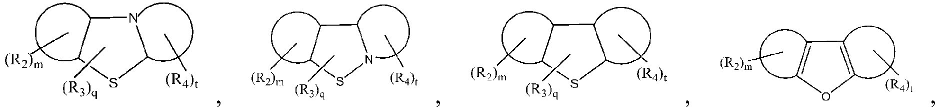 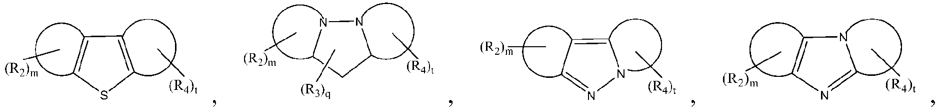 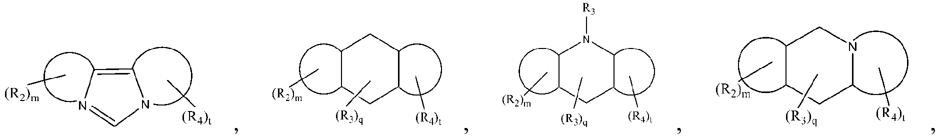 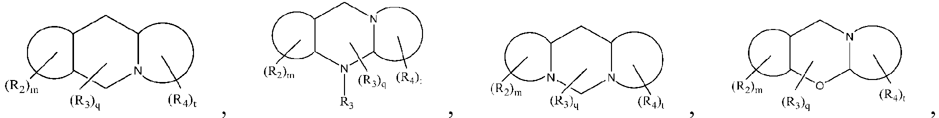 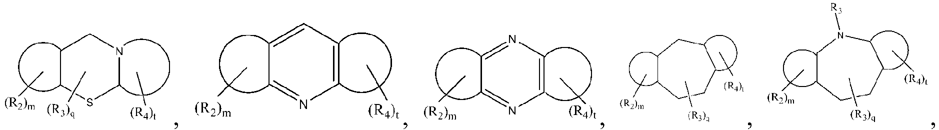 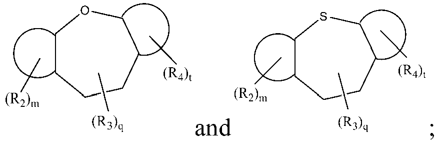 preferably, the structure formed by ring B, ring C, and ring D is selected from the group consisting of: 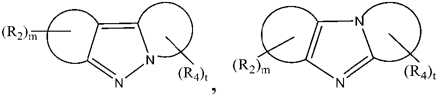 and 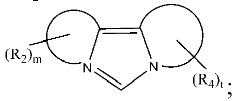 preferably, the structure formed by ring B, ring C, and ring D is selected from the group consisting of: 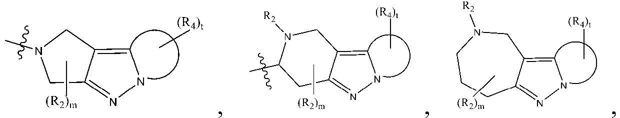 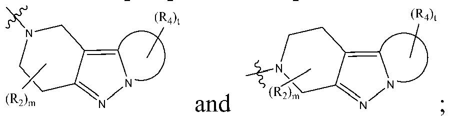 preferably, the structure formed by ring B, ring C, and ring D is selected from the group consisting of:

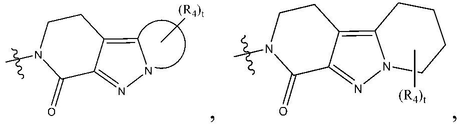

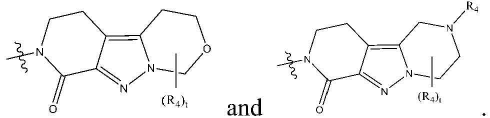

7. The compound of formula A, or a pharmaceutically acceptable salt thereof, or an enantiomer, diastereomer, tautomer, solvate, polymorph, prodrug, or metabolite thereof of claim 1, having the structure of formula II below: 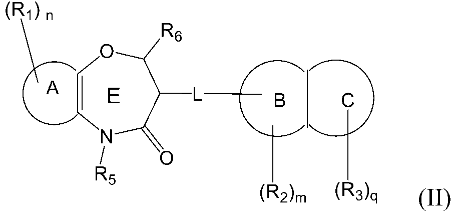 in formula II:
ring A, ring B, and ring C are each independently selected from: substituted or unsubstituted C₃₋₈ cycloalkyl, substituted or unsubstituted 3- to 8-membered heterocyclyl, substituted or unsubstituted C₆₋₁₀ aryl, substituted or unsubstituted 5- to 10-membered heteroaryl, wherein the heterocyclyl or heteroaryl comprises 1, 2, or 3 heteroatoms selected from the group consisting of N, O, S, and P;
L is selected from: 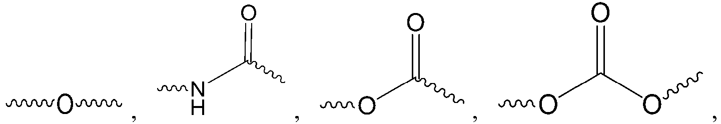 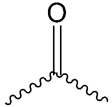 and C₁₋₁₀ alkylene;
R₁ is selected from: H, halogen, substituted or unsubstituted C₂₋₆ alkynyl, substituted or unsubstituted C₂₋₆ alkenyl, substituted or unsubstituted C₁₋₆ alkyl, substituted or unsubstituted C₁₋₆ alkoxy, cyano, -NO₂, -OH, substituted or unsubstituted C₃₋₈ cycloalkyl, substituted or unsubstituted 3- to 8- membered heterocyclyl, substituted or unsubstituted C₆₋₁₀ aryl, substituted or unsubstituted 5- to 10- membered heteroaryl, substituted or unsubstituted 3- to 8- membered heterocyclyl spiro 3- to 8- membered heterocyclyl, substituted or unsubstituted 3- to 8- membered heterocyclyl fused 3- to 8- membered heterocyclyl, substituted or unsubstituted C₃₋₈ cycloalkyl spiro C₃₋₈ cycloalkyl, substituted or unsubstituted C₃₋₈ cycloalkyl fused C₃₋₈ cycloalkyl, substituted or unsubstituted 3- to 8- membered heterocyclyl fused C₆₋₁₀ aryl, substituted or unsubstituted 3- to 8- membered heterocyclyl fused 5- to 10- membered heteroaryl, substituted or unsubstituted C₃₋₈ cycloalkyl fused C₆₋₁₀ aryl, substituted or unsubstituted C₃₋₈ cycloalkyl fused 5- to 10- membered heteroaryl, wherein the heterocyclyl or the heteroaryl comprises 1, 2 or 3 heteroatoms selected from the group consisting of N, O, S and P;
R₂, R₃, R₅, and R₆ are each independently selected from: H, oxo (=O), substituted or unsubstituted C₁₋₆ alkoxy, halogen, substituted or unsubstituted C₂₋₆ alkynyl, substituted or unsubstituted C₂₋₆ alkenyl, substituted or unsubstituted C₁₋₆ alkyl, cyano, -NO₂, -OH, substituted or unsubstituted C₃₋₈ cycloalkyl, substituted or unsubstituted 3- to 8-membered heterocyclyl, substituted or unsubstituted C₆₋₁₀ aryl, substituted or unsubstituted 5- to 10-membered heteroaryl, wherein the heterocyclyl or the heteroaryl comprises 1, 2, or 3 heteroatoms selected from the group consisting of N, O, S, and P;
n, m, and q are each independently any integer selected from 0, 1, 2, and 3;
the "substituted" means that one or more hydrogen atoms on the group are substituted with substituents selected from the group consisting of: halogen, -OH, -NO₂, -NH₂, -NH(unsubstituted or halogenated C₁₋₆ alkyl), -N(unsubstituted or halogenated C₁₋₆ alkyl)₂, -CN, unsubstituted C₁₋₈ alkyl or C₁₋₈ alkyl substituted with halogen or hydroxy, unsubstituted or halogenated C₁₋₈ alkoxy, unsubstituted or halogenated C₁₋₈ alkoxy-C₁₋₈ alkyl, unsubstituted or halogenated C₃₋₈ cycloalkyl-C₁₋₈ alkyl, unsubstituted or halogenated C₁₋₆ alkylcarbonyl, unsubstituted or halogenated C₁₋₆ alkoxycarbonyl, hydroxamic acid group, unsubstituted or halogenated C₁₋₆ alkylthio, - S(O)₂N(unsubstituted or halogenated C₁₋₆ alkyl)₂, -S(O)₂ unsubstituted or halogenated C₁₋₆ alkyl, - N(unsubstituted or halogenated C₁₋₆ alkyl)S(O)₂N(unsubstituted or halogenated C₁₋₆ alkyl)₂, - S(O)N(unsubstituted or halogenated C₁₋₆ alkyl)₂, -S(O)(unsubstituted or halogenated C₁₋₆ alkyl), - N(unsubstituted or halogenated C₁₋₆ alkyl)S(O)N(unsubstituted or halogenated C₁₋₆ alkyl)₂, - N(unsubstituted or halogenated C₁₋₆ alkyl)S(O)(unsubstituted or halogenated C₁₋₆ alkyl), unsubstituted or halogenated C₅₋₁₀ aryl, unsubstituted or halogenated 5- to 10- membered heteroaryl, unsubstituted or halogenated C₃₋₈ cycloalkyl, unsubstituted or halogenated 3- to 8- membered heterocyclyl, wherein the heterocyclyl and the heteroaryl comprise 1, 2, 3 or 4 heteroatoms selected from the group consisting of N, O and S.

8. The compound of formula A, or a pharmaceutically acceptable salt thereof, or an enantiomer, diastereomer, tautomer, solvate, polymorph, prodrug, or metabolite thereof of claim 7, in formula II:
ring A is selected from: substituted or unsubstituted C₆₋₁₀ aryl, substituted or unsubstituted 5-to 10- membered heteroaryl, wherein said heteroaryl comprises 1, 2, or 3 heteroatoms selected from the group consisting of N, O, and S; preferably, ring A is phenyl;
ring B is selected from: substituted or unsubstituted C₆₋₁₀ aryl, substituted or unsubstituted 5-to 10- membered heteroaryl, wherein said heteroaryl comprises 1 or 2 heteroatoms selected from the group consisting of N, O, and S; preferably, ring B is substituted or unsubstituted 5- to 10-membered heteroaryl; preferably, ring B is pyrazolyl;
ring C is selected from: substituted or unsubstituted C₃₋₈ cycloalkyl, substituted or unsubstituted 3- to 8- membered heterocyclyl, wherein said heterocyclyl comprises 1, 2, or 3 heteroatoms selected from the group consisting of N, O, and S; preferably, ring C is substituted or unsubstituted 3- to 8- membered heterocyclyl; preferably, ring C is pyrrolidinyl;
L is selected from: 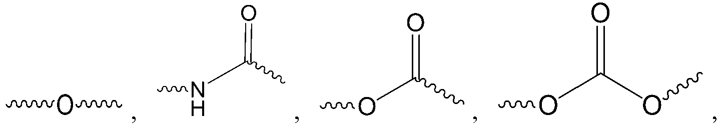 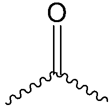 and C₁₋₁₀ alkylene; preferably, L is selected from: 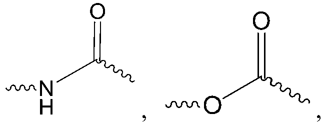 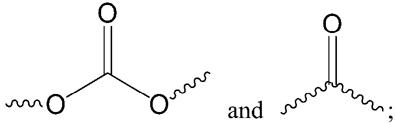 preferably, L is 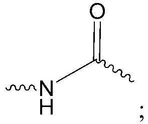
R₁ is selected from: H, halogen, substituted or unsubstituted C₂₋₆ alkynyl, substituted or unsubstituted C₂₋₆ alkenyl, substituted or unsubstituted C₁₋₆ alkyl, cyano, -NO₂, -OH, substituted or unsubstituted C₃₋₈ cycloalkyl, substituted or unsubstituted 3- to 8- membered heterocyclyl, substituted or unsubstituted C₆₋₁₀ aryl, substituted or unsubstituted 5- to 10- membered heteroaryl, wherein the heterocyclyl or heteroaryl comprises 1, 2, or 3 heteroatoms selected from the group consisting of N, O, S, and P; preferably, R₁ is selected from: H, halogen, cyano, -NO₂, -OH; preferably, R₁ is selected from: H or halogen;
R₂ is selected from: H, substituted or unsubstituted C₁₋₆ alkoxy, halogen, substituted or unsubstituted C₂₋₆ alkynyl, substituted or unsubstituted C₂₋₆ alkenyl, substituted or unsubstituted C₁₋₆ alkyl, cyano, -NO₂, -OH; preferably, R₂ is selected from: H, halogen, cyano, -NO₂, -OH; preferably, R₂ is H;
R₃ is selected from: H, oxo (=O), substituted or unsubstituted C₁₋₆ alkoxy, halogen, substituted or unsubstituted C₂₋₆ alkynyl, substituted or unsubstituted C₂₋₆ alkenyl, substituted or unsubstituted C₁₋₆ alkyl, cyano, -NO₂, -OH, substituted or unsubstituted C₆₋₁₀ aryl, substituted or unsubstituted 5-to 10- membered heteroaryl, wherein said heteroaryl comprises 1, 2, or 3 heteroatoms selected from the group consisting of N, O, S, and P; preferably, R₃ is selected from: H, halogen, substituted or unsubstituted C₁₋₆ alkyl, cyano, -NO₂, -OH, substituted or unsubstituted C₆₋₁₀ aryl; preferably, R₃ is selected from: H or C₆₋₁₀ aryl;
R₅ is selected from H, substituted or unsubstituted C₁₋₆ alkoxy, halogen, substituted or unsubstituted C₁₋₆ alkyl; preferably, R₅ is unsubstituted C₁₋₆ alkyl; preferably, R₅ is methyl;
R₆ is selected from H, substituted or unsubstituted C₁₋₆ alkoxy, halogen, substituted or unsubstituted C₁₋₆ alkyl; preferably, R₆ is H;
n, m, and q are each independently any integer selected from 0, 1, or 2; preferably, n, m, and q are each independently any integer selected from 0 or 1.

9. The compound of formula A, or a pharmaceutically acceptable salt thereof, or an enantiomer, diastereomer, tautomer, solvate, polymorph, prodrug, or metabolite thereof of claim 7, having the structure of formula II-1 below: 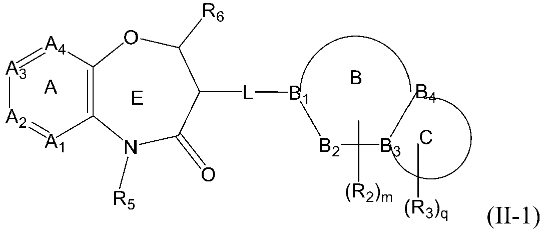 in formula II-1:
A₁, A₂, A₃, and A₄ are each independently selected from: CR₁ or N; preferably, A₁, A₂, A₃, and A₄ are all C;
B₁, B₂, B₃, and B₄ are each independently selected from: C(R₂)ₘ or N(R₂)ₘ; preferably, B₁ is C(R₂)ₘ; preferably, B₂ is N(R₂)ₘ; preferably, B₃ is N(R₂)ₘ; preferably, B₄ is C(R₂)ₘ.

10. The compound of formula A, or a pharmaceutically acceptable salt thereof, or an enantiomer, diastereomer, tautomer, solvate, polymorph, prodrug, or metabolite thereof of claim 1, having the structure of formula III below: 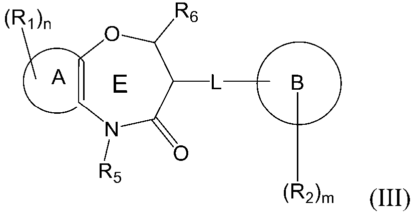 in formula III:
ring A and ring B are each independently selected from: substituted or unsubstituted C₃₋₈ cycloalkyl, substituted or unsubstituted 3- to 8-membered heterocyclyl, substituted or unsubstituted C₆₋₁₀ aryl, substituted or unsubstituted 5- to 10-membered heteroaryl, wherein the heterocyclyl or the heteroaryl comprises 1, 2, or 3 heteroatoms selected from the group consisting of N, O, S, and P;
L is selected from: 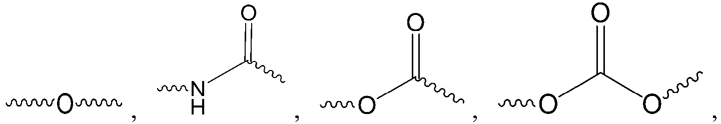 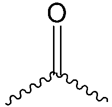 and C₁₋₁₀ alkylene;
R₁ is selected from: H, halogen, substituted or unsubstituted C₂₋₆ alkynyl, substituted or unsubstituted C₂₋₆ alkenyl, substituted or unsubstituted C₁₋₆ alkyl, substituted or unsubstituted C₁₋₆ alkoxy, cyano, -NO₂, -OH, substituted or unsubstituted C₃₋₈ cycloalkyl, substituted or unsubstituted 3- to 8- membered heterocyclyl, substituted or unsubstituted C₆₋₁₀ aryl, substituted or unsubstituted 5- to 10- membered heteroaryl, substituted or unsubstituted 3- to 8- membered heterocyclyl spiro 3- to 8- membered heterocyclyl, substituted or unsubstituted 3- to 8- membered heterocyclyl fused 3- to 8- membered heterocyclyl, substituted or unsubstituted C₃₋₈ cycloalkyl spiro C₃₋₈ cycloalkyl, substituted or unsubstituted C₃₋₈ cycloalkyl fused C₃₋₈ cycloalkyl, substituted or unsubstituted 3- to 8- membered heterocyclyl fused C₆₋₁₀ aryl, substituted or unsubstituted 3- to 8- membered heterocyclyl fused 5- to 10- membered heteroaryl, substituted or unsubstituted C₃₋₈ cycloalkyl fused C₆₋₁₀ aryl, substituted or unsubstituted C₃₋₈ cycloalkyl fused 5- to 10- membered heteroaryl, wherein the heterocyclyl or the heteroaryl comprises 1, 2 or 3 heteroatoms selected from the group consisting of N, O, S and P;
R₂, R₅, and R₆ are each independently selected from: H, oxo (=O), substituted or unsubstituted C₁₋₆ alkoxy, halogen, substituted or unsubstituted C₂₋₆ alkynyl, substituted or unsubstituted C₂₋₆ alkenyl, substituted or unsubstituted C₁₋₆ alkyl, cyano, -NO₂, -OH, substituted or unsubstituted C₃₋₈ cycloalkyl, substituted or unsubstituted 3- to 8-membered heterocyclyl, substituted or unsubstituted C₆₋₁₀ aryl, substituted or unsubstituted 5- to 10-membered heteroaryl, wherein the heterocyclyl or heteroaryl comprises 1, 2, or 3 heteroatoms selected from the group consisting of N, O, S, and P;
n and m are each independently any integer selected from 0, 1, 2, or 3;
the "substituted" means that one or more hydrogen atoms on the group are substituted with substituents selected from the group consisting of: halogen, -OH, -NO₂, -NH₂, -NH(unsubstituted or halogenated C₁₋₆ alkyl), -N(unsubstituted or halogenated C₁₋₆ alkyl)₂, -CN, unsubstituted C₁₋₈ alkyl or C₁₋₈ alkyl substituted with halogen or hydroxy, unsubstituted or halogenated C₁₋₈ alkoxy, unsubstituted or halogenated C₁₋₈ alkoxy-C₁₋₈ alkyl, unsubstituted or halogenated C₃₋₈ cycloalkyl-C₁₋₈ alkyl, unsubstituted or halogenated C₁₋₆ alkylcarbonyl, unsubstituted or halogenated C₁₋₆ alkoxycarbonyl, hydroxamic acid group, unsubstituted or halogenated C₁₋₆ alkylthio, - S(O)₂N(unsubstituted or halogenated C₁₋₆ alkyl)₂, -S(O)₂ unsubstituted or halogenated C₁₋₆ alkyl, - N(unsubstituted or halogenated C₁₋₆ alkyl)S(O)₂N(unsubstituted or halogenated C₁₋₆ alkyl)₂, - S(O)N(unsubstituted or halogenated C₁₋₆ alkyl)₂, -S(O)(unsubstituted or halogenated C₁₋₆ alkyl), - N(unsubstituted or halogenated C₁₋₆ alkyl)S(O)N(unsubstituted or halogenated C₁₋₆ alkyl)₂, - N(unsubstituted or halogenated C₁₋₆ alkyl)S(O)(unsubstituted or halogenated C₁₋₆ alkyl), unsubstituted or halogenated C₅₋₁₀ aryl, unsubstituted or halogenated 5- to 10- membered heteroaryl, unsubstituted or halogenated C₃₋₈ cycloalkyl, unsubstituted or halogenated 3- to 8- membered heterocyclyl, wherein the heterocyclyl and the heteroaryl comprise 1, 2, 3 or 4 heteroatoms selected from the group consisting of N, O and S.

11. The compound of formula A, or a pharmaceutically acceptable salt thereof, or an enantiomer, diastereomer, tautomer, solvate, polymorph, prodrug, or metabolite thereof of claim 10, wherein in formula III:
ring A is selected from: substituted or unsubstituted C₆₋₁₀ aryl, substituted or unsubstituted 5-to 10- membered heteroaryl, wherein said heteroaryl comprises 1, 2, or 3 heteroatoms selected from the group consisting of N, O, and S; preferably, ring A is phenyl;
ring B is selected from: substituted or unsubstituted C₆₋₁₀ aryl, substituted or unsubstituted 5-to 10- membered heteroaryl, wherein said heteroaryl comprises 1 or 2 heteroatoms selected from the group consisting of N, O, and S; preferably, ring B is substituted or unsubstituted 5- to 10-membered heteroaryl; preferably, ring B is pyridyl;
L is selected from: 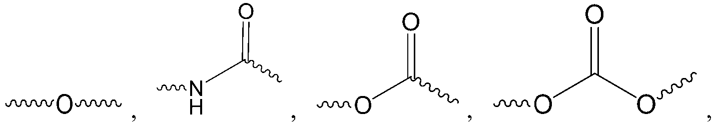 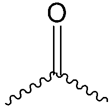 and C₁₋₁₀ alkylene; preferably, L is selected from: 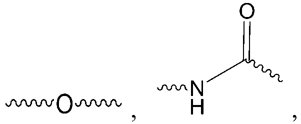 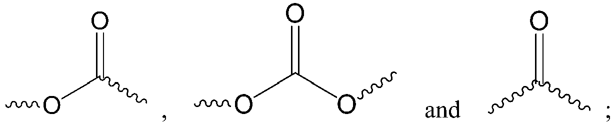 preferably, L is selected from: 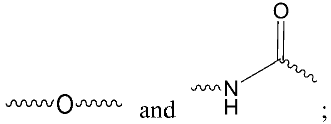
R₁ is selected from H, halogen, substituted or unsubstituted C₂₋₆ alkynyl, substituted or unsubstituted C₂₋₆ alkenyl, substituted or unsubstituted C₁₋₆ alkoxy, cyano, substituted or unsubstituted 3- to 8-membered heterocyclyl spiro 3- to 8-membered heterocyclyl, wherein the heterocyclyl comprises 1, 2, or 3 heteroatoms selected from the group consisting of N, O, S; preferably, R₁ is selected from H, halogen, substituted or unsubstituted C₂₋₅ alkynyl, substituted or unsubstituted C₁₋₃ alkoxy, cyano, substituted or unsubstituted 5- to 6- membered heterocyclyl spiro 4- to 6- membered heterocyclyl, wherein said C₂₋₅ alkynyl, C₁₋₃ alkoxy, and 5- to 6-membered heterocyclyl spiro 4- to 6-membered heterocyclyl are substituted with substituents selected from C₁₋₆ alkyl, 3- to 5-membered cycloalkyl, 4- to 6-membered heterocyclyl, hydroxyC₂₋₅ alkyl; preferably, said C₂₋₅ alkynyl, C₁₋₃ alkoxy, and 5- to 6-membered heterocyclyl spiro 4- to 6-membered heterocyclyl are substituted with substituents selected from cyclopropyl, pyranyl, hydroxyisopropyl; preferably, R₁ is selected from the group consisting of: H, 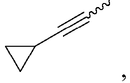 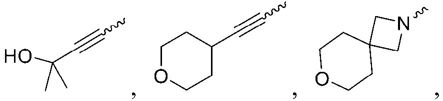 methoxy, cyano, chloro, and bromo;
R₂ is selected from H, oxo (=O), substituted or unsubstituted C₁₋₆ alkoxy, halogen, substituted or unsubstituted C₂₋₆ alkynyl, substituted or unsubstituted C₂₋₆ alkenyl, substituted or unsubstituted C₁₋₆ alkyl, cyano, -NO₂, -OH, substituted or unsubstituted C₆₋₁₀ aryl, substituted or unsubstituted 5-to 14- membered heteroaryl, wherein said heteroaryl comprises 1, 2, or 3 heteroatoms selected from the group consisting of N, O, S, and P; preferably, R₂ is selected from H, oxo (=O), C₁₋₆ alkoxy optionally substituted with C₆₋₁₀ aryl, halogen, substituted or unsubstituted C₁₋₆ alkyl, cyano, -NO₂, -OH, substituted or unsubstituted 5- to 10- membered heteroaryl; preferably, R₂ is selected from H, C₁₋₆ alkoxy optionally substituted with C₆₋₁₀ aryl, halogen, 5- to 10- membered heteroaryl;
R₅ is selected from H, substituted or unsubstituted C₁₋₆ alkoxy, halogen, substituted or unsubstituted C₁₋₆ alkyl; preferably, R₅ is unsubstituted C₁₋₆ alkyl; preferably, R₅ is methyl;
R₆ is selected from H, substituted or unsubstituted C₁₋₆ alkoxy, halogen, substituted or unsubstituted C₁₋₆ alkyl; preferably, R₆ is H;
n and m are each independently any integer selected from 0, 1, or 2; preferably, n and m are each independently any integer selected from 0 or 1.

12. The compound of formula A, or a pharmaceutically acceptable salt thereof, or an enantiomer, diastereomer, tautomer, solvate, polymorph, prodrug, or metabolite thereof of claim 10, wherein the compound has the structure of formula III-1 below: 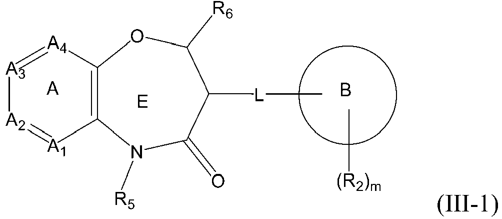 wherein A₁, A₂, A₃, and A₄ are each independently selected from CR₁ or N; preferably, A₁, A₂, A₃, and A₄ are C.

13. The compound of formula A, or a pharmaceutically acceptable salt thereof, or a solvate, isotopically substituted derivative, polymorph, prodrug or metabolite thereof of claim 1, wherein the compound is selected from the following structures: 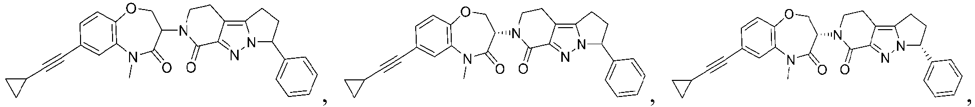 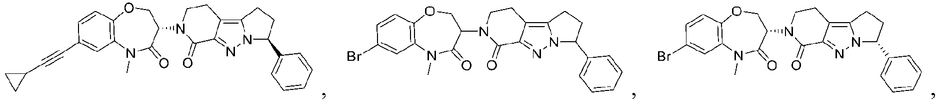 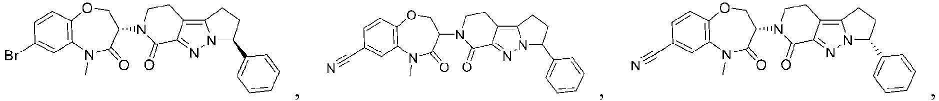 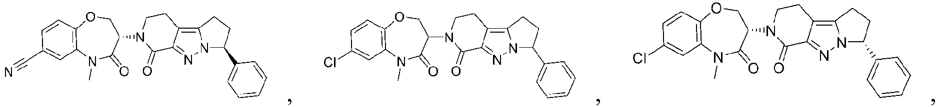 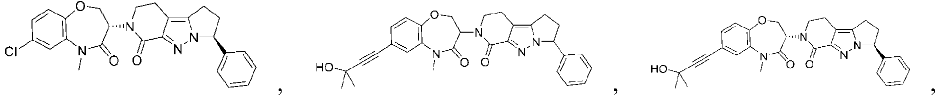 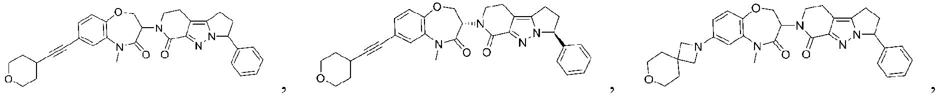 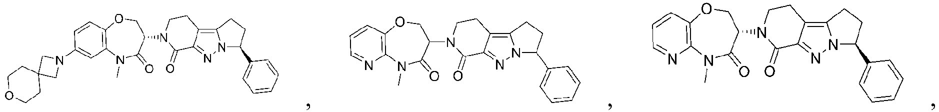 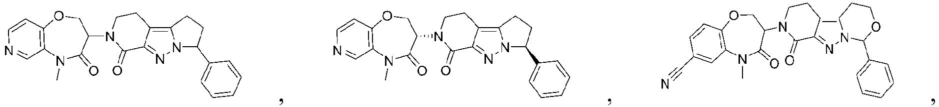 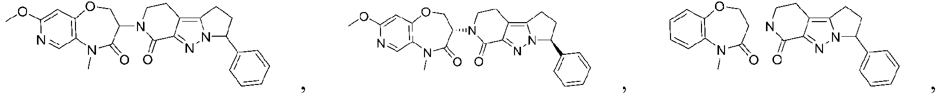 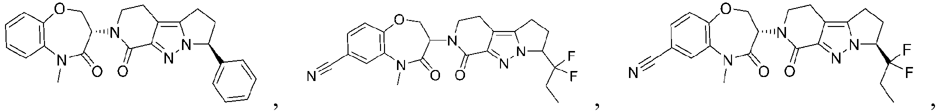 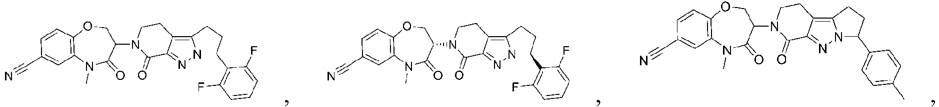 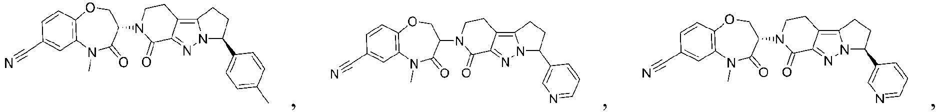 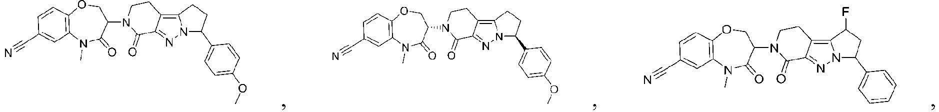 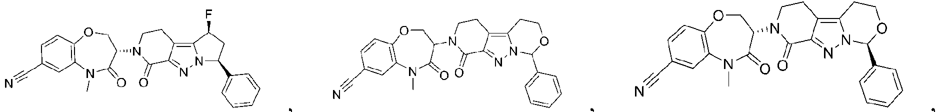 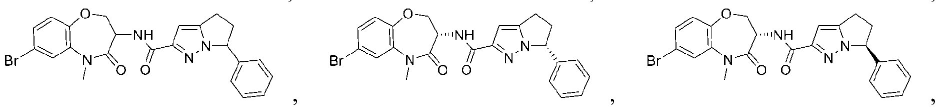 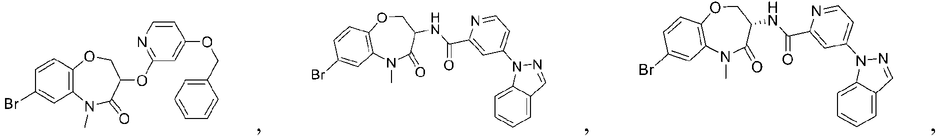 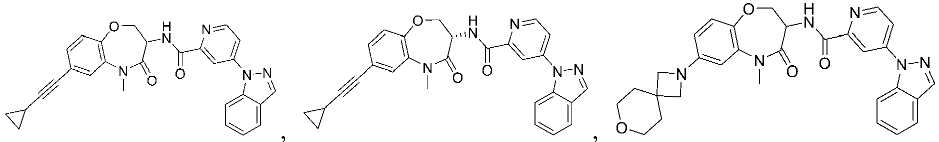 and

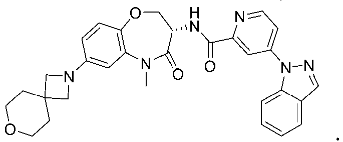

14. A pharmaceutical composition, wherein the pharmaceutical composition comprises: (a) the compound of Formula A, or a pharmaceutically acceptable salt thereof, or a solvate, isotopically substituted derivative, polymorph, prodrug or metabolite thereof of any one of claims 1-13, as an active ingredient, and (b) a pharmaceutically acceptable carrier or excipient.

15. The pharmaceutical composition of claim 14, wherein the pharmaceutical composition further comprises (c) a second active ingredient, wherein said second active ingredient is a Ripk1 inhibitor.

16. Use of the compound of formula A, or a pharmaceutically acceptable salt thereof, or a solvate, isotopically substituted derivative, polymorph, prodrug, or metabolite thereof of any one of claims 1-13, in the manufacture of a medicament for treating or preventing a Ripk1-related disease.

17. The use of claim 16, wherein the Ripkl-related disease is selected from inflammatory diseases (e.g., Crohn's disease and ulcerative colitis, inflammatory bowel disease, asthma, graft-versus-host disease, chronic obstructive pulmonary disease), inflammatory skin diseases (e.g., psoriasis, eczema), autoimmune diseases (e.g., Graves' disease, rheumatoid arthritis, systemic lupus erythematosus, psoriasis; destructive bone diseases such as bone resorption diseases, osteoarthritis, osteoporosis, multiple myeloma-related bone disease); proliferative diseases (e.g., acute myeloid leukemia, chronic myelogenous leukemia), angiogenesis disorders (e.g., angiogenesis disorders comprising solid tumors, ocular neovascularization and infantile hemangioma), infectious diseases (e.g., sepsis, septic shock and shigellosis); neurodegenerative diseases (e.g., Alzheimer's disease, Parkinson's disease, amyotrophic lateral sclerosis, cerebral ischemia or neurodegenerative diseases caused by traumatic injury), tumors and viral diseases (e.g., metastatic melanoma, Kaposi's sarcoma, multiple myeloma, fibrosarcoma, lymphoma, HIV infection and CMV retinitis, AIDS); preferably, said inflammatory skin disease is selected from contact eczema, atopic eczema, hand eczema, and radiation dermatitis.

18. The use of claim 16, wherein said Ripk1-related disease is selected from pancreatitis (acute or chronic), asthma, allergies, adult respiratory distress syndrome, chronic obstructive pulmonary disease, glomerulonephritis, rheumatoid arthritis, systemic lupus erythematosus, scleroderma, chronic thyroiditis, Graves' disease, autoimmune gastritis, diabetes, autoimmune hemolytic anemia, autoimmune neutropenia, thrombocytopenia, atopic dermatitis, chronic active hepatitis, myasthenia gravis, amyotrophic lateral sclerosis, multiple sclerosis, inflammatory bowel disease, ulcerative colitis, Crohn's disease, psoriasis, graft-versus-host disease, endotoxin-induced inflammatory response, tuberculosis, atherosclerosis, muscle degeneration, cachexia, ankylosing spondylitis, psoriatic arthritis, Reiter's syndrome, gout, traumatic arthritis, rubella arthritis, acute synovitis, pancreatic β cell disease; diseases **characterized by** massive neutrophil infiltration; rheumatoid spondylitis, gouty arthritis and other arthritic conditions, cerebral malaria, chronic pulmonary inflammation, silicosis, pulmonary sarcoidosis, bone resorption diseases, allograft rejection, infection-induced fever and myalgia, cachexia secondary to infection, corpus luteum formation, scar tissue formation, ulcerative colitis, fever, influenza, osteoporosis, osteoarthritis, acute myeloid leukemia, chronic myeloid leukemia, metastatic melanoma, Kaposi's sarcoma, multiple myeloma, sepsis, septic shock and shigellosis; Alzheimer's disease, Parkinson's disease, cerebral ischemia or neurodegenerative diseases caused by traumatic injury; angiogenesis disorders comprising solid tumors, ocular neovascularization and infantile hemangioma; viral diseases comprising acute hepatitis infection (comprising hepatitis A, hepatitis B and hepatitis C), HIV infection and CMV retinitis, Acquired Immunodeficiency Syndrome, ARC or malignant tumors and herpes; stroke, myocardial ischemia, stroke heart attack, organ ischemia, vascular proliferation, cardiac and renal reperfusion injury, thrombosis, cardiac hypertrophy, thrombin-induced platelet aggregation, endotoxemia and/or toxic shock syndrome, disorders associated with prostaglandin endoperoxide synthase-2, and pemphigus vulgaris; NASH, inflammatory diseases, diseases associated with necrotic cell death, heart failure; preferably, said Ripk1-related disease is selected from ischemia-reperfusion injury, comprising cerebral ischemia-reperfusion injury caused by stroke and myocardial ischemia-reperfusion injury caused by myocardial infarction.
